# EUROPÄISCHE PATENTSCHRIFT

(11) **EP 2 103 297 B1**
(45) Veröffentlichungstag und Bekanntmachung des Hinweises auf die Patenterteilung: **16.05.2012**
(21) Anmeldenummer: 08102841.7
(22) Anmeldetag: 20.03.2008
(51) Int. Cl.: C08F 4/44, C08F 4/72, C08F 2/50, C08F 4/16, A61K 6/083

(54) **Polymerisierbare Zusammensetzung mit mehrere Germanium-Atome enthaltenden Initiatoren**
Polymerisable compound with multiple Germanium atoms containing initiators
Composition polymérisable contenant des initiateurs comprenant plusieurs atomes de Germanium

(43) Veröffentlichungstag der Anmeldung: 23.09.2009
(73) Patentinhaber: Ivoclar Vivadent AG, 9494 Schaan (LI)
(72) Erfinder: Moszner, Norbert, Prof. Dr., 9493 Mauren (LI); Zeuner, Frank, Dr., 9488, Schellenberg (LI); Liska, Robert, Prof. Dr., 2123 Schleinbach (AT); Rheinberger, Volker, Dr., 9490, Vaduz (LI); Lamparth, Iris, Dr., 9472, Grabs (CH); Fischer, Urs-Karl, 9320, Arbon (CH)
(74) Vertreter: UEXKÜLL & STOLBERG

(56) Entgegenhaltungen:
- EP-A- 0 405 786
- EP-A- 1 905 413
- GB-A- 1 429 806

## Beschreibung

Die vorliegende Erfindung betrifft polymerisierbare Zusammensetzungen, die eine Acylgermanium-Verbindung mit mehreren Germaniumatomen als Polymerisationsinitiator enthalten. Die Zusammensetzungen eignen sich besonders zur Herstellung von Adhäsiven, Beschichtungen, Zementen, Kompositen, Formteilen, wie Stäben, Platten, Scheiben oder Linsen etc., und insbesondere von Dentalwerkstoffen.

Für die Aushärtung von polymerisationsfähigen Harzen spielt der eingesetzte Initiator eine entscheidende Rolle. Photoinitiatoren absorbieren bei Bestrahlung UV- oder sichtbares Licht und bilden die polymerisationsauslösenden Spezies. Im Fall der radikalischen Polymerisation handelt es sich dabei um freie Radikale. Basierend auf dem chemischen Mechanismus der Radikalbildung werden die Photoinitiatoren in zwei Klassen eingeteilt.

Norrish-Typ-I-Photoinitiatoren bilden beim Bestrahlen durch eine unimolekulare Bindungsspaltung freie Radikale. Norrish-Typ-II-Photoinitiatoren durchlaufen bei der Bestrahlung eine bimolekulare Reaktion wobei der Photoinitiator im angeregten Zustand mit einem zweiten Molekül, dem Coinitiator, reagiert und durch Elektronen- und Protonentransfer oder direkte Wasserstoffabstraktion die polymerisationsauslösenden Radikale bildet. Für die UV-Lichthärtung werden Typ-I- und Typ-II-Photoinitiatoren eingesetzt, für den sichtbaren Lichtbereich kommen bisher nahezu ausschliesslich Typ-II-Photoinitiatoren zum Einsatz.

Die UV-Härtung zeichnet sich durch eine hohe Reaktionsgeschwindigkeit aus und wird häufig für die Beschichtungen von unterschiedlichen Substraten wie z.B. Holz, Metall oder Glas eingesetzt. So wird zum Beispiel in EP 1 247 843 ein UV-härtendes Beschichtungsmaterial beschrieben, bei dem Typ-I-Photoinitiatoren wie Diethoxyphenylacetophenon oder Acylphosphinoxide zum Einsatz kommen.

WO 01/51533 beschreibt eine UV-härtendes Holzbeschichtungsmaterial bei dem ebenfalls Acylphosphinoxide, α-Hydroxyalkylphenone oder α-Dialkoxyacetophenone als Photoinitiator Anwendung finden. Bedingt durch die geringe Wellenlänge des UV-Lichts lassen sich mit der UV-Härtung vor allem transparente Beschichtungen mit geringer Schichtstärke härten, bei starker Einfärbung oder Pigmentierung und größeren Schichtstärken stößt man jedoch an das Limit der UV-Härtung, derartige Harze härten mit UV-Licht nur unvollständig aus. Bei pigmentierten Zusammensetzungen muß außerdem ein Absorptionsbereich für den Photoinitiator gefunden werden, in dem das Pigment nur schwach absorbiert.

Werden größere Durchhärtungstiefen benötigt, wie zum Beispiel bei der Aushärtung von lichthärtenden Dentalfüllungsmaterialien, so wird in der Regel mit sichtbarem Licht bestrahlt. Das dafür am häufigsten eingesetzte Photoinitiatorsystem ist eine Kombination aus einem α-Diketon mit einem Amincoinitiator wie sie in GB 1 408 265 beschrieben ist.

Dentalzusammensetzungen, in denen dieses Photoinitiatorsystem eingesetzt wird, werden z.B. in der US 4,457,818 oder US 4,525,256 offenbart, wobei vorzugsweise Campherchinon als α-Diketon eingesetzt wird. Campherchinon hat ein Absorptionsmaximum bei einer Wellenlänge von 468 nm. Dadurch zeigt Campherchinon eine starke Gelbfärbung, mit dem Nachteil, daß mit Campherchinon/Amin initiierte Materialien nach der Aushärtung einen deutlichen Gelbstich aufweisen. Dies ist vor allem bei hellen Weißtönen des auspolymerisierten Materials sehr nachteilig.

Ein weiterer Nachteil von Typ-II-Photoinitiatoren ist, daß sie bei der Polymerisation zur Bildung einer klebrigen Oberflächenschicht führen. Diese sogenannte Inhibierungschicht ist auf die Inhibierung der radikalischen Polymerisation durch den Sauerstoff der Luft zurückzuführen.

Die EP 0 405 786 A2 offenbart Initiatoren auf der Basis von Silicium, Germanium oder Zinn, die sich für die Massepolymerisation von Acrylmonomeren in einem Extruder eignen sollen. Die Initiatoren werden zusammen mit Co-Katalysatoren wie Tetrabutylammoniumfluorid eingesetzt. Initiatoren auf der Basis von Silicium sind bevorzugt, wie beispielsweise 9-Trimethylsilylcarbazol.

Die EP 1 905 413 A1 offenbart polymerisierbare Zusammensetzungen, die Acylgermane mit einem Germaniumatom als Initiatoren enthalten.

Der Erfindung liegt die Aufgabe zugrunde, Polymerisationsinitiatoren zur Verfügung zu stellen, die mit Licht aktiviert werden können, und die eine hohe Durchhärtungstiefe des zu härtenden Materials ergeben. Die Initiatoren sollen in geringer Konzentration wirksam sein und eine schnelle Aushärtung des zu härtenden Materials ermöglichen. Außerdem sollen sie nicht zu Verfärbungen des Materials führen.

Erfindungsgemäß wird diese Aufgabe durch Zusammensetzungen mit mindestens einem polymerisierbaren Bindemittel gelöst, die mindestens eine Acylgermanium-Verbindung gemäß der allgemeinen Formel (I) als Polymerisationsinitiator enthalten, 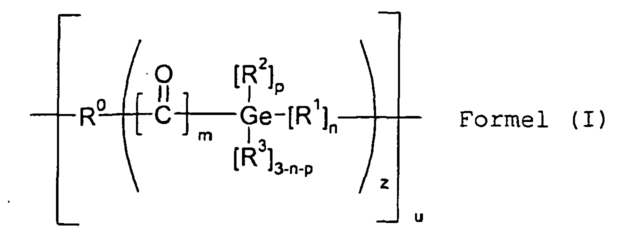 in der gemäß einer ersten Alternative
- u: 1 ist und die beiden yl-Stellen an R⁰ und R¹ entfallen,
- z: eine ganze Zahl von 2 bis 10 ist,
wobei
- R⁰: ein z-wertiger verzweigter oder vorzugsweise linearer ali- phatischer, aromatischer oder aliphatisch-aromatischer Kohlenwasserstoffrest mit 1 bis 50, vorzugsweise 1 bis 20 Kohlenstoffatomen und 0 bis 10, vorzugsweise 0 bis 6 He- teroatomen, vorzugsweise O, S, N, Si, besonders bevorzugt O und/oder S ist, der z-fach durch die in runden Klammern stehende Gruppe substituiert ist, wobei R⁰ im Fall z = 2 oder 3 auch N bzw. NH, N-C₁₋₃-Alkyl oder N-Phenyl sein kann, und wobei R⁰ im Fall z = 2 und m = 0 auch entfallen kann, so daß zwei der in runden Klammern stehenden Gruppen durch eine chemische Bindung zwischen den Germaniumatomen miteinander verbunden sind, und wobei der Rest R⁰ durch ein oder mehrere Sauerstoffatome (=O), CN, Halogen, ein oder mehrere verzweigte oder lineare C₁₋₆-Alkylreste, -O- C₁₋₆-Alkylreste und/oder polymerisationsfähige Gruppen sub- stituiert sein kann;
- R¹, R²: unabhängig voneinander 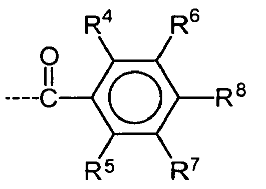 oder H sind, oder eine der für R³ angegebenen Bedeutungen haben; wobei
R⁴, R⁵ unabhängig voneinander jeweils H, Halogen, ein verzweigter oder linearer C₁₋₆-Alkyl- oder -O-C₁₋₆- Alkylrest sind, der durch ein oder mehrere Sauerstof- fatome unterbrochen sein kann;
R⁶, R⁷, R⁸ unabhängig voneinander jeweils H, Halogen, ein verzweigter, cyclischer oder vorzugsweise linearer C₁₋₂₀-Alkyl-, -Alkenyl-, -Alkyloxy- oder -Alkenoxy- Rest sind, der durch ein oder mehrere O, S oder -NR²⁰- unterbrochen und durch eine oder mehrere poly- merisationsfähige Gruppen und/oder Reste R⁹ substitu- iert sein kann, wobei R⁹ -OH, -CₓF₂ₓ₊₁ mit x = 1 bis 20, -[Si(CH₃)₂]_{y}-CH₃ mit y = 1 bis 20 ist, und R²⁰ H, Halogen, ein verzweigter, cyclischer oder vorzugswei- se linearer C₁₋₂₀-Alkyl-, -Alkenyl-, -Alkyloxy- oder - Alkenoxy-Rest ist, und
wobei R² auch ein linearer oder verzweigter aliphatischer, aromatischer oder aliphatisch-aromatischer Kohlenwasserstoffrest mit 1 bis 50, vorzugsweise 1 bis 20 Kohlenstoffatomen und 0 bis 10, vorzugsweise 0 bis 6 Heteroatomen, vorzugsweise O, S, N, Si, besonders bevorzugt O und/oder S, insbesondere ein C₁₋₂₀-Alkenylrest sein kann, der zwei Germaniumatome miteinander verbrückt.

Verbindungen der ersten Alternative lassen sich durch die folgenden Formel (I') darstellen:

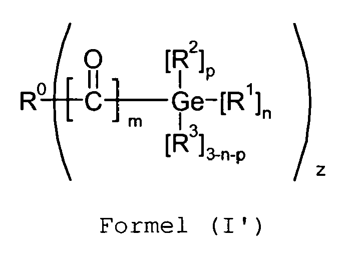

Die in runden Klammern stehende Gruppe der Formel (I') wird im folgenden auch als Klammerausdruck bezeichnet. Verbindungen der Formel (I') sind bevorzugt.

Gemäß einer zweiten Alternative ist
- u: eine ganze Zahl von 2 bis 100 und die beiden yl-Stellen an den endständigen Resten R⁰ und R¹, bzw. Ge wenn R¹ ent- fällt, sind durch H oder OH abgesättigt oder unter Ausbil- dung einer chemischen Bindung zwischen R⁰ und R¹ bzw. Ge miteinander verbunden,
- z: 1,
wobei
- R⁰: ein zweiwertiger linearer oder verzweigter aliphatischer, aromatischer oder aliphatisch-aromatischer Kohlenwasser- stoffrest mit 1 bis 50, vorzugsweise 1 bis 20 Kohlenstof- fatomen und 0 bis 10, vorzugsweise 0 bis 6 Heteroatomen, vorzugsweise O, S, N, Si, besonders bevorzugt O und/oder S ist, wobei R⁰ auch N-R²² oder -O-Si(R²³)₂-O- bedeuten kann, wobei R²² H, C₁₋₁₀-Alkyl, vorzugsweise H oder C₁₋₄-Alkyl, oder Phenyl ist, und R²³ C₁₋₁₀-Alkyl, vorzugsweise C₁₋₄-Alkyl ist oder zwei Reste R²³ eine Sauerstoffbrücke (-O-) zwi- schen zwei Si-Atomen bilden, und wobei der Rest R⁰ durch ein oder mehrere Sauerstoffatome (=O), CN, Halogen, ein oder mehrere verzweigte oder lineare C₁₋₆-Alkylreste, -O- C₁₋₆-Alkylreste und/oder polymerisationsfähige Gruppen sub- stituiert sein kann;
- R¹: -C(=O)- ist oder entfällt,
- R²: 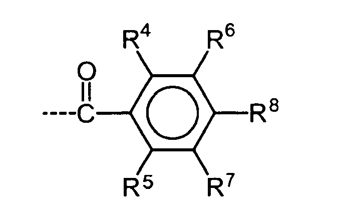 oder H ist
oder eine der für R³ angegebenen Bedeutungen hat; wo- bei
- R⁴, R⁵: unabhängig voneinander jeweils H, Halogen, ein verzweigter oder linearer C₁₋₆-Alkyl- oder -O-C₁₋₆- Alkylrest sind, der durch ein oder mehrere Sauerstof- fatome unterbrochen sein kann;
- R⁶, R⁷, R⁸: unabhängig voneinander jeweils H, Halogen, ein verzweigter, cyclischer oder vorzugsweise linearer C₁₋₂₀-Alkyl-, -Alkenyl-, -Alkyloxy- oder -Alkenoxy- Rest sind, der durch ein oder mehrere O, S oder -NR²⁰- unterbrochen und durch eine oder mehrere poly- merisationsfähige Gruppen und/oder Reste R⁹ substitu- iert sein kann, wobei R⁹ -OH, -CₓF₂ₓ₊₁ mit x = 1 bis 20, -[Si(CH₃)₂]_{y}-CH₃ mit y = 1 bis 20 ist, und R²⁰ H, Halogen, ein verzweigter, cyclischer oder vorzugswei- se linearer C₁₋₂₀-Alkyl-, -Alkenyl-, -Alkyloxy- oder -Alkenoxy-Rest ist.

Die übrigen Variablen haben in beiden Alternativen die folgenden Bedeutungen:
- m: ist 0 oder 1,
- n: ist 0 oder 1,
- p: ist 0 oder 1,
- R³: ist ein verzweigter oder vorzugsweise linearer C₁₋₁₈-Alkyl- Rest oder C₂₋₁₈-Alkenyl-Rest, wobei diese Reste unsubstitu- iert oder einfach oder mehrfach durch einen Rest substitu- iert sein können, der aus der folgenden Gruppe ausgewählt ist: Halogen, CN, 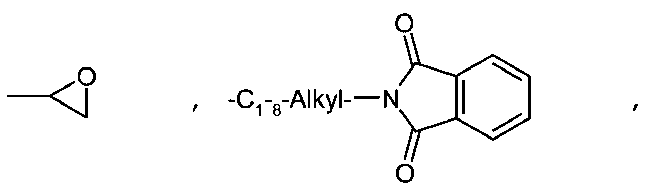 -OR¹⁰, -SR¹⁰, -OCO-R¹⁰, -COO-R¹⁰, -CH=CH-CO-OR¹⁰, -C(C₁₋₄- Alkyl)=C(C₁₋₄-Alkyl)-CO-OR¹⁰, -CO-R¹³, -CO-CH=CH-CO-C₁₋₆- Alkyl, -CO-CH=CH-CO-Phenyl, -CO-CH=CH-COO-C₁₋₁₈-Alkyl, -NR¹¹R¹², -N(R¹¹)-CO-R¹⁰, -N(R¹¹)-COO-R¹⁰, -N(R¹¹)-CO-NR¹¹R¹², -N(R¹¹)-CO-Hal, -CO-NR¹¹R¹², -SO₂-R¹⁰, -SO₂-OR¹⁰, -SO₂-NR¹¹R¹², -PO(OC₁₋₆-Alkyl)₂, -SiR¹⁴R¹⁵R¹⁶, -CH=CH-Phenyl, -C(C₁₋₄- Alkyl)=C(C₁₋₄-Alkyl)-Phenyl, Phenyl-C₁₋₄-Alkyl, Phenyl, Naphthyl, Biphenyl, C₅₋₁₂-Cycloalkyl, ein gesättigter oder ungesättigter 5- oder 6-gliedriger O-, S- oder N-haltiger heterocyclischer Ring, Benzophenonyl, Thisanthonyl, wobei
R¹⁰ H, C₁₋₁₈-Alkyl, C₁₋₁₆-Alkyl, das durch ein oder mehrere Sauerstoffatome unterbrochen ist, C₂₋₁₈-Alkenyl, C₂₋₁₈- Alkenyl, das durch ein oder mehrere Sauerstoffatome unterbrochen ist, C₃₋₁₂-Cycloalkyl, Tetrahydropyran-2- yl, Phenyl-C₁₋₂₀-Alkylen, Phenyl-C₁₋₂₀-Alkenylen, C₁₋₆- Alkyl, das unsubstituiert oder durch Halogen, Cyclo- hexyl, Cyclopentyl, Tetrahydrofuranyl, Furanyl oder Isopropyl-4-methyl-cyclohexyl substituiert sein kann, Phenyl, Naphthyl oder Biphenyl ist, wobei diese Ring- systeme unsubstituiert oder durch 1 bis 5 Halogenato- me, C₁₋₈-Alkyl-, C₁₋₈-Alkoxy- und/oder C₁₋₈- Alkylthioreste substituiert sein können,
R¹¹, R¹² unabhängig voneinander H, C₁₋₁₈-Alkyl, C₁₋₁₈- Alkyl, das durch ein oder mehrere Sauerstoffatome un- terbrochen ist, C₂₋₁-Alkenyl, C₂₋₁₈-Alkenyl, das durch ein oder mehrere Sauerstoffatome unterbrochen ist, C₃₋₁₂-Cycloalkyl, Phenyl-C₁₋₄-Alkyl, Phenyl, Naphthyl oder Pyridyl sind, wobei diese Ringsysteme unsubsti- tuiert oder durch 1 bis 5 Halogenatome, C₁₋₈-Alkyl-, C₁₋₈-Alkoxy- und/oder C₁₋₈-Alkylthioreste substituiert sein können, oder R¹¹ und R¹² bilden zusammen einen 5- oder 6-gliedrigen O-, S- oder N-haltigen heterocy- clischen Ring, der seinerseits mit einem aliphati- schen oder aromatischen Ring anelliert sein kann,
R¹³ C₁₋₁₈-Alkyl, C₂₋₁₈-Alkenyl, das durch ein oder mehrere O-Atome unterbrochen ist, C₃₋₁₂-Cycloalkyl, Phenyl- C₁₋₄-Alkyl, Phenyl, Naphthyl oder Biphenyl ist, wobei die genannten Ringsysteme unsubstituiert oder durch 1 bis 5 C₁₋₈-Alkyl-, C₁₋₈-Alkoxy-, C₁₋₈-Alkylthioreste und/oder Halogenatome substituiert sein können;
R¹⁴, R¹⁵, R¹⁶ unabhängig voneinander jeweils H, C₁₋₈- Alkyl, C₂₋₈-Alkenyl, C₇₋₉-Phenylalkyl, -O-C₁₋₈-Alkyl, Phenyl oder -O-SiR¹⁷R¹⁸R¹⁹ sind, wobei R¹⁷,R¹⁸, R¹⁹ unabhängig voneinander jeweils H, C₁₋₈- Alkyl, C₂₋₈-Alkenyl, C₇₋₉-Phenylalkyl, -O-C₁₋₈-Alkyl oder Phenyl sind, oder
- R³: ist ein verzweigter oder vorzugsweise linearer C₂₋₁₈-Alkyl- Rest oder ein C₂₋₁₈-Alkylen-Rest, der einfach oder mehrfach durch -O-, -NH-, -NR¹¹-, -S- unterbrochen ist, wobei die Reste unsubstituiert oder einfach oder mehrfach durch ei- nen Rest substituiert sein können, der aus der folgenden Gruppe gewählt ist: Halogen, CN, -OR¹⁰, -SR¹⁰, -OCO-R¹⁰, -COO-R¹⁰, -NR¹¹R¹², -N(R¹¹) --CO-R¹⁰, -N(R¹¹) -COO-R¹⁰, -N(R¹¹)-CO-NR¹¹R¹², -N(R¹¹)- CO-Hal, -CO-NR¹¹R¹², _SO₂-R¹⁰, -SO₂-OR¹⁰, -SO₂-NR¹¹R¹², -PO(OC₁₋₈-Alkyl)₂, -SiR¹⁴R¹⁵R¹⁶, Phenyl-C₁₋₄-Alkyl, Phenyl, C₅₋₁₂-Cycloalkyl; wobei R¹⁰, R¹¹, R¹², R¹⁴, R¹⁵ und R¹⁶ wie oben definiert sind;
oder
- R³: ist ein verzweigter oder vorzugsweise linearer C₂₋₁₈-Alkyl- Rest oder ein C₂₋₁₈-Alkylen-Rest, der einfach oder mehrfach durch -CO-, -COO-, -OCO-, -OCOO-, -CO-N(R¹¹)-, -N(R¹¹)-CO-, -N(R¹¹)-CO-N(R¹¹)-, -N(R¹¹)-COO-, -COO-C₁₋₆-Alkylen, -COS-C₁₋₁₈- Alkylen, -SO₂-, -SO₂-O-, -SO₂-N(R¹¹)-, -(CH₃)₂Si[OSi(CH₃)₂]_{q}-, mit q = 1 bis 6; Phenyl-C₁₋₄- Alkylen, Phenylen, Naphthylen, Biphenylen, C₅₋₁₂- Cycloakylen oder einen 5- oder 6-gliedrigen O-, S- oder N- haltigen heterocyclischen Ring unterbrochen ist;
wobei R¹¹ wie oben definiert ist;
oder
- R³: ist Trimethylsilyl, Hal-(CH₃)₂Si-[OSi(CH₃)₂]ᵣ-, (CH₃)₃Si- [OSi(CH₃)₂]ᵣ- mit r = 1 bis 6, -COOH, -COO-R¹⁰, -CO-NR¹¹R¹², -CO-Vinyl, -CO-Phenyl, wobei der Phenylrest unsubstituiert oder durch -CH₃, -OCH₃ und/oder -Cl substituiert sein kann;
wobei R¹⁰, R¹¹ und R¹² wie oben definiert sind;
oder
- R³: ist Phenyl-C₁₋₂₀-Alkyl, Phenyl, Naphthyl oder Biphenyl, C₅₋₁₂-Cycloalkyl oder ein gesättigter oder ungesättigter 5- oder 6-gliedriger O-, S- oder N-haltiger heterocyclischer Ring, wobei diese Ringsysteme unsubstituiert oder durch 1 bis 5 Halogenatome, C₁₋₈-Alkyl-, C₁₋₈-Alkoxy-, C₁₋₈- Alkylthioreste und/oder -NR¹¹R¹² substituiert sein können,
wobei R¹¹ und R¹² wie oben definiert sind.

Vorzugsweise ist
- R³: Halogen, OH, ein aromatischer C₆₋₃₀-Rest, der durch einen verzweigten, cyclischen oder vorzugsweise linearen C₁₋₂₀- Alkyl-, -Alkenyl-, -Alkoxy- oder -Alkenoxy-Rest substituiert sein kann, wobei die genannten Reste durch ein oder mehrere O, S oder N-Atome unterbrochen und/oder durch eine oder mehrere polymerisationsfähige Gruppen und/oder Reste R⁹ substituiert sein können, oder ein verzweigter, cyclischer oder vorzugsweise linearer C₁₋₂₀- Alkyl-, -Alkenyl-, -Alkoxy- oder -Alkenoxy-Rest ist, der ein- oder mehrfach durch O, S oder -NR²⁰- unterbrochen und durch eine oder mehrere polymerisationsfähige Gruppen und/oder Reste R⁹ substituiert sein kann, wobei
R⁹ -OH, -CₓF₂ₓ₊₁ mit x = 1 bis 20, -[Si(CH₃)₂]_{y}-CH₃ mit y = 1 bis 20 ist, und
R²⁰ H, Halogen, ein verzweigter, cyclischer oder vorzugsweise linearer C₁₋₂₀-Alkyl-, -Alkenyl-, -Alkyloxy- oder -Alkenoxy-Rest ist.

Darüber hinaus können jeweils zwei der Reste R¹, R² oder R³ der Formel (I) oder (I') unter Ausbildung eines 5- bis 8- gliedrigen Rings, der seinerseits mit einem oder mehreren, vorzugsweise 1 oder 2 aliphatischen oder aromatischen Ringen anelliert sein kann, miteinander verbunden sein. Diese Ringe können neben dem Germaniumatom weitere Heteroatome enthalten, vorzugsweise O-, S-, oder N-Atome. Die Anzahl zusätzlicher Hereroatome beträgt vorzugsweise 1 oder 2. Es können unterschiedliche Reste oder im Fall von m, n, p oder 3-n-p > 1 auch gleiche Reste unter Ausbildung eines oder mehrerer Ringe miteinander verbunden sein. Beispielsweise können im Fall p = 2 die beiden Reste R² miteinander verbunden sein. Durch die Verbindung zwischen den Resten werden cyclische Germanium-Verbindungen gebildet, d.h. Verbindungen, bei denen das Germaniumatom in einen Ring integriert ist. Sind zwei mal zwei Gruppen miteinander verknüpft, so handelt es sich um Spiroverbindungen mit Germanium als Zentralatom (Spiroatom). Die gebildeten Ringe können unsubstituiert oder ein- oder mehrfach, vorzugsweise ein- oder zweifach substituiert sein. Bevorzugte Substituenten sind C₁₋₄-Alkylgruppen oder =O.

Vorzugsweise haben die Variablen der Formeln (I) und insbesondere (I') die folgenden Bedeutungen:
- R⁰: ein gesättigter aliphatischer Kohlenwasserstoffrest mit 1 bis 18, vorzugsweise 1 bis 6 Kohlenstoffatomen, ein aroma- tischer Kohlenwasserstoffrest mit 6 bis 10, vorzugsweise 6 Kohlenstoffatomen, ein aliphatisch-aromatischer Kohlenwas- serstoffrest mit 7 bis 24 Kohlenstoffatomen, wobei in die- sen Resten 1 bis z nicht benachbarte Kohlenstoffatome durch Sauerstoff ersetzt sein können,
- R¹, R²: unabhängig voneinander 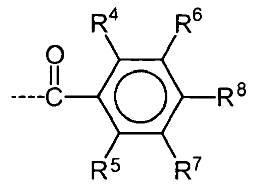 oder H oder eine der für R³ angegebenen Bedeutungen; wobei
R⁴, R⁵ unabhängig voneinander jeweils H, Halogen, ein verzweigter oder linearer C₁₋₆-Alkyl- oder -O-C₁₋₆- Alkylrest sind;
R⁶, R⁷, R⁸ unabhängig voneinander jeweils H, Halogen, ein verzweigter, cyclischer oder vorzugsweise linearer C₁₋₂₀-Alkyl-, -Alkenyl-, -Alkyloxy- oder -Alkenoxy- Rest sind, der durch ein oder mehrere O, S oder -NR'- unterbrochen und durch eine oder mehrere polymerisa- tionsfähige Gruppen und/oder Reste R⁹ substituiert sein kann, wobei R' H, Halogen, ein verzweigter, cy- clischer oder vorzugsweise linearer C₁₋₂₀-Alkyl-, -Alkenyl-, -Alkyloxy- oder -Alkenoxy-Rest ist;
- R³: ein verzweigter oder vorzugsweise linearer C₁₋₁₈-Alkyl-Rest oder C₂₋₁₈-Alkenyl-Rest, wobei diese Reste unsubstituiert oder einfach oder mehrfach durch einen Rest substituiert sein können, der aus der folgenden Gruppe ausgewählt ist: Halogen, CN, 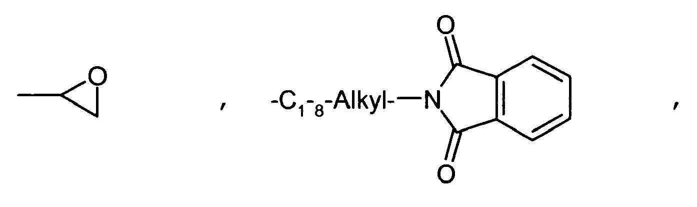 -OR¹⁰, -SR¹⁰, -OCO-R¹⁰, -COO-R¹⁰, -CH=CH-CO-OR¹⁰, -C(C₁₋₄- Alkyl) =C (C₁₋₄-Alkyl) -CO-OR¹⁰, -CO-R¹³, -CO-CH=CH-CO-C₁₋₆- Alkyl, -CO-CH=CH-CO-Phenyl, -CO-CH=CH-COO-C₁₋₁₈-Alkyl, -NR¹¹R¹², -N(R¹¹)-CO-R¹⁰, -N (R¹¹)-COO-R¹⁰, -N(R¹¹)-CO-NR¹¹R¹², - N(R¹¹)-CO-Hal, -CO-NR¹¹R¹², -SO₂-R¹⁰, -SO₂-OR¹⁰, -SO₂-NR¹¹R¹², -PO(OC₁₋₈-Alkyl)₂, -SiR¹⁴R¹⁵R¹⁶, -CH=CH-Phenyl, -C(C₁₋₄- Alkyl)=C(C₁₋₄-Alkyl)-Phenyl, Phenyl-C₁₋₄-Alkyl, Phenyl, Naphtyl, Biphenyl, C₅₋₁₂-Cycloalkyl, ein 5- oder 6- gliedriger O-, S- oder N-haltiger heterocyclischer Ring, Benzophenonyl, Thisanthonyl, wobei
R¹⁰ H, C₁₋₁₈-Alkyl, C₂₋₁₈-Alkenyl, C₂₋₁₈-Alkenyl, das durch ein oder mehrere Sauerstoffatome unterbrochen ist, C₃₋₁₂-Cycloalkyl, Tetrahydropyran-2-yl, Phenyl-C₁₋₄- Alkylen, Phenyl-C₁₋₄-Alkenylen, C₁₋₆-Alkyl, das unsub- stituiert oder durch Halogen, Cyclohexyl, Cyclopen- tyl, Tetrahydrofuranyl, Furanyl oder Isopropyl-4- methyl-cyclohexyl substituiert sein kann, Phenyl, Naphthyl oder Biphenyl ist, wobei diese Ringsysteme unsubstituiert oder durch 1 bis 5 Halogenatome, C₁₋₈- Alkyl-, C₁₋₈-Alkoxy- und/oder C₁₋₈-Alkylthioreste sub- stituiert sein können,
R¹¹, R¹² unabhängig voneinander H, C₁₋₁₈-Alkyl, C₂₋₁₈- Alkenyl, C₂₋₁₈-Alkenyl, das durch ein oder mehrere Sauerstoffatome unterbrochen ist, C₃₋₁₂-Cycloalkyl, Phenyl-C₁₋₄-Alkyl, Phenyl, Naphthyl oder Pyridyl sind, wobei diese Ringsysteme unsubstituiert oder durch 1 bis 5 Halogenatome, C₁₋₈-Alkyl-, C₁₋₈-Alkoxy- und/oder C₁₋₈-Alkylthioreste substituiert sein können, oder R¹¹ und R¹² bilden zusammen einen 5- oder 6-gliedrigen O-, S- oder N-haltigen heterocyclischen Ring, der sei- nerseits mit einem aliphatischen oder aromatischen Ring anelliert sein kann,
R¹³ C₁₋₁₈-Alkyl, C₂₋₁₈-Alkenyl, das durch ein oder mehrere O-Atome unterbrochen ist, C₃₋₁₂-Cycloalkyl, Phenyl-C₁₋₄- ₄-Alkyl, Phenyl, Naphthyl oder Biphenyl ist, wobei die genannten Ringsysteme unsubstituiert oder durch 1 bis 5 C₁₋₈-Alkyl-, C₁₋₈-Alkoxy-, C₁₋₈-Alkylthioreste und/oder Halogenatome substituiert sein können;
R¹⁴, R¹⁵, R¹⁶ unabhängig voneinander jeweils H, C₁₋₈- Alkyl, C₂₋₈-Alkenyl, C₇₋₉-Phenylalkyl, -O-C₁₋₈-Alkyl, Phenyl oder -O-SiR¹⁷R¹⁸R¹⁹ sind, wobei R¹⁷, R¹⁸, R¹⁹ unabhängig voneinander jeweils H, C₁₋₈- Alkyl, C₂₋₈-Alkenyl, C₇₋₉-Phenylalkyl, -O-C₁₋₈-Alkyl oder Phenyl sind, und wobei R¹⁰, R¹¹ und R¹² wie oben definiert sind; oder
- R³: ist ein verzweigter oder vorzugsweise linearer C₂₋₁₈-Alkyl- Rest oder ein C₂₋₁₈-Alkylen-Rest, der einfach oder mehrfach durch -O-, -NH-, -NR¹¹-, -S- unterbrochen ist, wobei die Reste unsubstituiert oder einfach oder mehrfach durch ei- nen Rest substituiert sein können, der aus der folgenden Gruppe gewählt ist: Halogen, CN, -OR¹⁰, -SR¹⁰, -OCO-R¹⁰, -COO-R¹⁰, -NR¹¹R¹², -N (R¹¹)-CO-R¹⁰, -N (R¹¹)-COO-R¹⁰, -N (R¹¹)-CO-NR¹¹R¹², -N(R¹¹)- CO-Hal, -CO-NR¹¹R¹², -SO₂-R¹⁰, -SO₂-OR¹⁰, -SO₂-NR¹¹R¹², -PO(OC₁₋₈-Alkyl)₂, -SiR¹⁴R¹⁵R¹⁶, Phenyl-C₁₋₄-Alkyl, Phenyl, C₅₋₁₂ -Cycloalkyl; wobei R¹⁰, R¹¹, R¹², R¹⁴, R¹⁵ und R¹⁶ wie oben definiert sind;
oder
- R³: ist ein verzweigter oder vorzugsweise linearer C₂₋₁₈-Alkyl- Rest oder ein C₂₋₁₈-Alkylen-Rest, der einfach oder mehrfach durch -CO-, -COO-, -OCO-, -OCOO-, -CO-N(R¹²)-, -N(R¹²)-CO-, -N (R¹²)-CO-N(R¹²) -, -N(R¹²)-COO-, -COO-C₁₋₆-Alkylen, -COS-C₁₋₁₈-Alkylen, -SO₂-, -SO₂-O-, -SO₂-N (R¹²) -, -(CH₃)₂Si[OSi(CH₃)₂]_{q}-, mit q = 1 bis 6; Phenyl-C₁₋₄- Alkylen, Phenylen, Naphthylen, Biphenylen, C₅₋₁₂- Cycloalkylen oder einen 5- oder 6-gliedrigen O-, S- oder N-haltigen heterocyclischen Ring unterbrochen ist;
wobei R¹² wie oben definiert ist;
oder
- R³: ist Trimethylsilyl, Hal-(CH₃)₂Si-[OSi(CH₃)₂]ᵣ-, (CH₃)₃Si- [OSi(CH₃)₂]ᵣ- mit r = 1 bis 6, -COOH, -COO-R¹⁰, -CO-NR¹¹R¹², -CO-Vinyl, -CO-Phenyl, wobei der Phenylrest unsubstituiert oder durch -CH₃, -OCH₃ und/oder -Cl substituiert sein kann;
wobei R¹⁰, R¹¹ und R¹² wie oben definiert sind;
oder
- R³: ist Phenyl-C₁₋₄-Alkyl, Phenyl, Naphthyl oder Biphenyl, C_{5-***}-Cycloalkyl oder ein 5- oder 6-gliedriger O-, S- oder N- haltiger heterocyclischer Ring, wobei diese Ringsysteme unsubstituiert oder durch 1 bis 5 Halogenatome, C₁₋₈-Alkyl-, C₁₋₈-Alkoxy-, C₁₋₈-Alkylthioreste und/oder -NR¹¹R¹² substi- tuiert sein können, wobei R¹¹ und R¹² wie oben definiert sind und
- m: 0 oder 1,
- n: 0 oder 1,
- p: 0 oder 1,
- z: 2 bis 10, vorzugsweise 2 bis 6 und insbesondere 2, 3 oder 4 ist.

Gemäß einer besonders bevorzugten Ausführungsform hat R³ der Formeln (I) und (I') die folgende Bedeutung:
- R³: ein aromatischer C₆₋₁₀-Rest, der durch einen verzweigten, oder vorzugsweise linearen C₁₋₁₈-Alkyl-, -Alkenyl-, -Alkoxy- oder -Alkenoxy-Rest substituiert sein kann, wobei die genannten Reste durch ein oder mehrere O-Atome unterbrochen sein können, oder ein verzweigter oder vorzugsweise linearer C₁₋₁₈-Alkyl-, -Alkenyl-, -Alkoxy- oder -Alkenoxy-Rest ist, der ein- oder mehrfach durch O - unterbrochen sein kann.

Gemäß einer ganz besonders bevorzugten Ausführungsform ist R³ C₁₋₄-, insbesondere C₁-, C₂- und/oder C₃-Alkyl.

Im Fall der Formel (I') sind als Rest R⁰ Alkylgruppen mit 1 bis 6 Kohlenstoffatomen besonders bevorzugt, wobei z Wasserstoffatome dieser Gruppen durch den Klammerausdruck der Formel (I') substituiert sind. Lineare Alkylgruppen sind besonders bevorzugt. Bevorzugte aromatische Kohlenwasserstoffreste sind Benzolreste, die durch den Klammerausdruck z-fach substituiert sind. Bevorzugte aliphatisch-aromatische Kohlenwasserstoffreste werden durch Kombination der bevorzugten aliphatischen und aromatischen Gruppen erhalten. Besonders zu nennen sind Kombinationen aus zwei oder' mehr Benzolringen, vorzugsweise 2 bis 3 Benzolringen und einer aliphatischen Gruppe. Die Verknüpfung mit dem Klammerausdruck erfolgt vorzugsweise über Benzolreste. Die genannten Reste können ein oder mehrere Sauerstoffatome enthalten, wobei vorzugsweise aliphatische Kohlenstoffatome durch Sauerstoffatome ersetzt werden. Die erfindungsgemäßen Germaniumverbindungen der Formel (I') sind vorzugsweise symmetrisch aufgebaut, so daß die Zahl an Sauerstoffatomen oder anderen Heteroatomen vorzugsweise der Anzahl der Gruppen in runden Klammern also z oder einem vielfachen davon, vorzugsweise z entspricht.

Gemäß einer Ausführungsform der Erfindung sind demnach Zusammensetzungen bevorzugt, die mindestens eine Verbindung gemäß der Formel (I') enthalten, 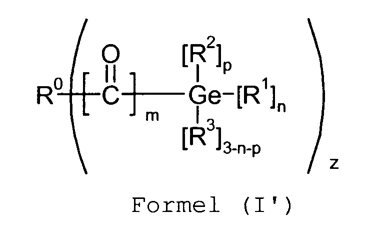 in der
- R⁰: eine Alkylgruppe mit 1 bis 6 Kohlenstoffatomen ist, wobei z Wasserstoffatome dieser Gruppe durch den Klammerausdruck der Formel (I') substituiert sind,
- R¹, R²: unabhängig voneinander 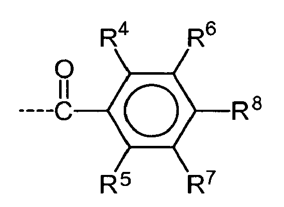 oder H sind oder eine der für R³ angegebenen Bedeutungen haben, wobei
- R⁴, R⁵: unabhängig voneinander jeweils H, Halogen, ein ver- zweigter oder linearer C₁₋₆-Alkyl- oder -O-C₁₋₆-Alkylrest sind;
- R⁶, R⁷, R⁸: unabhängig voneinander jeweils H, Halogen, ein ver- zweigter, cyclischer oder vorzugsweise linearer C₁₋₂₀- Alkyl-, -Alkenyl-, -Alkyloxy- oder -Alkenoxy-Rest sind, der durch ein oder mehrere O, S oder -NR'- unterbrochen und durch eine oder mehrere polymerisationsfähige Gruppen und/oder Reste R⁹ substituiert sein kann, wobei R' H, Ha- logen, ein verzweigter, cyclischer oder vorzugsweise li- nearer C₁₋₂₀-Alkyl-, -Alkenyl-, -Alkyloxy- oder -Alkenoxy- Rest ist;
- R³: ein aromatischer C₆₋₁₀-Rest ist, der durch einen verzweigten, oder vorzugsweise linearen C₁₋₁₈-Alkyl-, -Alkenyl-, -Alkoxy- oder -Alkenoxy-Rest substituiert sein kann, wobei die genannten Reste durch ein oder mehrere O- Atome unterbrochen sein können, oder ein verzweigter oder vorzugsweise linearer C₁₋₁₈-Alkyl-, -Alkenyl-, -Alkoxy- oder -Alkenoxy-Rest ist, der ein- oder mehrfach durch O - unterbrochen sein kann,
- m: 0 oder 1,
- n: 0 oder 1,
- p: 0 oder 1,
- z: 2 bis 6 ist.

Erfindungsgemäß besonders bevorzugte Verbindungen der Formel (I) lassen sich durch die folgende Formel (II) darstellen.: 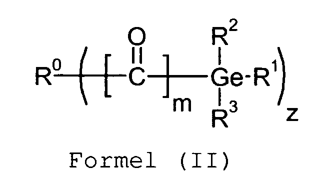 in der
- R¹, R², R³: unabhängig voneinander 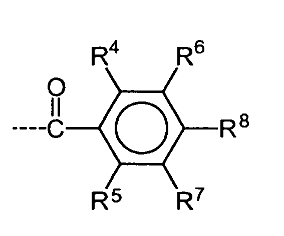 ein C₁₋₃-Alkylrest oder eine C₁₋₃-Acylgruppe wie z.B. eine Acetylgruppe sind;
- R⁴, R⁵: unabhängig voneinander jeweils H, Halogen, ein ver- zweigter oder linearer C₁₋₆-Alkyl- oder -O-C₁₋₆- Alkylrest sind;
- R⁶, R⁷, R⁸: unabhängig voneinander jeweils H, Halogen, ein ver- zweigter, cyclischer oder vorzugsweise linearer C₁₋₂₀- Alkyl-, -Alkenyl-, -Alkyloxy- oder -Alkenoxy-Rest sind, der ein- oder mehrfach durch O, S oder -NR²⁰- unterbrochen und durch eine oder mehrere polymerisa- tionsfähige Gruppen und/oder Reste R⁹ substituiert sein kann; wobei
- R⁹: -OH, -CₓF₂ₓ₊₁ mit x = 1 bis 20, - [Si(CH₃)₂]_{y}-CH₃ mit y = 1 bis 20 ist; und
- R²⁰: H, Halogen, ein verzweigter, cyclischer oder vorzugs- weise linearer C₁₋₂₀-Alkyl-, -Alkenyl-, -Alkyloxy- oder -Alkenoxy-Rest ist und
m die oben angegebene Bedeutung hat.

Weiter bevorzugt sind Germanium-Verbindungen gemäß der folgenden Formel (III) 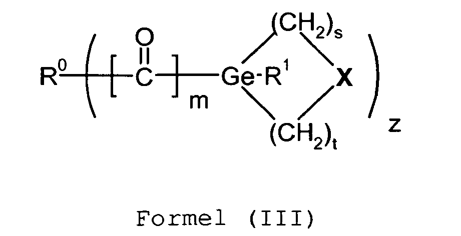 in der
- R¹: 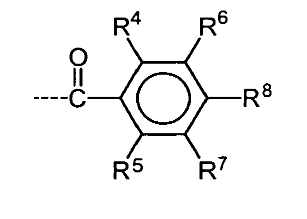 oder
ein C₁₋₃-Alkylrest oder eine C₁₋₃-Acylgruppe wie z.B. eine Acetylgruppe ist;
- s,t: unabhängig voneinander eine ganze Zahl von 0 bis 6, vorzugsweise 1 bis 3 sind, wobei s und t so gewählt sind, daß die Summe der Ringatome einschließlich des Germaniumatoms 5 bis 8 beträgt, und
- X: N-R²¹, O, S oder entfällt bedeutet, wobei R²¹ H oder C₁₋₁₀-Alkyl, vorzugsweise H oder C₁₋₄-Alkyl ist und
R⁰ und m die oben angegebenen Bedeutungen haben.

Der Germanium enthaltende Ring kann mit einem oder mehreren, vorzugsweise 1 oder 2 aliphatischen oder aromatischen Ringen anelliert und unsubstituiert oder ein- oder mehrfach, vorzugsweise ein- oder zweifach substituiert sein, wodurch die Anzahl der Wasserstoffatome des Rings entsprechend reduziert wird. Bevorzugte Substituenten sind C₁₋₄-Alkylgruppen oder =O. Bevorzugt sind Verbindungen der Formel (III) in denen der Germanium enthaltende Ring nicht mit weiteren Ringen anelliert und abgesehen von R²¹ unsubstituiert ist.

Weiter bevorzugt sind Germanium-Verbindungen gemäß den folgenden Formeln (IV) und (V) 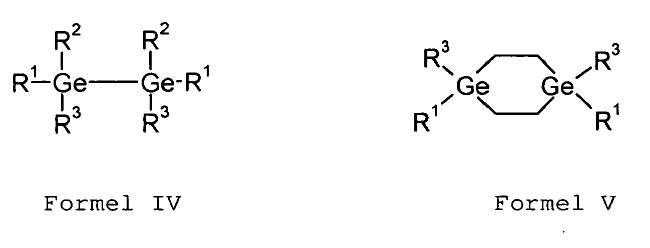 in denen R¹, R² und R³ die oben angegebenen Bedeutungen haben.

Außerdem bevorzugt sind Germanium-Verbindungen gemäß der folgenden Formel (VI): 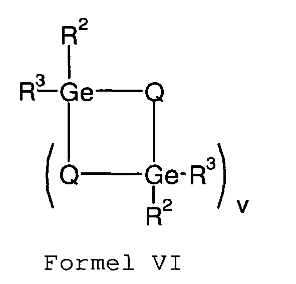 in der
- R², R³: unabhängig voneinander 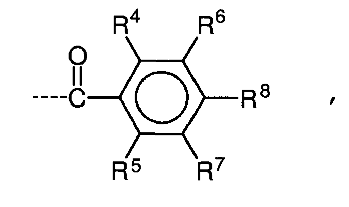 ein C₁₋₃-Alkylrest oder eine C₁₋₃-Acylgruppe wie z.B. eine Acetylgruppe sind;
- v: eine ganze Zahl von 1 bis 3, vorzugsweise 1 bis 2 ist, und
- Q: N-R²², -CH₂-, -CH=CH-, -CH₂-CH₂- oder -O-Si(R²³)₂-O- bedeutet, wobei R²² H, C₁₋₁₀-Alkyl, vorzugsweise H oder C₁₋₄-Alkyl, oder Phenyl ist, und R²³ C₁₋₁₀-Alkyl, vorzugsweise C₁₋₄-Alkyl ist oder zwei Reste R²³ eine Sauerstoffbrücke (-O-) zwischen zwei Si-Atomen bilden.

In allen erfindungsgemäßen Acylgermanium-Verbindungen der obigen Formeln sind jeweils 1 bis 3 Acylgruppen an jedes Germaniumatom gebunden, wobei Germanium-Verbindungen mit 1 oder 2 Acylgruppen pro Germaniumatom bevorzugt sind. Die erfindungsgemäßen Acylgermanium-Verbindungen zeichnen sich somit dadurch aus, daß sie mindestens zwei Germaniumatome enthalten, die jeweils mindestens eine Acylgruppe aufweisen.

Durch die Formel (I) und die übrigen hierin gezeigten Formeln werden sämtliche stereoisomere Formen sowie Gemische verschiedener stereoisomerer Formen, wie z.B. Racemate, erfaßt. Die Formeln erfassen nur solche Verbindungen, die mit der chemischen Valenzlehre vereinbar sind.

Der Hinweis, daß ein Rest durch ein Heteroatom wie O unterbrochen sein kann, ist so zu verstehen, daß die O-Atome in die Kohlenstoffkette des Restes eingeschoben werden, d.h. beidseitig von Kohlenstoffatomen begrenzt werden. Die Anzahl der Heteroatome ist daher um mindestens 1 kleiner als die Zahl der Kohlenstoffatome, und die Heteroatome können nicht endständig sein.

Bei Kohlenwasserstoffresten, die Kohlenstoff- und Heteroatome enthalten, ist die Anzahl der Heteroatome ohne Berücksichtigung von Substituenten stets kleiner als die Zahl der Kohlenstoffatome.

Halogen (abgekürzt Hal) steht vorzugsweise für F, Cl, Br oder I, insbesondere für F, Cl, ganz besonders bevorzugt für Cl.

Alkyl steht, auch wenn dies nicht ausdrücklich angegeben wird, für lineare und verzweigte Gruppen, wobei lineare Alkyl-Reste in allen Fällen bevorzugt sind.

Bevorzugte polymerisationsfähige Gruppen, die bei den obigen Resten als Substituenten vorhanden sein können, sind Vinyl, Styryl, (Meth)acrylat, (Meth)acrylamid und/oder N-Alkylacrylamid, besonders bevorzugt (Meth)acrylat, (Meth)acrylamid und/oder N-Alkylacrylamid. Vorzugsweise sind die Reste R², R³, R⁶, R⁷ und R⁸ mit 0 bis 3, insbesondere 0 bis 1 polymerisierbaren Gruppen substituiert. Die polymerisationsfähigen Gruppen sind vorzugsweise endständig angeordnet.

Erfindungsgemäß sind solche Verbindungen der allgemeinen Formeln (I'), (II) und (IV) bevorzugt, bei denen die Variablen die folgenden Bedeutungen haben, die unabhängig voneinander gewählt werden können:
- R¹: 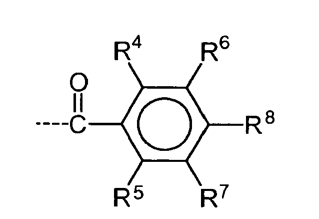 oder H, oder eine der
für R² und R³ angegebenen Bedeutungen;
- R², R³: unabhängig voneinander ein linearer C₁₋₄-Alkyl- oder -Alkenyl-Rest, der durch eine oder mehrere polymeri- sationsfähige Gruppen substituiert sein kann, oder eine C₁₋₃-Acylgruppe wie z.B. eine Acetylgruppe;
- R⁴, R⁵: unabhängig voneinander jeweils H, Halogen, ein ver- zweigter oder linearer C₁₋₄-Alkyl- oder -O-C₁₋₄- Alkylrest, insbesondere H;
- R⁶, R⁷, R⁸: unabhängig voneinander jeweils H, Halogen, ein linea- rer C₁₋₂₀-Alkyl-, -Alkenyl-, -Alkoxy- oder -Alkenoxy- Rest, der durch ein oder mehrere -O-, -S- oder -NR²⁰- Reste unterbrochen und durch ein oder mehrere polyme- risationsfähige Gruppen substituiert sein kann, ins- besondere H, und
wobei die übrigen Variablen die oben genannten Bedeutungen haben.

Besonders bevorzugte Definitionen der Variablen der allgemeinen Formeln (I'), (II) und (IV), die ebenfalls unabhängig voneinander gewählt werden können, sind:
- R¹: 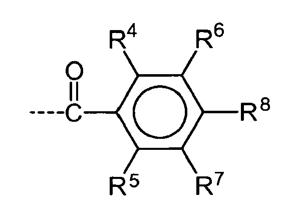 oder eine der für R² und R³
angegebenen Bedeutungen;
- R², R³: C₁-C₄-Alkyl;
- R⁴, R⁵, R⁸: H, Cl, CH₃, OCH₃;
- R⁶,R⁷: H, C₁-C₄-Alkyl, das durch ein oder mehrere O-Atome unterbrochen sein kann, und
wobei die übrigen Variablen die oben genannten Bedeutungen haben.

Ganz besonders bevorzugt sind Verbindungen der Formel (II) in denen R⁴=R⁵ und/oder R⁶=R⁷ ist.

Besonders bevorzugt sind naturgemäß solche Verbindungen, bei denen alle Variablen eine der bevorzugten und insbesondere der besonders bevorzugten Bedeutungen haben.

Konkrete Beispiele für besonders bevorzugte Verbindungen sind:

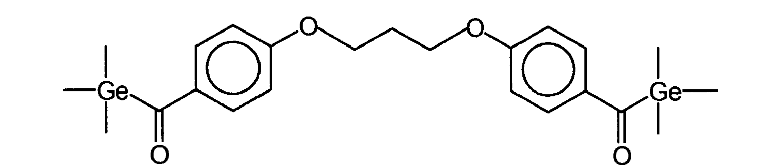

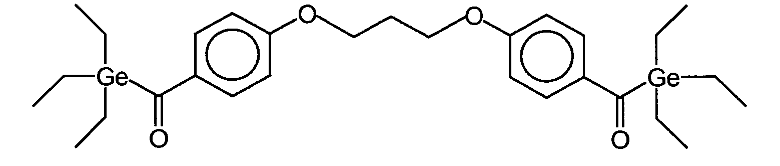

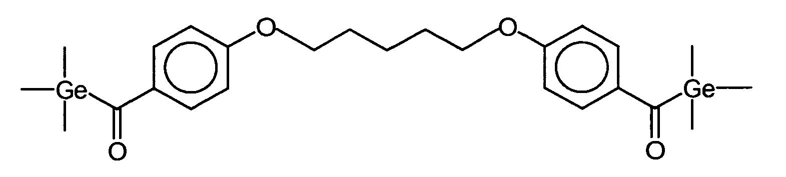

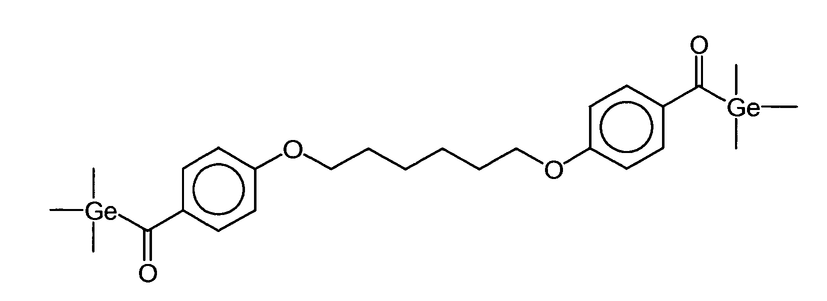

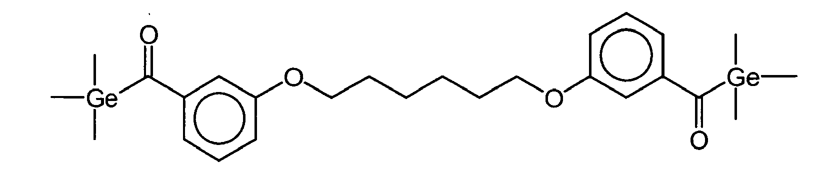

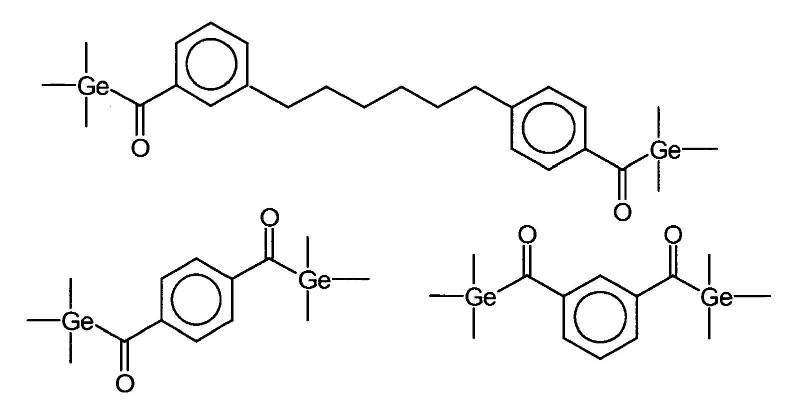

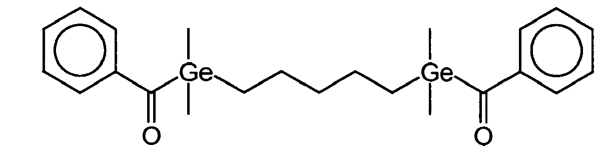

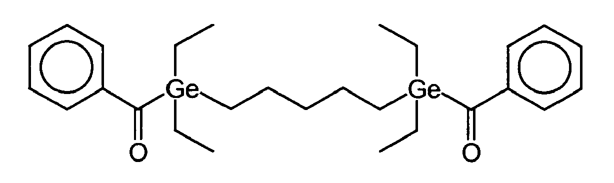

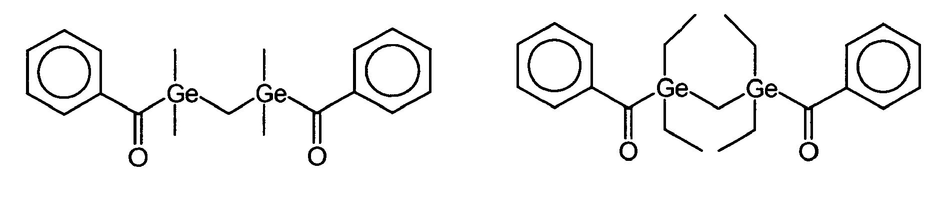

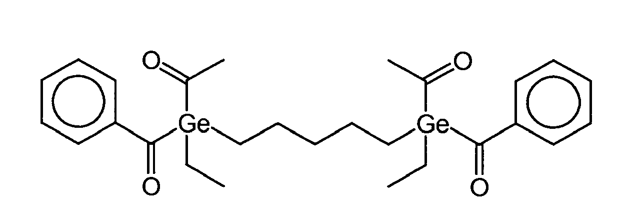

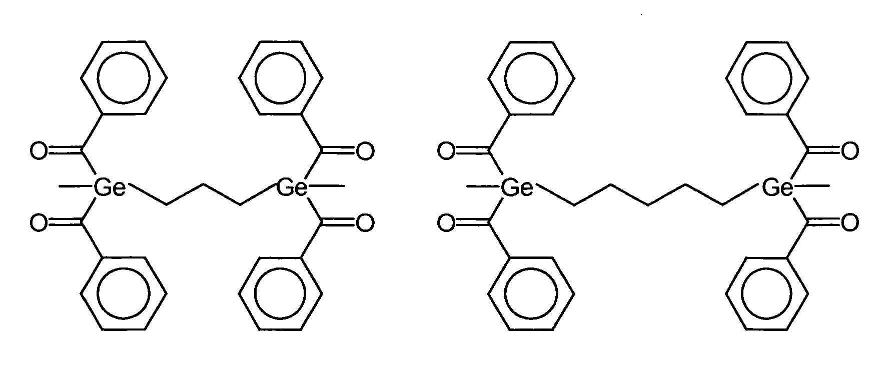

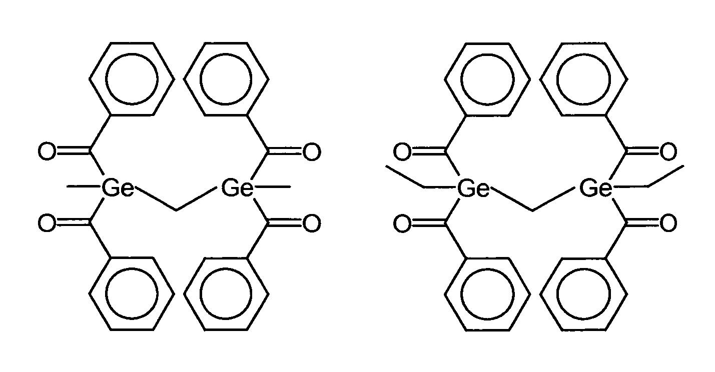

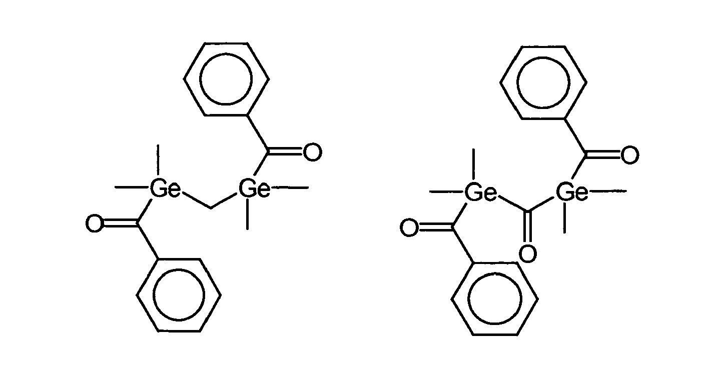

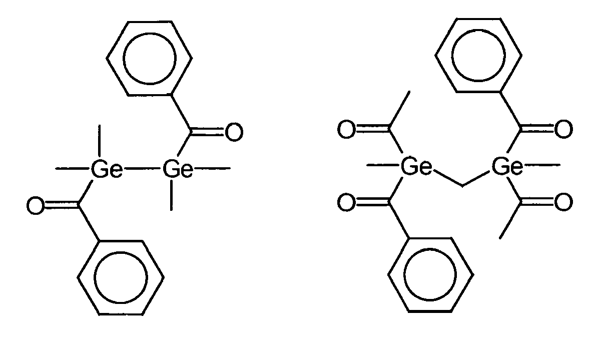

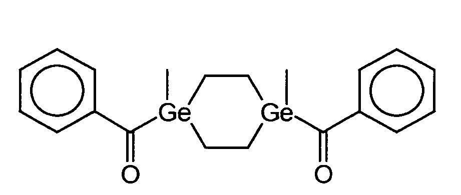

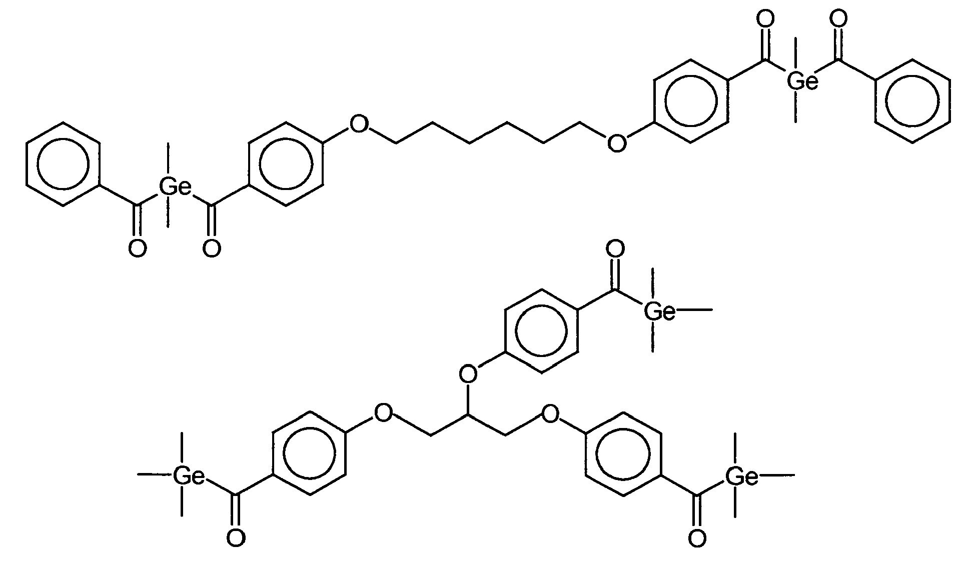

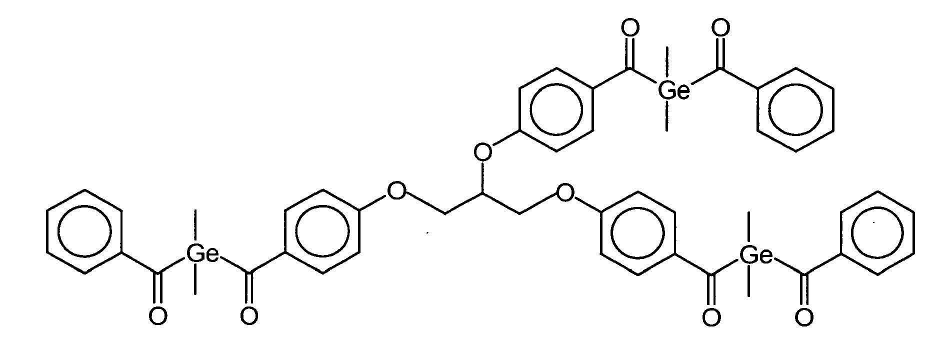

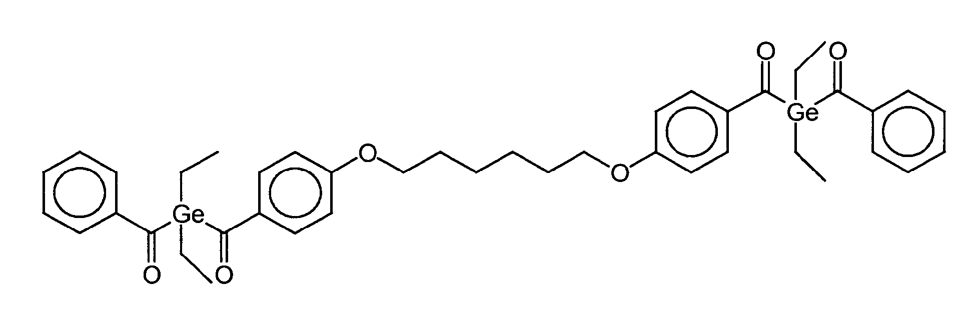

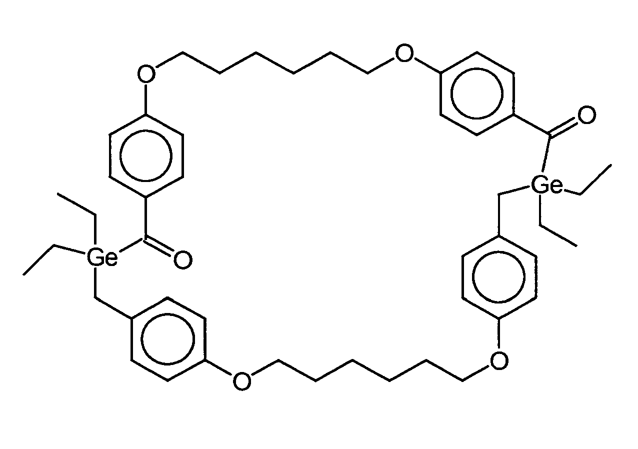

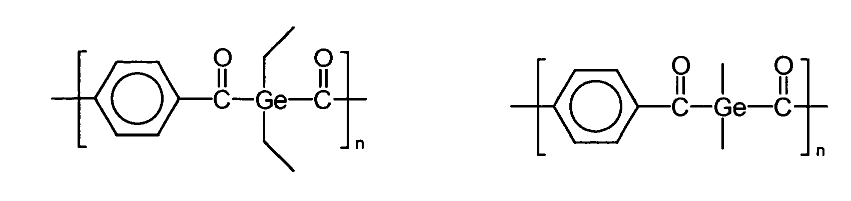

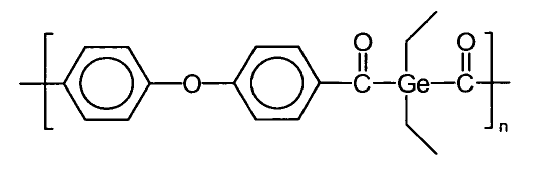

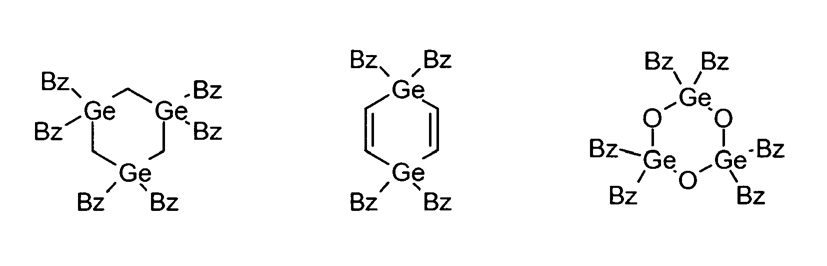

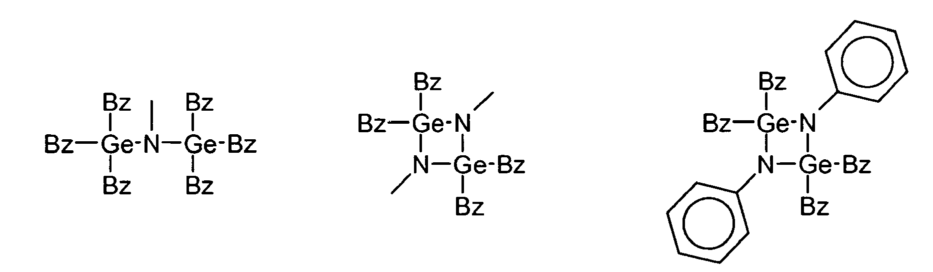

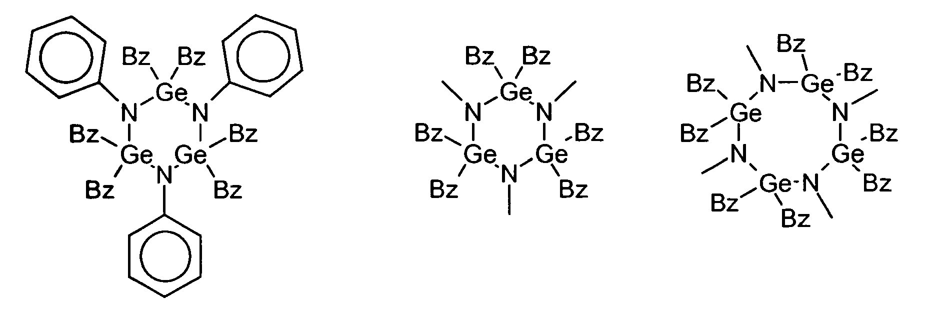

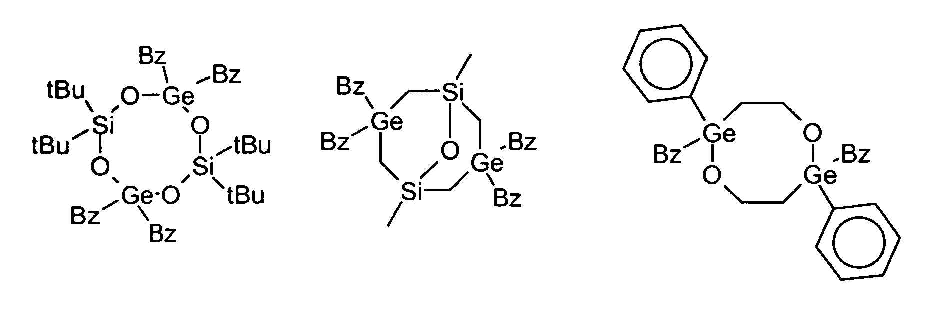

Die sich aus den oben gezeigten bevorzugten Verbindungen ableitenden Bedeutungen für die Variablen der Formeln (I) bis (VI) sind generell bevorzugt und nicht auf die konkret gezeigten Verbindungen beschränkt.

Die Synthese von Verbindungen gemäß der allgemeinen Formel (I) mit mehreren Monoacylgermaniumgruppen kann z.B. analog zu der Methode von Yamamoto et. al. (Yamamoto, K.; Hayashi, A.; Suzuki, S.; Tsuji J.; Organometallics; 6 (1987) 974) durch Umsetzung von Hexaalkyldigermanium mit Säurechlorid erfolgen:

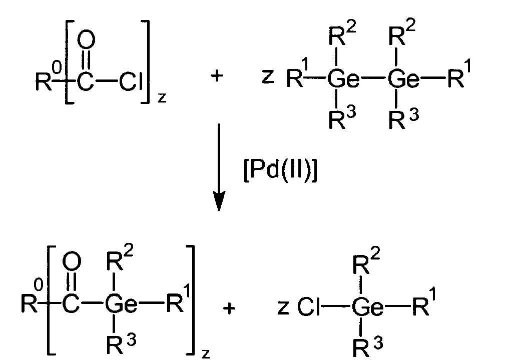

### Konkretes Beispiel:

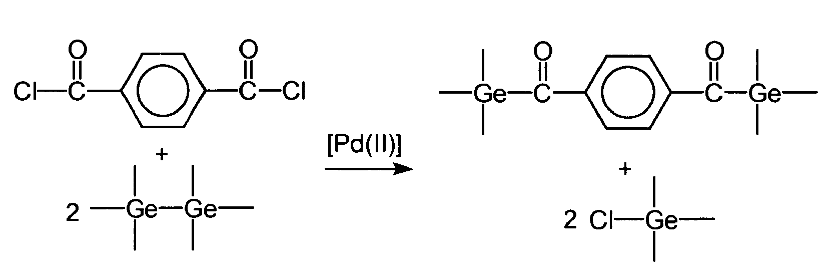

Eine Möglichkeit zur Herstellung von Verbindungen gemäß der allgemeinen Formel (I) mit mehreren Bisacylgermaniumgruppen oder von oligomeren bzw. polymeren Verbindungen, ist die Umsetzung der entsprechenden lithierten Germaniumverbindungen mit Säurechloriden nach Castel et. al. (Castel, A.; Riviere, P.; Satgé, J.; Ko, H.Y.; Organometallics; 9 (1990) 205):

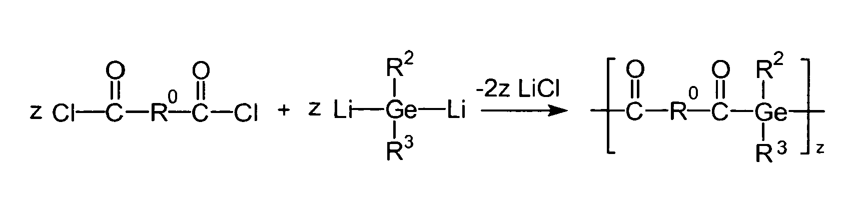

### Konkretes Beispiel:

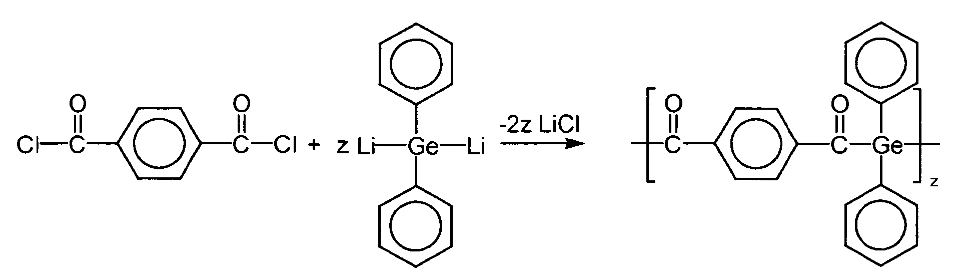

Die Herstellung von lithierten aromatischen Germaniumverbindungen kann z.B. durch Reaktion des entsprechenden Germaniumhalids (X = Halogen) mit Lithium (Li) (Nishimura, T.; Inoue-Ando, S.; Sato, Y., J. Chem. Soc., Perkin Trans.1; (1994) 1589) oder des Hydrogermaniums mit n-Butyllithium (BuLi) erfolgen (Castel, A.; Riviere, P.; Satgé, J.; Ko, H.Y.; Organometallics; 9 (1990) 205):

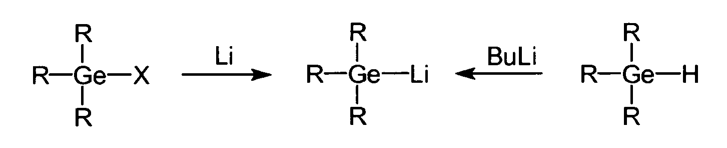

Des weiteren lassen sich Mono- und Bisacylgermane synthetisieren, indem ein Carbanion, welches aus 1,3-Dithianen erhalten wird, mit Germaniumchloriden nach Brook et. al. umgesetzt wird (Brook, A. G.; Duff, J. M.; Jones, P. F.; Davis, N. R.; "Synthesis of Silyl and Germyl Ketones" J. Am Chem. Soc. 89(2), 431 - 434 (1967)). Dieser Syntheseweg eignet sich besonders zur Herstellung von Bisalkylbisacylgermanen:

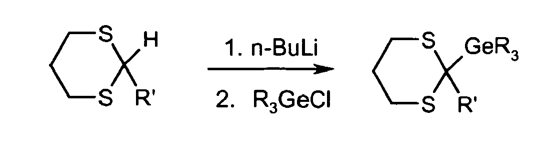

Die erhaltenen Dithiane können nach Methoden, die dem Fachmann allgemein bekannt sind, zu den entsprechenden Ketonen hydrolysiert werden (nach Brook, A. G.; Duff, J. M.; Jones, P. F.; Davis, N. R.; "Synthesis of Silyl and Germyl Ketones" J. Am Chem. Soc. 89(2), 431-434 (1967) oder z.B. auch nach Sharma, H.K.; Cervanes-Lee, F.; Pannel, K. H.; "Organometalloidal derivatives of the transition metals, XXVII. Chemical and structural investigations on (ferrocenylacyl)germanes)", J. Organomet. Chem. 409, 321-330 (1991):

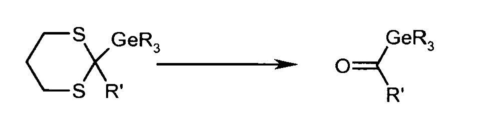

Die Acylgermanium-Verbindungen der allgemeinen Formel (I) eignen sich besonders als Photoinitiatoren für die Polymerisation, insbesondere als Initiatoren für die radikalische Polymerisation, Photoaddition und für die Thiol-En-Reaktion (Polyaddition). Es wurde gefunden, daß mit diesen Initiatoren bei Bestrahlung mit Licht im Vergleich zu herkömmlichen Photoinitiatoren eine hohe Durchhärtungstiefe erzielt werden kann, ohne daß die Initiatoren zu gefärbten Materialien führen. Dies ist bei vielen technischen und besonders medizinischen Werkstoffen, wie z.B. Dentalwerkstoffen und Knochenzementen von großem Vorteil.

Darüber hinaus zeichnen sich die erfindungsgemäß verwendeten Acylgermanium-Verbindungen der Formel (I) im Vergleich zu herkömmlichen Initiatoren durch eine geringere Zytotoxizität aus, was für medizinische Anwendungen ein besonderer Vorteil ist. Die Acylgermanium-Verbindungen eignen sich daher beispielweise als Initiatoren für Materialien zur Herstellung von Kontaktlinsen aber auch von gewöhnlichen optischen Linsen, die von der geringen Verfärbungsneigung der Initiatoren profitieren.

In diesem Zusammenhang ist auch von Bedeutung, daß sich die Initiatoren der Formel (I) durch eine ungewöhnlich hohe Aktivität auszeichnen, so daß die Zugabe geringer Konzentrationen zur Härtung ausreichend ist.

Die Verwendung der Initiatoren der Formel (I) ist nicht auf medizinische Anwendungen beschränkt. Die große Durchhärtungstiefe bei der Härtung mit Licht im sichtbaren Wellenlängenbereich ist auch bei technischen Anwendungen ein erheblicher Vorteil. Die erfindungsgemäßen Zusammensetzungen eignen sich für eine Vielzahl von Anwendungszwecken, wie zum Beispiel als Druck- oder Anstrichfarben, Lacke, Adhäsive, zur Herstellung von Druckplatten, integrierten Schaltkreisen, Photoresists, Lötmasken, Tinten für Farbdrucker, als Materialien für die holographische Datenspeicherung, zur Herstellung von nanodimensionierten mikroelektromechanischen Elementen, Lichtwellenleitern, Formteilen und zur optischen Herstellung von Informationsträgern.

Die erfindungsgemäßen Acylgermanium-Verbindungen lassen sich mit Licht, vorzugsweise mit UV-Vis-Licht, besonders bevorzugt mit sichtbarem Licht (400-800 nm) und insbesondere mit Licht einer Wellenlänge von 400 bis 500 nm aktivieren. Zur Initiierung der Polymerisation werden die Acylgermanium-Verbindungen der Formel (I) daher vorzugsweise mit Licht im Wellenlängenbereich von 200 bis 750 nm, besonders bevorzugt 200 bis 550 nm, weiter bevorzugt 300 bis 550 nm und ganz besonders bevorzugt 350 bis 500 nm bestrahlt. Sie sind damit als Initiatoren für die Laser-Härtung und die Laser induzierte 3D Härtung sowie für die 2-Photonen Polymerisation brauchbar. Sie eignen sich insbesondere als Initiatoren für pigmentierte Systeme.

Besonders vorteilhaft ist, daß die Initiatoren auch mit LED Lichtquellen aktiviert werden können. Die Wellenlänge von LEDs ist abhängig von der Gitterkonstante des Substrates. Die Qualität (thermische Festigkeit, Wärmeausdehnung, Konstanz der Atomabstände etc.) des Substrats bestimmt die Höhe der möglichen Leistung der LEDs. In der intra-oralen Anwendung sind Wellenlängen erst ab etwa 380 nm erlaubt, so daß Initiatoren der Formel (I), die sich mit einer Wellenlänge im Bereich von 380 nm und mehr aktivieren lassen, besonders bevorzugt sind.

Gegenstand der Erfindung sind auch Kombinationen von LED-Lichtquellen mit Initiatoren gemäß der Formel (I) oder mit Zusammensetzungen, die einen solchen Initiator enthalten. Zur dentalen Anwendung sind Systeme von LED-Lichtquellen mit einer Wellenlänge im Bereich von 400 bis 550 nm, vorzugsweise 400 bis 480 nm und insbesondere 450 ± 20 nm, und darauf abgestimmten Initiatoren bzw. Zusammensetzungen bevorzugt, d.h. Initiatoren mit einer Aktivierungswellenlänge im Bereich von 400 bis 550 nm, vorzugsweise 400 bis 480 nm und ganz besonders bevorzugt ca. 450 ± 20 nm und diese enthaltende Zusammensetzungen. Daneben sind LED-Lichtquellen mit einer Wellenlänge von etwa 650 ± 30 nm oder etwa 360 ± 30 nm zusammen mit darauf abgestimmten Initiatoren oder Zusammensetzungen erfindungsgemäß bevorzugt.

Die erfindungsgemäßen Zusammensetzungen enthalten neben mindestens einer Acylgermanium-Verbindung der Formel (I) vorzugsweise auch ein polymerisierbares Bindemittel. Bevorzugt sind Bindemittel auf der Basis radikalisch und/oder kationisch polymerisierbarer Monomere und/oder Prepolymere.

Als radikalisch polymerisierbare Bindemittel eignen sich besonders mono- oder multifunktionelle (Meth)acrylate oder deren Mischung. Unter monofunktionellen (Meth)acrylverbindungen werden Verbindungen mit einer, unter mehrfach funktionellen (Meth)acrylverbindungen Verbindungen mit zwei oder mehr, vorzugsweise 2 bis 3 polymerisierbaren Gruppen verstanden.

Diesbezügliche Beispiele sind Methyl-, Ethyl-, Hydroxyethyl-, Butyl-, Benzyl-, Tetrahydrofurfuryl- oder Isobornyl-(meth)acrylat, Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat und 2,2,4-Trimethylhexamethylen-diisocyanat), Di-, Tri- oder Tetraethylenglycoldi(meth)-acrylat, Trimethylolpropantri(meth)acrylat, Pentaerythrittetra(meth)acrylat, sowie Glycerindimethacrylat, 1,4-Butandioldi(meth)acrylat, 1,10-Decandioldi(meth)acrylat oder 1,12-Dodecandioldi(meth)acrylat. Zusammensetzungen, die mindestens ein radikalisch polymerisierbares Monomer mit 2 oder mehr, vorzugsweise 2 bis 3 radikalisch polymerisierbaren Gruppen enthalten, sind besonders bevorzugt. Mehrfach funktionelle Monomere haben vernetzende Eigenschaften.

Als radikalisch polymerisierbare Bindemittel lassen sich auch hydrolysestabile Monomere, wie hydrolysestabile Mono(meth)acrylate, z.B. Mesitylmethacrylat oder 2-(Alkoxymethyl)acrylsäuren, z.B. 2-(Ethoxymethyl)acrylsäure, 2-(Hydroxymethyl)acrylsäure, N-mono- oder -disubstitiuierte Acrylamide, wie z.B. N-Ethylacrylamid, N,N-Dimethylacrylamid, N-(2-Hydroxyethyl)acrylamid, N-Methyl-N-(2-hydroxyethyl)-acrylamid oder N-monosubstituierte Methacrylamide, wie z.B. N-Ethylmethacrylamid oder N-(2-Hydroxyethyl)methacrylamid sowie außerdem N-Vinylpyrrolidon oder Allylether einsetzen. Bevorzugte Beispiele für hydrolysestabile Vernetzermonomere sind Urethane aus 2-(Hydroxymethyl)acrylsäure und Diisocyanaten, wie 2,2,4-Trimethylhexamethylendiisocyanat oder Isophorondiisocyanat, vernetzende Pyrrolidone, wie z.B. 1,6-Bis(3-vinyl-2-pyrrolidonyl)-hexan, oder kommerziell zugängliche Bisacrylamide wie Methylen- oder Ethylenbisacrylamid, Bis(meth)acrylamide, wie z.B. N,N'-Diethyl-1,3-bis(acrylamido)-propan, 1,3-Bis(methacrylamido)-propan, 1,4-Bis(acrylamido)-butan oder 1,4-Bis(acryloyl)-piperazin, die durch Umsetzung aus den entsprechenden Diaminen mit (Meth)acrylsäurechorid synthetisiert werden können. Bevorzugt sind bei Raumtemperatur flüssige Monomere, die als Verdünnermonomere eingesetzt werden können.

Weiterhin lassen sich als radikalisch polymerisierbare Bindemittel auch schrumpfungsarme radikalisch ringöffnend polymerisierbare Monomere wie z.B. mono- oder multifunktionelle Vinylcyclopropane oder bicyclische Cyclopropanderivate, vorzugsweise die in DE 196 16 183 C2 oder EP 1 413 569 beschriebenen, oder cyclische Allylsulfide, vorzugsweise die in US 6,043,361 und US 6,344,556 beschriebenen, einsetzen. Diese können auch in Kombination mit den voranstehend aufgeführten Di(meth)acrylat-Vernetzern verwendet werden. Bevorzugte ringöffnend polymerisierbare Monomere sind Vinylcyclopropane, wie 1,1-Di(ethoxycarbonyl)- oder 1,1-Di(methoxycarbonyl)-2-vinylcyclopropan, die Ester der 1-Ethoxycarbonyl- oder 1-Methoxycarbonyl-2-vinylcyclopropancarbonsäure mit Ethylenglycol, 1,1,1-Trimethylolpropan, 1,4-Cyclohexandiol oder Resorcin. Bevorzugte bicyclische Cyclopropanderivate sind 2-(Bicyclo[3.1.0]hex-1-yl)acrylsäuremethyl- oder -ethylester, deren Disubstitutionsprodukte in 3-Stellung wie (3,3-Bis(ethoxycarbonyl)bicyclo[3.1.0]hex-1-yl)acrylsäuremethyl- oder -ethylester. Bevorzugte cyclische Allylsulfide sind die Additionprodukte von 2-(Hydroxymethyl)-6-methylen-1,4-dithiepan oder 7-Hydroxy-3-methylen-1,5-dithiacylooctan mit 2,2,4-Trimethylhexamethylen-1,6-diisocyanat oder dem asymmetrischen Hexamethylendiisocyanat-Trimeren (Desmodur^{®} VP LS 2294 der Bayer AG).

Außerdem können als radikalisch polymerisierbare Bindemittel auch Styrol, Styrolderivate oder Divinylbenzol, ungesättigte Polyester-, Polyurethan- und Epoxy-Harze sowie Allylverbindungen oder radikalisch polymerisierbare Polysiloxane, die aus geeigneten Methacrylsilanen, wie z.B. 3-(Methacryloyloxy)propyltrimethoxysilan, hergestellt werden können und z.B. in der DE 199 03 177 C2 beschrieben sind, eingesetzt werden.

Weiterhin lassen sich als radikalisch polymerisierbare Bindemittel auch Mischungen der voranstehend genannten Monomeren mit radikalisch polymerisierbaren, säuregruppenhaltigen Monomeren verwenden, die auch als Haftmonomere bezeichnet werden. Bevorzugte säuregruppenhaltige Monomere sind polymerisationsfähige Carbonsäuren, wie Maleinsäure, Acrylsäure, Methacrylsäure, 2-(Hydroxymethyl)acrylsäure, 4-(Meth)acryloyloxyethyltrimellitsäureanhydrid, 10-Methacryloyloxydecylmalonsäure, N-(2-Hydroxy-3-methacryloyloxypropyl)-N-phenylglycin oder 4-Vinylbenzoesäure.

Als Haftmonomere eignen sich auch radikalisch polymerisierbare Phosphonsäuremonomere, insbesondere Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentyl-phosphonsäure, 2-[4-(Dihydroxyphosphoryl)-2-oxa-butyl]-acrylsäure oder 2-[2-Dihydroxyphosphoryl)-ethoxymethyl]-acrylsäure-ethyl- oder 2,4,6-trimethylphenylester.

Zudem eignen sich als Haftmonomere acide polymerisationsfähige Phosphorsäureester, insbesondere 2-Methacryloyloxypropylmono- oder -dihydrogenphosphat, 2-Methacryloyloxyethylmono- oder - dihydrogenphosphat, 2-Methacryloyloxyethylphenyl-hydrogenphosphat, Dipentaerythritol-pentamethacryloyloxyphosphat, 10-Methacryloyloxydecyl-dihydrogenphosphat, Dipentaerythritol-pentamethacryloyloxyphosphat, Phosphorsäuremono-(1-acryloylpiperidin-4-yl)-ester, 6-(Methacrylamido)hexyldihydrogen-phosphat und 1,3-Bis-(N-acryloyl-N-propyl-amino)-propan-2-yl-dihydrogenphosphat.

Darüber hinaus eignen sich polymerisationsfähige Sulfonsäuren als Haftmonomere, insbesondere Vinylsulfonsäure, 4-Vinylphenylsulfonsäure oder 3-(Methacrylamido)propylsulfonsäure.

Als durch Polyaddition härtbare Bindemittel eignen sich besonders Thiol-En-Harze, die Mischungen von mono- oder multifunktionellen Mercaptoverbindungen und di- oder multifunktionellen ungesättigten Monomeren, vor allem Allyl- oder Norbonenverbindungen enthalten.

Beispiele für mono- oder multifunktionelle Mecaptoverbindungen sind o-, m- oder p-Dimercaptobenzol und Ester der Thioglykol- oder der 3-Mercaptopropionsäure von Ethylen-, Propylen- oder Butylenglykol, Hexandiol, Glycerin, Trimethylolpropan oder Pentaerythrit.

Beispiele für di- oder multifunktionelle Allylverbindungen sind Ester von Allylalkohol mit Di- oder Tricarbonsäuren, wie Malon- Malein- Glutar-, Bernstein-, Adipin-, Sebacin-, Phthal-, Terephthal- oder Gallussäure sowie mono- oder trifunktionelle Allylether, wie z.B. Diallylether, α,ω-Bis[allyloxy]alkane, Resorcin- oder Hydrochinondiallylether sowie Pyrogalloltriallylether, oder andere Verbindungen wie z.B. 1,3,5-Triallyl-1,3,5-triazin-2,4,6-(1H,3H,5H)-trion, Tetraallylsilan oder Tetraallylorthosilikat.

Beispiele für di- oder multifunktionelle Norbonenverbindungen sind Diels-Alder-Additionsprodukte von Cyclopentadien oder Furan mit di- oder multifunktionellen (Meth)acrylaten, sowie Ester und Urethane von 5-Norbornen-2-methanol oder 5-Norbornen-2-ol mit Di- oder Polycarbonsäuren, wie z.B. Malon-Malein- Glutar-, Bernstein-, Adipin-, Sebacin-, Phthal-, Terephthal- oder Gallussäure, mit Di- oder Polyisocyanaten, wie Hexamethylendiisocyanat oder dessen cyclischem Trimer, 2,2,4-Trimethylhexamethylendiisocyanat, Toluylendiisocyanat oder Isophorondiisocyanat.

Weiterhin lassen sich Acylgermanium-Verbindungen der Formel (I) als Co-Initiatoren bzw. Photosensibilisatoren für die kationische Polymerisation von kationisch polymerisierbaren Monomeren einsetzen. Bevorzugte kationisch polymerisierbare Verdünner- oder Vernetzermonomere sind z.B. Glycidylether oder cycloaliphatische Epoxide, cyclische Ketenacetale, Vinylether, Spiroorthocarbonate, Oxetane oder bicyclische Orthoester. Besonders bevorzugte Beispiele sind: Triethylenglycoldivinylether, Cyclohexandimethanoldivinylether, 2-Methylen-1,4,6-trioxaspiro[2.2]nonan, 3,9-Dimethylen-1,5,7,11-tetraoxaspiro[5.5]undecan, 2-Methylen-1,3-dioxepan, 2-Phenyl-4-methylen-1,3-dioxolan, Bisphenol-A-diglycidylether, 3,4-Epoxy-cyclohexylmethyl-3,4-epoxycyclohexancarboxylat, Bis(-(3,4-epoxycyclohexylmethyl)adipat, Vinylcyclohexendioxid, 3-Ethyl-3-hydroxymethyloxetan, 1,10,-Decandiylbis(oxymethylen)-bis(3-ethyloxetan) oder 3,3-(4-Xylylendioxy)-bis-(methyl-3-ethyloxetan).

Als kationisch polymerisierbare Bindemittel eignen sich auch Kieselsäurepolykondensate, die beispielsweise durch hydrolytische Kondensation von Silanen zugänglich sind, die kationisch polymerisierbare Gruppen, bevorzugt z.B. Epoxid-, Oxetan-, Spiroorthoester und/oder Vinylethergruppen tragen. Solche Kieselsäurepolykondensate sind beispielsweise in der DE 41 33 494 C2 oder US 6,096,903 beschrieben.

Neben Acylgermanium-Verbindungen der allgemeinen Formel (I) können die erfindungsgemäßen Zusammensetzungen vorteilhaft zusätzlich auch bekannte Photoinitiatoren (vgl. J.P. Fouassier, J.F. Rabek (Hrsg.), Radiation Curing in Polymer Science and Technology, Vol. II, Elsevier Applied Science, London und New York 1993) für den UV- oder sichtbaren Bereich, wie z.B: Benzoinether, Dialkylbenzilketale, Dialkoxyacetophenone, Acyl- oder Bisacylphosphinoxide, α-Diketone wie 9,10-Phenanthrenchinon, Diacetyl, Furil, Anisil, 4,4'-Dichlorbenzil und 4,4'-Dialkoxybenzil und Campherchinon sowie ggf. Coinitiatoren wie tertiäre Amine z.B Dimethylaminobenzoesäureethylester oder N-Methyldiethanolamin enthalten.

Außderdem können die erfindungsgemäßen Zusammensetzungen neben den Acylgermanium-Verbindungen der allgemeinen Formel (I) zusätzlich einen oder mehrere kationische Photoinitiatoren enthalten, vorzugsweise Diaryliodonium- oder Triarylsulfoniumsalze. Bevorzugte Diaryliodoniumsalze sind die kommerziell erhältlichen Photoinitiatoren 4-Octyloxy-phenyl-phenyliodoniumhexafluoroantimonat, Isopropylphenyl-methylphenyl-iodoniumtetrakis(pentafluorophenyl)borat und 4-Phenylsulfanylphenyl diphenylsulfonium hexafluorphosphat. Diese kationischen Photoinitiatoren können zur Beschleunigung der Aushärtung von erfindungsgemäßen Zusammensetzungen auf der Basis von Acylgermanium-Verbindungen der allgemeinen Formel (I) eingesetzt werden. Umgekehrt läßt sich die Aushärtung von Zusammensetzungen mit kationischen Initiatoren durch die Zugabe von Acylgermanium-Verbindungen der Formel (I) beschleunigen.

Weiterhin können die erfindungsgemäßen Zusammensetzungen neben den Acylgermanium-Verbindungen der allgemeinen Formel (I) zur dualen Härtung auch Azoverbindungen, wie 2,2'-Azobis(isobutyronitril) (AIBN) oder Azobis-(4-cyanovalerian-säure), oder Peroxide, wie Dibenzoylperoxid, Dilauroylperoxid, *tert*-Butylperoctoat, *tert*-Butylperbenzoat oder Di-(*tert-*butyl)-peroxid enthalten. Zur Beschleunigung der Initiierung mittels Peroxiden lassen sich Kombinationen mit aromatischen Aminen einsetzen. Bevorzugte Redoxsysteme sind Kombinationen aus Benzoylperoxid mit Aminen, wie N,N-Dimethyl-p-toluidin, N,N-Dihydroxyethyl-p-toluidin, p-Dimethylaminobenzoesäureethylester oder strukturverwandte Systeme. Darüber hinaus sind auch Redoxsysteme bestehend aus Peroxiden und Reduktionsmitteln, wie z.B. Ascorbinsäure, Barbiturate oder Sulfinsäuren, für die duale Aushärtung geeignet. Die Menge an zusätzlichen Initiatoren liegt vorzugsweise im Bereich von 0 bis 3 Gew.-%.

Erfindungsgemäß sind Zusammensetzungen bevorzugt, die einen oder mehrere Füllstoffe, vorzugsweise organische oder anorganische partikuläre Füllstoffe enthalten. Bevorzugte anorganische partikulären Füllstoffe sind amorphe kugelförmige nanopartikuläre Füllstoffe auf der Basis von Oxiden, wie pyrogener Kieselsäure oder Fällungskieselsäure, ZrO₂ und TiO₂ oder Mischoxiden von SiO₂, ZrO₂ und/oder TiO₂ mit einem mittleren Partikeldurchmesser von 10 bis 200 nm, Minifüllstoffe, wie Quarz-, Glaskeramik- oder Glaspulver mit einer durchschnittlichen Teilchengröße von 0,2 bis 5 µm sowie röntgenopake Füllstoffe, wie Ytterbiumtrifluorid, nanopartikuläres Tantal(V)-oxid oder Bariumsulfat. Darüber hinaus können auch faserförmige Füllstoffe wie Nanofasern, Glasfasern, Polyamid- oder Kohlenstoff-Fasern eingesetzt werden.

Als weite Komponente können die erfindungsgemäßen Zusammensetzungen Farbmittel wie Farbstoffe und/oder Pigmente enthalten.

Außerdem können die erfindungsgemäßen Zusammensetzungen im Bedarfsfalle weitere Additive sowie Lösungsmittel, wie z.B. Wasser, Ethanol, Aceton und/oder Ethylacetat, enthalten.

Die Additive sind vorzugsweise aus Stabilisatoren, UV-Absorbern, Gleitmitteln, Netzmitteln, Dispergiermitteln, Haftvermittlern, Mattierungs- und Glanzmitteln, Verlaufs- und Filmbildehilfsmitteln, Hautverhinderungsmitteln, Lichtschutzmitteln, Korrosionsschutzmitteln, flammhemmenden Mitteln, Antioxidantien, optischen Aufhellern, Fließverbesserern, Verdikkern und Antischaummitteln ausgewählt.

Die Initiatoren gemäß den Formeln (I) und (II) zeichnen sich durch eine hohe Reaktivität aus und können daher in geringen Konzentrationen eingesetzt werden (vgl. Beispiel 7). Darüber hinaus gestatten sie die Aushärtung auch von dünnen Schichten oder Filmen ohne die Ausbildung einer Inhibierungsschicht. Die erfindungsgemäßen Initiatoren können daher zur Verhinderung der Ausbildung einer Inhibierungsschicht eingesetzt werden.

Die erfindungsgemäßen Zusammensetzungen enthalten, bezogen auf die Gesamtmasse der Zusammensetzung, vorzugsweise 0,001 bis 5 Gew.-%, besonders bevorzugt 0,01 bis 4 Gew.-% und insbesondere 0,1 bis 3 Gew.-% Acylgermanium-Verbindung der Formel (I).

Die erfindungsgemäßen Materialien enthalten somit vorzugsweise:
(a) 0,001 bis 5 Gew.-% Acylgermanium-Verbindung der allgemeinen Formel (I), vorzugsweise 0,01 bis 4 Gew.-%, besonders bevorzugt 0,1 bis 3 Gew.-%,
(b) 5 bis 99,9 Gew.-% polymerisierbares Bindemittel, vorzugsweise 10 bis 95 Gew.-%, besonders bevorzugt 15 bis 90 Gew.-%, und
(c) 0 bis 90 Gew.-% Füllstoff, vorzugsweise 5 bis 87 Gew.-%, besonders bevorzugt 10 bis 85 Gew.-%.

Die Zusammensetzungen können vorteilhaft außerdem enthalten:
(d) 0 bis 50 Gew.-% Additiv, vorzugsweise 0,01 bis 4 Gew.-%, besonders bevorzugt 0,1 bis 3 Gew.-%, wobei sich diese Mengenangaben auf die Gesamtmasse aller Additive beziehen, und
(e) 0 bis 10 Gew.-%, vorzugsweise 0.01 bis 5 Gew.-% Pigmente und/oder Farbstoffe.

Alle Prozentangaben beziehen sich, wenn nicht anders angegeben, auf die Gesamtmasse der Zusammensetzung.

Die erfindungsgemäßen Zusammensetzungen eignen sich besonders als Adhäsive, Beschichtungen, Lacke, Tinten, Zemente, Komposite, zur Herstellung von Formteilen bzw. Formkörpern, wie Stäben, Platten, Scheiben, optischen Linsen, Kontaktlinsen, und insbesondere als Dentalwerkstoffe, ganz besonders als Füllungskomposite.

Zusammensetzungen zur Anwendung als dentale Zemente enthalten bevorzugt:
(a) 0,001 bis 3 Gew.-% Acylgermanium-Verbindung der allgemeinen Formel (I),
(b) 20 bis 70 Gew.-% polymerisierbares Bindemittel,
(c) 30 bis 75 Gew.-% Füllstoff und
(d) 0,01 bis 5 Gew.-% Additiv.

Zusammensetzungen zur Anwendung als dentale Komposite enthalten bevorzugt:
(a) 0,001 bis 2 Gew.-% Acylgermanium-Verbindung der allgemeinen Formel (I),
(b) 10 bis 60 Gew.-% polymerisierbares Bindemittel,
(c) 40 bis 85 Gew.-% Füllstoff und
(d) 0,01 bis 5 Gew.-% Additiv.

Zusammensetzungen zur Anwendung als dentale Beschichtungsmaterialien enthalten bevorzugt:
(a) 0,001 bis 5 Gew.-% Acylgermanium-Verbindung der allgemeinen Formel (I),
(b) 20 bis 99,9 Gew.-% polymerisierbares Bindemittel,
(c) 0 bis 20 Gew.-% nanopartikuläre Füllstoffe und
(d) 0,01 bis 2 Gew.-% Additiv,
(e) 0 bis 50 Gew.-% Lösungsmittel.

Zusammensetzungen zur Anwendung als Druckertinte enthalten bevorzugt:
(a) 0,001 bis 5 Gew.-% Acylgermanium-Verbindung der allgemeinen Formel (I),
(b) 30 bis 60 Gew.-% polymerisierbares Bindemittel,
(c) 1 bis 45 Gew.-% Farbmittel und
(d) 0,01 bis 30 Gew.-% Additiv.

Zusammensetzungen zur Anwendung als Lack, beispielsweise als Weißlack oder als Lack für optische Fasern, enthalten bevorzugt:
(a) 0,001 bis 5 Gew.-% Acylgermanium-Verbindung der allgemeinen Formel (I),
(b) 55 bis 99,5 Gew.-% polymerisierbares Bindemittel,
(c) 0,1 bis 50 Gew.-% Pigment und ggf.
(d) 0,01 bis 30 Gew.-% Additiv.

Ein bevorzugtes Pigment zur Herstellung von Lacken ist TiO₂.

Dentalwerkstoffe, die sich durch Thiol-en-Reaktion härten lassen, enthalten vorzugsweise eine Mischung aus einer oder mehreren Polythiolverbindungen und einer oder mehreren Polyvinylverbindungen, wobei eine oder mehrere dieser Verbindungen in oligomerer Form vorliegen können. Vorzugsweise sind 45 bis 55 % der funktionellen Gruppen dieser Mischungen Thiolgruppen, die übrigen Gruppen können Vinylgruppen sein. Die Mischungen können weiterhin einen oder mehrere Füllstoffe enthalten, wobei der Menge an polymerisierbaren Harzen vorzugsweise im Bereich von 10 bis 40 Gew.-% und die Füllstoffmenge vorzugsweise im Bereich von 60 bis 90 Gew.-% liegt. Geeignete Mischungen aus Polythiol- und Polyvinylverbindungen sowie geeignete füllstoffhaltige Mischungen werden in der WO 2005/086911 beschrieben. Die Menge an Initiator gemäß Formel (I) beträgt vorzugsweise 0,05 bis 0,5 Gew.-%.

Gegenstand der Erfindung ist auch die Verwendung von Acylgermanen der Formel (I) zur Herstellung von Adhäsiven, Beschichtungen, Lacken, Tinten, Zementen, Kompositen, Formteilen oder Dentalwerkstoffen sowie deren Verwendung als Initiator für die radikalische Polymerisation.

Außerdem betrifft die Erfindung ein Verfahren zur Herstellung von Formkörpern, insbesondere dentalen Kronen, Brücken, Inlays und künstlichen Zähnen, bei dem man eine erfindungsgemäße Zusammensetzung auf an sich bekannte Weise zu dem Formkörper formt und dann zumindest teilweise, vorzugsweise vollständig, härtet. Die Härtung erfolgt vorzugsweise durch radikalische Polymerisation.

Die erfindungsgemäßen Photoinitiatoren zeichnen sich besonders durch eine hohe Reaktivität und eine hohe Aktivität schon bei geringer Einsatzkonzentration aus. Dadurch kann eine äußerst rasche Aushärtung des Photopolymers im Vergleich zu bekannten Photoinitiatoren, die im sichtbaren Bereich absorbieren erreicht werden. Beispielsweise ergaben Messungen von Bisacyldiethylgermanium in einer Harzmischung aus Decandioldimethacrylat (D₃MA):UDMA:Bis-GMA = 1:1:1 eine beinahe doppelt so hohe Polymerisationsrate (Rₚ) wie Campherchinon in Kombination mit einem Aminbeschleuniger in der gleichen Formulierung. Die Aushärtungszeit konnte im Vergleich zu Campherchinon/Amin ebenfalls halbiert werden. Selbst bei 15-facher Verdünnung an Bisacyldiethylgermanium kann noch immer eine mit Campherchinon/Amin als Photoinitiator vergleichbare Reaktivität erreicht werden (siehe Ausführungsbeispiele, Tabellen 7, 8, 9, Summe aus Initiator und Beschleuniger).

Außerdem weisen die naturgemäß gelblich gefärbten Photoinitiatoren gemäß Formel (I) einen ausgezeichneten Photobleachingeffekt auf, d.h. die Verbindungen der Formel (I) werden bei der Härtung entfärbt und dadurch Verfärbungen des Materials nach dem Aushärten vermieden.

Die Erfindung wird im Folgenden anhand von Beispielen näher erläutert.

### Ausführungsbeispiele

### Beispiel 1: Synthese von Benzoyltrimethylgerman (Vergleichsbeispiel)

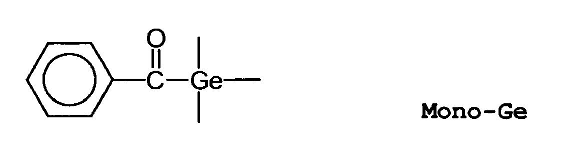

In einem trockenen 50 ml Dreihalskolben mit Rückflusskühler, Magnetrührer und Septum wurden unter Argon 1.64 g (4.49 mmol) Allylpalladium(II)chlorid-Dimer, 1.49 g (8.97 mmol) Triethylphosphit und 23.24 g (98.7 mmol) Hexamethyldigerman vorgelegt, und 5 min bei Raumtemperatur gerührt. Anschließend wurde 12.62 g (89.7 mmol) frisch destilliertes Benzoylchlorid zugetropft. Nach 4h Rühren bei 110°C, wurde der Pd-Katalysator vom Reaktionsgemisch abgetrennt und flüchtige Reaktionsprodukte sowie der Überschuss an Hexamethyldigerman am Rotationsverdampfer abgezogen. Das Reaktionsgemisch wurde säulenchromatographisch (PE:EE = 40:1) aufgetrennt. Es ergaben sich 7.8 g (78 % d. Th.) an Benzoyltrimethylgerman **(Mono-Ge)** als gelbe Flüssigkeit. DC (PE:EE = 20:1): R_{f}=0.58.
UV-VIS: λₘₐₓ: 411.5 nm, ε = 1374 dm²/mol

¹H-NMR (400 MHz; CDCl₃): δ (ppm): 7.78-7.82 (m, 2H, Ar-H^{2,6}), 7.48-7.58 (m, 3H, Ar-H^{3,4,5}), 0.51 (s, 9H, -CH₃).

¹³C-NMR (100 MHz; CDCl₃): δ (ppm): 234.39 (-C=O), 140.61 (Ar-C¹), 132. 90 (Ar-C⁴), 128.75 (Ar-C^{2,6}), 127.71 (Ar-C^{3,5}), -1.14 (-CH₃).

### Beispiel 2: Synthese von 1,6-Bis[4-(trimethylgermiocarbonyl)phenoxy]hexan

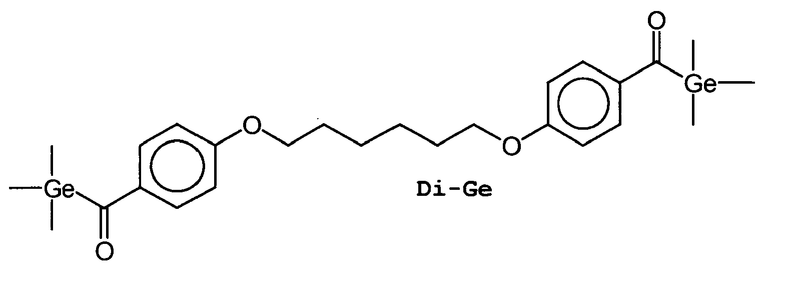

### 1. Stufe: 1,6-Bis(4-formylphenoxy)hexan:

In einem 500 ml Dreihalskolben mit Rückflusskühler, Innenthermometer und Magnetrührer wurde eine Mischung aus 73,3 g (0,6 mol) p-Hydroxybenzaldehyd, 73,2 g (0,3 mol) 1,6-Dibromhexan, 39,6 g (0,6 mol) Kaliumhydroxid und 240 ml Dimethylformamid unter Rühren 1 h unter Rückfluss erhitzt. Nach Abkühlen der Reaktionsmischung auf Raumtemperatur gab man den erhaltenen Kristallbrei in ein 2 1 Becherglas, das 1 1 deionisiertes Wasser enthielt. Die gebildete Suspension wurde mittels eines mechanischen Rührers intensiv gerührt bis die anfänglich gestiegene Temperatur wieder auf Raumtemperatur gefallen war. Danach filtrierte man die vorliegenden Kristalle ab, wusch diese gründlich mit Wasser und kristallisierte das Produkt aus einer Mischung von 1200 ml Ethanol und 300 ml Wasser um. Nach dem Trocknen im Vakuumtrockenschrank bei 70-80 °C bis zur Gewichtskonstanz erhielt man 63,5 g (65% d. Th.) 1,6-Bis(4-formylphenoxy)hexan (Schmp.: 109,8-111,2 °C) in Form weisser Kristalle.

¹H-NMR (400 MHz; CDCl₃) : δ (ppm): 9.88 (s, 2H, CHO), 7.84-7.81 (m, 4H, Ar-H^{2,6}), 7.01-6.98 (m, 4H, Ar-H^{3,5}), 4.06 (t, 4H, O-CH₂), 1.88-1.83 (m, 4H, O-CH₂-CH₂) und 1.59-1.55 (m, 4H, CH₂).

### 2. Stufe: 1,6-Bis[4-(1,3-dithian-2-yl)phenoxy]hexan:

In einem 500 ml Dreihalskolben mit Gaseinleitungsrohr, Rückflusskühler, Innenthermometer und Magnetrührer wurde eine Suspension aus 52,55 g (0,16 mol) 1,6-Bis(4-formylphenoxy)hexan, 200 ml Chloroform und 34,85 g (0,32 mol) 1,3-Propandithiol gerührt und auf -10 °C abgekühlt. Man leitete ca. 15 min trocknes HCl-Gas ein, wodurch sich die Suspension stark verdickte. Nach der Beendigung der HCl-Einleitung rührte man noch 30 min unter Eiskühlung, entfernte das Eisbad und ließ die rotbraune Suspension noch 2 h bei Raumtemperatur rühren. Danach wurde das Lösungsmittel mit einer Membranpumpe in eine Kühlfalle abdestilliert (400 bis 10 mbar), der Rückstand mit 300 ml Ethylacetat und 100 ml Methanol versetzt und gerührt. Die Suspension wurde filtriert, der feste Rückstand mit 100 ml Methanol und 100 ml Aceton gewaschen und im Vakuumtrockenschrank bis zur Gewichtskonstanz getrocknet. Man erhielt 78,2 g (96% d. Th.) 1,6-Bis[4-(1,3-dithian-2-yl)phenoxy]hexan als rötlichen, schwer löslichen Feststoff (Schmp.: 189-192 °C).

¹H-NMR (400 MHz; CDCl₃): δ (ppm) : 7.39-7.26 (m, 4H, Ar-H^{2,6}), 6.86-6.83 (m, 4H, Ar-H^{3,5}), 5.13 (s, 2H, CH), 3.96 (t, 4H, O-CH₂), 3.09-2.87 (m, 8H, SCH₂) und 2.19-1.52 (m, 12H, 2x O-CH₂-CH₂, 4x CH₂).

### 3. Stufe: 1,6-Bis{4-[2-(trimethylgermio)-1,3-dithian-2-yl]phenoxy}hexan:

In einem trockenen 250 ml Dreihalskolben mit 50 ml Tropftrichter, Innenthermometer, Magnetrührer und Septum wurde unter Argon eine Suspension von 8,11 g (16.0 mmol) 1,6-Bis[4-(1,3-dithian-2-yl)phenoxy]hexan in 50 ml wasserfreiem Tetrahydrofuran (THF) hergestellt. Man kühlte die Suspension auf -10 °C ab und tropfte über einen Zeitraum von 10 min 14,1 ml einer 2,5 molaren Lösung von n-Butyllithium (35,2 mmol) in Hexan so zu, dass die Innentemperatur 0 °C nicht überstieg. Anschliessend wurde die braune Lösung 3 h bei -10 °C gerührt und dazu eine Lösung von 4,90 g (32,0 mmol) Trimethylgermaniumchlorid in 20 ml wasserfreiem THF innerhalb von 5 min bei -5 °C zugetropft. Das Reaktionsgemisch wurde 24 h bei Raumtemperatur weitergerührt. Dann gab man 50 ml Wasser und 150 ml Ethylacetat zu und trennte die gebildeten Phasen. Die organische Phase wurde 2 mal mit je 50 ml Wasser gewaschen und die vereinigten wässrigen Phasen wurden mit 50 ml Ethylacetat reextrahiert. Schliesslich wurden die vereinigten organischen Phasen mit 80 ml gesättigter wässriger NaCl-Lösung gewaschen und über wasserfreiem Natriumsulfat getrocknet. Nach Abtrennen des Trockenmittels wurde am Rotationsverdampfer eingeengt und das Produkt im Feinvakuum bis zur Gewichtskonstanz getrocknet. Man erhielt 10,9 g (92 % d. Th.) 1,6-Bis{4-[2-(trimethylgermio)-1,3-dithian-2-yl]phenoxy}hexan als leicht gelblichen Feststoff (Schmp.: 147-153 °C).

¹H-NMR (400 MHz; CDCl₃) : δ (ppm): 7.78-7.76 (m, 4H, Ar-H^{2,6}), 6.91-6.88 (m, 4H, Ar-H^{3,5}), 3.99 (t, 4H, O-CH₂), 2.81-2.36 (m, 8H, SCH₂), 2.04-1.58 (m, 12H, 2x O-CH₂-CH₂, 4x CH₂) und 0.18 (s, 18H, CH₃).

### 4. Stufe: 1,6-Bis[4-[2-(trimethylgermiocarbonyl)phenoxy]hexan:

In einem 250 ml Zweihalskolben mit Innenthermometer und Magnetrührer wurden 3,70 g (5,0 mmol) 1,6-Bis{4-[2-(trimethylgermio)-1,3-dithian-2-yl]phenoxy}hexan in 60 ml THF gelöst. Die gesamte Synthese einschliesslich der Reinigungsoperationen wurde in einer Gelblichtkapelle durchgeführt. Man gab 15 ml deionisiertes Wasser zu und dann in etwa 8 gleich grossen Portionen jeweils insgesamt 12,01 g (0,12 mol) Calciumcarbonat und 30,46 g (0,12 mol) Iod so zu, dass die Zeitspanne zwischen den Zugaben ca. 30 min. betrug. Man ließ das rot-braune Reaktionsgemisch noch 4 h bei Raumtemperatur rühren, filtrierte über eine mit 25 g Kieselgel 60 (0.035-0.070 mm) gefüllte Fritte mit einem Durchmesser von 5 cm und wusch mit 100 ml THF nach. Das Filtrat wurde bis zum Farbumschlag nach gelb mit ca. 200 ml gesättigter wässriger Natriumdithionit-Lösung versetzt. Anschliessend wurde die Suspension filtriert und der Filtrationsrückstand 4 mal mit je 50 ml Ethylacetat gewaschen. Das so erhaltene trübe, gelbe 2-PhasenGemisch wurde mit 800 ml Ethylacetat versetzt und die Phasen wurden getrennt. Die organische Phase wurde 2 mal mit je 50 ml Wasser gewaschen und die vereinigten Wasserphasen mit 50 ml Ethylacetat reextrahiert. Die vereinigten organischen Phasen wurden mit 80 ml gesättigter wässriger NaCl-Lösung gewaschen und über wasserfreiem Natriumsulfat getrocknet. Nach Abtrennen des Trockenmittels wurde am Rotationsverdampfer eingeengt und das Produkt im Feinvakuum getrocknet. Der verbliebene ölige, rot-braune Feststoff wurde mit 20 ml Dichlormethan versetzt und über eine mit 40 g Kieselgel 60 (0,035-0,070 mm) gefüllte Fritte mit einem Durchmesser von 5 cm filtriert und mit 800 ml Dichlormethan gewaschen. Der Eluent wurde am Rotationsverdampfer eingeengt und im Feinvakuum (0,06 mbar) bei 50 °C bis zur Gewichtskonstanz getrocknet. Der nunmehr erhaltene rot-braune Feststoff von ca. 2 g wurde in 10 ml Dichlormethan gelöst und mittels Flash-Säulenchromatographie gereinigt: Säule: 75 g Kieselgel 60 (0,035-0,070 mm), 2,5 cm Durchmesser, Elutionsmittel Dichlormethan. Nach ca. 100 ml Vorlauf und 400 ml Zwischenlauf werden 1100 ml Hauptfraktion gesammelt, diese am Rotationsverdampfer eingeengt und im Feinvakuum (0,06 mbar) bei 50 °C bis zur Gewichtskonstanz getrocknet. Man erhielt 1,20 g (43% d. Th.) 1,6-Bis[4-[2-(trimethylgermiocarbonyl)-phenoxy]hexan **(Di-Ge)** als blassgelben Feststoff (Schmp.: 85,0-86,4 °C).

¹H-NMR (400 MHz; Cd13): δ (ppm): 7.80-7.78 (m, 4H, Ar-H^{2,6}), 6.97-6.94 (m, 4H, Ar-H^{3,5}), 4.04 (t, 4H, O-CH₂), 1.68-1.64 (m, 4H, O-CH₂-CH₂), 1.57-1.55 (m, 4H, CH₂) und 0.49 (s, 18H, CH₃). ¹³C-NMR (100 MHz; CDCl₃) : δ (ppm): 231.13 (-C=O), 162.82 (Ar-C¹), 134.35 (Ar-C⁴), 129. 97 (Ar-C^{2,6}), 114.28 (Ar-C^{3,5}), 67.95 (O-CH₂), 28.93 (O-CH₂-CH₂), 25.70 (CH₂) und -1.12 (-CH₃).

Der erfindungsgemässe Photoinitiator **Di-Ge** mit einem langwelligen Absorptionsmaximum von 400.5 nm zeigt einen Extinktionskoeffizienten ε = 3260 dm²/mol, der damit im Vergleich zum Benzoyltrimethylgerman **Mono-Ge** (ε = 1461 dm²/mol) mehr als doppelt so hoch ist, was zu einer Verbesserung der Photopolymerisationsaktivität führt. Im Vergleich zum im Dentalbereich weit verbreitet verwendeten Norrish-Typ-II-Photoinitiator Campherchinon, dessen Extinktionskoeffizient ε bei λₘₐₓ von 468 nm nur 380 dm/mol beträgt und der ausserdem zur wirksamen Radikalbildung ein zusätzliches Reduktionsmittel, meist Amin, benötigt, liegt das Absorptionsmaximum von **Di-Ge** deutlich kurzwelliger und zeigt damit ein sehr gutes Ausbleichen beim Bestrahlen.

### Beispiel 3: Herstellung eines Kompositzementes unter Verwendung des Di-Ge aus Beispiel 2

Entsprechend der nachfolgend aufgeführten Tabelle 1 wurden Kompositbefestigungszemente auf der Basis einer Methacrylatmischung und unter Einbeziehung entweder unterschiedlicher Konzentrationen des Photoiniatotors **Di-Ge** (Zemente A und B), des Benzoyltrimethylgermans **Mono-Ge** aus Beispiel 1 (Zement C, Vergleich) oder einer Mischung aus Campherchinon und p-N,N-Dimethylaminobenzoesäureethylester (Zement D, Vergleich) mittels eines Walzenstuhles "Exakt" (Exakt Apparatebau, Norderstedt) hergestellt. Dabei enthalten die Zemente B, C bzw. D die gleiche molare Konzentration an Photoinitiator, d.h. an Benzoyltrimethylgerman (Zement C) bzw. Campherchinon (Zement D) Von den Materialien wurden entsprechende Prüfkörper präpariert, die 2 mal 3 Minuten mit einer dentalen Lichtquelle (Spectramat^{®}, Ivoclar Vivadent AG) bestrahlt und damit ausgehärtet wurden. Nach der ISO-Norm ISO-4049 (Dentistry - Polymer-based filling, restorative and luting materials) erfolgte die Bestimmung der Biegefestigkeit, des Biege-E-Moduls und der Exothermzeit.

Aus Tabelle 2 ist ersichtlich, dass die auf **Di-Ge** basierenden Zemente mit zunehmender Photoinitiatorkonzentration eine bessere Aushärtung ergeben. Es wird sichtbar, dass der Zement B mit **Di-Ge** als Photoinitiator im Vergleich zum Zement C, basierend auf **Mono-Ge** bzw. Zement D, basierend auf einer konventionellen Photoinitiatormischung aus Campherchinon und p-N,N-Dimethylaminobenzoesäureethylester, zu Kompositen mit besseren mechanischen Eigenschaften führt.

**Tabelle 1: Zusammensetzung der Kompositzemente (Angaben in Gew.-%)**

| **Komponente** | **Zement A** | **Zement B** | **Zement C¹⁾** | **Zement D¹⁾** |
|---|---|---|---|---|
| Di-Ge | 0.34 | 1.01 | - | - |
| Mono-Ge aus Beispiel 1 | | | 0.32 | |
| Campherchinon | - | - | - | 0.24 |
| p-N,N-Dimethylaminobenzoesäureethylester | - | - | - | 0.23 |
| UDMA²⁾ | 31.87 | 31.20 | 31.89 | 31.80 |
| Triethylenglycoldimethacrylat | 7.81 | 7.81 | 7.81 | 7.81 |
| Aerosil OX-50 (Degussa) | 41.27 | 41.27 | 41.27 | 41.23 |
| Ytterbiumtrifluorid (Rhone-Poulenc) | 18.71 | 41.27 | 18.71 | 18.69 |

| | | | | |
|---|---|---|---|---|
| ¹⁾ Vergleichsbeispiel ²⁾ Additionsprodukt aus 2 mol 2-Hydroxyethylmethacrylat und 1 mol 2,2,4-Trimethylhexamethylendiisocyanat | | | | |

**Tabelle 2: Kompositzementeigenschaften**

| **Komponente** | **Zement A** | **Zement B** | **Zement C¹⁾** | **Zement D¹⁾** |
|---|---|---|---|---|
| Exothermzeit (s) | 11 | 10 | 12 | 8 |
| Biegefestigkeit (MPa) nach 24 h WL²⁾ | 115 | 124 | 116 | 118 |
| E-Modul (MPa) nach 24 h WL¹⁾ | 5930 | 6570 | 5240 | 5580 |

| | | | | |
|---|---|---|---|---|
| ¹⁾ Vergleichsbeispiel ²⁾ WL = Wasserlagerung der Prüfkörper bei 37 °C | | | | |

### Beispiel 4: Herstellung eines Füllungskomposites unter Verwendung des Di-Ge aus Beispiel 2

Entsprechend der nachfolgend aufgeführten Tabelle 3 wurden Füllungskomposite auf der Basis einer Methacrylatmischung und unter Einbeziehung gleicher molarer Konzentrationen des erfindungsgemässen Photoinitiators **Di-Ge** (Komposit F), des Benzoyltrimethylgermans **Mo-Ge** (Vergleich) aus Beispiel 1 (Komposit G) oder einer Mischung aus Campherchinon und p-N,N-Dimethylaminobenzoesäureethylester (Komposit G) mittels eines Kneters LPM 0.1 SP (Linden, Marienheide) hergestellt. Von den Materialien wurden analog Beispiel 2 Prüfkörper hergestellt und ausgehärtet. Nach der ISO-Norm ISO-4049 erfolgte die Bestimmung der Biegefestigkeit, des Biege-E-Moduls. Die in Tabelle 4 dargestellten Ergebnisse belegen eine signifikant bessere Aushärtung der Komposite mit dem erfindungsgemässen Photoinitiators **Di-Ge** im Vergleich zu herkömmlichen Campherchinon basierenden Initiatorsystemen oder Acylgermaniumverbindungen mit einem Ge-Atom im Molekül.

**Tabelle 3: Zusammensetzung Füllungskomposit (Angaben in Gew.-%)**

| **Komponente** | **Komposit E** | **Komposit F¹⁾** | **Komposit G¹⁾** |
|---|---|---|---|
| Monomerharz²⁾ | 18.14 | 18.06 | 17.99 |
| **Di-Ge** | 0.19 | - | - |
| **Mono-Ge** | | 0,08 | - |
| Campherchinon | - | - | 0.05 |
| p-N,N-Dimethylaminobenzoesäureethylester | - | - | 0.09 |
| Glasfüller GM27884 (Schott)³⁾ | 51.61 | 51.61 | 51.61 |
| Sphärosil (Tokoyama Soda)⁴⁾ | 14.36 | 14.37 | 14,36 |
| Ytterbiumtrifluorid (Rhone-Poulene) | 14.89 | 14.89 | 14,89 |
| OX-50⁴⁾ | 0.81 | 0.99 | 1.01 |

| | | | |
|---|---|---|---|
| ¹⁾ Vergleichsbeispiel ²⁾ Mischung aus 42.4 Gew.-% Bis-GMA, 37.4 Gew.-% UDMA und 20.2 Gew.-% Triethylenglycoldimethacrylat ³⁾ Silanisierter Ba-Al-Borosilikatglasfüller mit einer mittleren Partikelgrösse von 1.5 µm, ⁴⁾ SiO₂-ZrO₂-Mischoxid (mittlere Primärpartikelgrösse: 250 nm)⁴⁾ Silanisiertes pyrogenes SiO₂ OX-50 (Degussa) | | | |

**Tabelle 4: Füllungskompositeigenschaften**

| **Materialeigenschaft** | **Komposit E** | **Komposit F¹⁾** | **Komposit G¹⁾** |
|---|---|---|---|
| Exothermzeit (s) | 3.6 | 10 | 9 |
| Biegefestigkeit (MPa) nach 24 h WL²⁾ | 173 | 150 | 168 |
| Biege-E-Modul (GPa) nach 24 h WL²⁾ | 14370 | 10540 | 12190 |

| | | | |
|---|---|---|---|
| ¹⁾ Vergleichsbeispiel ²⁾ WL = Wasserlagerung der Prüfkörper bei 37 °C | | | |

### Beispiel 5: Synthese von 1,6-Bis[4-(benzoyldiethylgermio)-carbonyl)-phenoxy]hexan

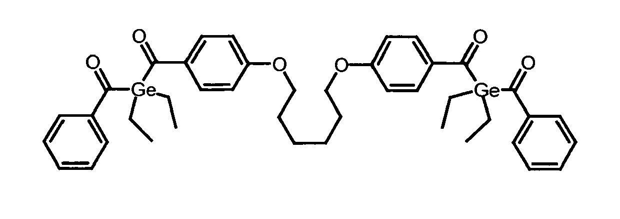

### 1. Stufe: Chlordiethyl(2-phenyl-1,3-dithian-2-yl)germanium:

In einem trockenen 500 ml Dreihalskolben mit 50 ml Tropftrichter, Innenthermometer, Magnetrührer und Septum wurden unter Argon 19,63 g (0,10 mol) 2-Phenyl-1,3-dithian in 200 ml wasserfreiem Tetrahydrofuran (THF) gelöst. Die Lösung wurde auf -10 °C abgekühlt. Über einen Zeitraum von 50 min wurden 44 ml einer 2,5 molaren Lösung von *n*-Butyllithium (0,11 mol) in *n-*Hexan bei -5 °C zugetropft. Nach beendeter Zugabe wurde die grüne Lösung 2 h bei 0 °C gerührt. Anschließend wurde sie innerhalb von 45 min zu einer auf -72 °C abgekühlten Lösung von 20,16 g (0,10 mol) Diethylgermaniumdichlorid in 500 ml wasserfreiem THF zugetropft, wobei die Temperatur -65 °C nicht überstieg. Das Reaktionsgemisch wurde 16 h im auftauenden Kältebad weitergerührt, dann wurde es am Rotationsverdampfer eingeengt. Der Rückstand wurde mit 100 ml trockenem *n*-Hexan versetzt und 1 h bei Raumtemperatur gerührt. Die Suspension wurde filtriert und das Filtrat wurde am Rotationsverdampfer eingeengt und am Feinvakuum getrocknet. Das ölige, gelbe Rohprodukt wurde mittels Hochvakuumdestillation gereinigt (Kp.: 135-150 °C/4x10⁻⁵ mbar). Man erhielt 27,23 g (75 % d. Th.) Chlordiethyl(2-phenyl-1,3-dithian-2-yl)germanium als gelbes Öl.

¹H-NMR (400 MHz; CDCl₃) : δ (ppm): 7.99-8.01 (m, 2H, Ar-H^{3,5}), 7.38-7.42, (m, 2H, Ar-H^{2,6}), 7.20-7.25 (m, 1H, Ar-H⁴), 2.79-2.86 und 2.44-2.49 (m, 2 x 2H, CH₂S), 2.04-2.15 und 1.89-1.93 (m, 2 x 1H, CH₂CH₂CH₂), 1.17-1.29 (m, 4H, CH₂CH₃), 1.03-1.10 (m, 6H, CH₃).

### 2. Stufe: 1,6-Bis-{[4-(2-diethyl-2'-phenyl-1',3'-dithian-2'-yl-germyl)-1,3-dithian-2-yl]phenoxy}hexan:

In einem trockenen 250 ml Dreihalskolben mit 50 ml Tropftrichter, Innenthermometer, Magnetrührer und Septum wurden unter Argon 8,11 g (16,0 mmol) 1,6-Bis[4-(1,3-dithian-2-yl)phenoxy]hexan (Beispiel 2, 2. Stufe) mit 100 ml wasserfreiem THF versetzt. Die Suspension wurde auf -10 °C abgekühlt. Innerhalb von 15 min wurden 14,1 ml einer 2,5 molaren Lösung von *n*-Butyllithium (35,2 mmol) in *n*-Hexan so zugetropft, dass die Temperatur des Reaktionsgemischs 0 °C nicht überstieg. Nach beendeter Zugabe wurde die braune Lösung 3 h bei -10 °C gerührt. Anschließend wurde eine Lösung von 11,57 g (32,0 mmol) Chlordiethyl(2-phenyl-1,3-dithian-2-yl)germanium in 40 ml trockenem THF innerhalb von 15 min bei -5 °C zugetropft. Das Reaktionsgemisch wurde 16 h bei Raumtemperatur weitergerührt. Dann wurden 50 ml Wasser und 150 ml Ethylacetat zugegeben und die Phasen wurden getrennt. Die organische Phase wurde zwei mal mit je 50 ml Wasser gewaschen und die vereinigten wässrigen Phasen wurden mit 80 ml Ethylacetat reextrahiert. Die vereinigten organischen Phasen wurden mit 100 ml gesättigter wässriger NaCl-Lösung gewaschen und über wasserfreiem Natriumsulfat getrocknet. Nach Abtrennung des Trockenmittels wurde am Rotationsverdampfer eingeengt und der Rückstand im Feinvakuum bis zur Gewichtskonstanz getrocknet. Das Rohprodukt wurde mittels Säulenchromatographie gereinigt (SiO₂, Dichlormethan). Man erhielt 12,90 g (70 % d. Th.) 1,6-Bis-{[4-(2-diethyl-2'-phenyl-1',3'-dithian-2'-yl-germyl)-1,3-dithian-2-yl]phenoxy}hexan als leicht gelblichen Schaum.

¹H-NMR (400 MHz; CDCl₃): δ (ppm): 7.95 und 7.78 (d, 2 x 4H, Ar-H), 7.25-7.31 und 7.12-7.15 (m, 4H + 2H, Ar-H), 6.82 (d, 4H, Ar-H), 4.00 (t, 4 H, CH₂O), 2.69-2.79 und 2.22-2.30 (m, 2 x 8H, CH₂S), 1.72-2.05 (m, 12H, OCH₂CH₂ + SCH₂CH₂), 1.56-1.61 (m, 4H, OCH₂CH₂CH₂), 1.25-1.32 (m, 8H, CH₂CH₃), 1.10-1.16 (m, 12H, CH₃).

### 3. Stufe: 1,6-Bis-{4-[(benzoyl-diethylgermio)carbonyl]-phenoxy}hexan:

In einem 250 ml Zweihalskolben aus Braunglas mit Innenthermometer und Magnetrührer wurden 7,90 g (6,8 mmol) 1,6-Bis-{[4-(2-diethyl-2'-phenyl-1',3'-dithian-2'-yl-germyl)-1,3-dithian-2-yl]phenoxy}hexan in 120 ml THF gelöst (die gesamte Synthese einschließlich der Reinigungsoperationen wurde unter Gelblicht durchgeführt). Die Lösung wurde mit 30 ml deionisiertem Wasser versetzt. Insgesamt 16,40 g (0,16 mol) Calciumcarbonat und 41,60 g (0,16 mol) Iod wurden in acht etwa gleich großen Portionen so zugegeben, dass die Zeitspanne zwischen den Zugaben ca. 30 min betrug. Die Reaktion wurde mittels HPLC verfolgt. Um eine vollständige Umsetzung zu gewährleisten wurden insgesamt weitere 6 g Calciumcarbonat und 15 g Iod zugegeben. Nach 24 h wurde das rot-braune Reaktionsgemisch über eine mit Kieselgel gefüllte Fritte filtriert. Es wurde mit 150 ml THF nachgewaschen. Das Filtrat wurde bis zum Farbumschlag nach gelb mit ca. 200 ml gesättigter wässriger Natriumdithionit-Lösung versetzt. Anschließend wurde die Suspension filtriert und der Filtrationsrückstand mit 300 ml Ethylacetat gewaschen. Die beiden Phasen des Filtrats wurden getrennt. Die organische Phase wurde zwei mal mit je 100 ml Wasser gewaschen und die vereinigten Wasserphasen wurden mit 100 ml Ethylacetat reextrahiert. Die vereinigten organischen Phasen wurden mit 100 ml gesättigter wässriger NaCl-Lösung gewaschen und über wasserfreiem Natriumsulfat getrocknet. Nach Abtrennung des Trockenmittels wurde am Rotationsverdampfer eingeengt und der Rückstand wurde im Feinvakuum getrocknet. Das ölige, gelbe Rohprodukt wurde mittels Säulenchromatographie gereinigt (SiO₂, n-Hexan/Ethylacetat 9:1). Man erhielt 1,74 g (32% d. Th.) 1,6-Bis-{4-[(benzoyl-diethylgermio)carbonyl]phenoxy}hexan als gelben Feststoff (Schmp.: 78-80 °C).

¹H-NMR (400 MHz; CDCl₃): δ (ppm) : 7.68-7.76 (m, 8H, Ar-H), 7.46-7.52 (m, 2H, Ar-H), 7.38-7.44 und 6.85-6.89 (m, 2 x 4H, Ar-H), 3.97 (m, 4H, CH₂O), 1.77-1.80 (4H, OCH₂CH₂), 1.44-1.51 (m, 12H, OCH₂CH₂CH₂ + CH₂CH₃), 1.11 (t, 12H, CH₃).

¹³C-NMR (100 MHz; CDCl₃): δ (ppm): 230.7 und 226.6 (C=O), 163.3, 141.1, 134.8, 133.4, 130.5, 128.8, 128.0, 114.5 (alle Ar-C), 68.1 (CH2O), 28.9 und 25.7 (OCH₂CH₂CH₂), 8.98 (CH₃) 6.43 (CH₂CH₃).

### Beispiel 6: Synthese von 22,45-Digerma-22,22,45,45-tetraethyl-7,14,30,37-tetraoxa -21,23,44,46-tetraoxo[8.3.8.3](1,4) (1,4) (1,4) (1,4)cyclophan

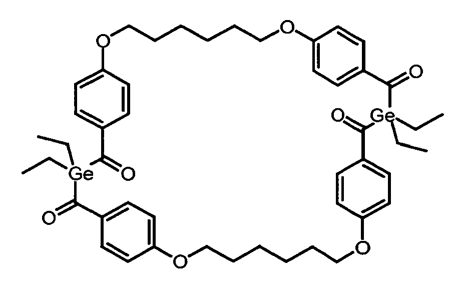

### 1. Stufe: 22,45-Digerma-22,22,45,45-tetraethyl-7,14,30,37-tetraoxa-21,23,44,46-tetrakis(2-phenyl-1,3-dithian-2-yl)[8.3.8.3](1,4) (1,4) (1,4) (1,4)cyclophan:

In einem trockenen 2 1 Dreihalskolben mit 250 ml Tropftrichter, Innenthermometer, Magnetrührer und Septum wurden unter Argon 40,55 g (80,0 mmol) 1,6-Bis[4-(1,3-dithian-2-yl)phenoxy]hexan (Beispiel 2, 2. Stufe) mit 800 ml wasserfreiem Tetrahydrofuran (THF) versetzt. Die Suspension wurde auf -5 °C abgekühlt. Innerhalb von 1 h wurden 70,4 ml einer 2,5 molaren Lösung von *n*-Butyllithium (0,176 mol) in *n*-Hexan so zugetropft, dass die Temperatur des Reaktionsgemischs 0 °C nicht überstieg. Nach beendeter Zugabe wurde die braune Lösung 3 h bei -5 °C gerührt. Anschließend wurde eine Lösung von 16,13 g (80,0 mmol) Diethylgermaniumdichlorid in 200 ml trockenem THF innerhalb von 2 h bei -5 °C zugetropft. Das Reaktionsgemisch wurde 16 h bei Raumtemperatur weitergerührt. Dann wurden 200 ml Wasser und 500 ml Ethylacetat zugegeben. Eine dabei abgeschiedene ölige, braune Masse wurde abgetrennt und verworfen. Die erhaltenen Phasen wurden getrennt. Die organische Phase wurde zwei mal mit je 200 ml Wasser gewaschen und die vereinigten wässrigen Phasen wurden mit 200 ml Ethylacetat reextrahiert. Die vereinigten organischen Phasen wurden mit 200 ml gesättigter wässriger NaCl-Lösung gewaschen und über wasserfreiem Natriumsulfat getrocknet. Nach Abtrennung des Trockenmittels wurde am Rotationsverdampfer eingeengt und der Rückstand im Feinvakuum bis zur Gewichtskonstanz getrocknet. Das Rohprodukt wurde unter Erhitzen am Rückfluss in 750 ml Dichlormethan gelöst und heiß filtriert. Das Filtrat wurde auf ca. ein Drittel des Volumens aufkonzentriert. Der nach dem Abkühlen gebildete Feststoff wurde abfiltriert, zwei mal mit je 20 ml Dichlormethan gewaschen und im Vakuumtrockenschrank bis zur Gewichtskonstanz getrocknet. Man erhielt 7,95 g (16% d. Th.) 22,45-Digerma-22,22,45,45-tetraethyl-7,14,30,37-tetraoxa-21,23,44,46-tetrakis(2-phenyl-1,3-dithian-2-yl)[8.3.8.3](1,4) (1,4) (1,4) (1,4)cyclophan als weißen Feststoff (Schmp.: 260-262 °C).

¹H-NMR (400 MHz; CDCl₃): δ (ppm) : 7.41 und 6.58 (d, 2 x 8H, Ar-H), 3.96 (t, 8H, CH₂O), 2.68-2.78 und 2.25-2.33 (m, 2 x 8H, CH₂S), 1.74-2.05 (m, 16H, CH₂CH₂O + CH₂CH₂S), 1.60-1.69 (m, 8H, CH₂CH₂CH₂O), 1.41-1.54 (m, 20H, CH₂CH₃.

### 2. Stufe: 22,45-Digerma-22,22,45,45-tetraethyl-7,14,30,37-tetraoxa-21,23,44,46-tetraoxo[8.3.8.3](1,4) (1,4) (1,4) (1, 4) cyclophan:

In einem 250 ml Zweihalskolben aus Braunglas mit Innenthermometer und Magnetrührer wurden 2,54 g (2,0 mmol) 22,45-Digerma-22,22,45,45-tetraethyl-7,14,30,37-tetraoxa-21,23,44,46-tetrakis(2-phenyl-1,3-dithian-2-yl)[8.3.8.3](1,4) (1,4) (1,4) (1,4)cyclophan mit 60 ml THF und 15 ml deionisiertem Wasser versetzt (die gesamte Synthese einschließlich der Reinigungsoperationen wurde unter Gelblicht durchgeführt). Insgesamt 4,80 g (48,0 mmol) Calciumcarbonat und 12,18 g (48,0 mmol) Iod wurden in acht etwa gleich großen Portionen so zugegeben, dass die Zeitspanne zwischen den Zugaben ca. 30 min betrug. Die Suspension wurde insgesamt 24 h bei Raumtemperatur gerührt. Dann wurde das rot-braune Reaktionsgemisch über eine mit Kieselgel gefüllte Fritte filtriert. Es wurde mit 50 ml Aceton nachgewaschen. Das Filtrat wurde bis zum Farbumschlag nach gelb mit ca. 50 ml gesättigter wässriger Natriumdithionit-Lösung versetzt. Anschließend wurde die Suspension filtriert und der Filtrationsrückstand mit 200 ml Ethylacetat gewaschen. Die beiden Phasen des Filtrats wurden getrennt. Die organische Phase wurde zwei mal mit je 50 ml Wasser gewaschen und die vereinigten Wasserphasen wurden zwei mal mit je 50 ml Ethylacetat reextrahiert. Die vereinigten organischen Phasen wurden mit 100 ml gesättigter wässriger NaCl-Lösung gewaschen und über wasserfreiem Natriumsulfat getrocknet. Nach Abtrennung des Trockenmittels wurde am Rotationsverdampfer eingeengt und der Rückstand wurde im Feinvakuum getrocknet. Der ölige, gelbe Feststoff wurde mittels Säulenchromatographie gereinigt (SiO₂, Dichlormethan). Man erhielt 22,45-Digerma-22,22,45,45-tetraethyl-7,14,30,37-tetraoxa-21,23,44,46-tetraoxo-[8.3.8.3](1,4) (1,4) (1,4) (1,4)cyclophan als gelben Feststoff (Schmp.: 162-164 °C).

¹H-NMR (400 MHz; CDCl₃): δ (ppm) : 7.67 und 6.83 (d, 2 x 8H, Ar-H), 3.95 (t, 8H, CH₂O), 1.71-1.81 (m, 8H, CH₂CH₂O), 1.39-1.50 (m, 16H, CH₂CH₂CH₂O + CH₂CH₃), 1.05-1.15 (t, 12H, CH₃).

¹³C-NMR (100 MHz; CDCl₃): δ (ppm): 227.5 (C=O), 163.2, 134.8, 130.5, 114.5 (alle Ar-C), 67.73 (CH2O), 28.7 und 25.3 (CH₂CH₂CH₂O), 9.00 (CH₃), 6.04 (CH₂CH₃).

## Patentansprüche

1. Zusammensetzung mit mindestens einem polymerisierbaren Bindemittel und einem Polymerisationsinitiator, **dadurch gekennzeichnet, daß** sie mindestens eine Acylgermanium-Verbindung mit mindestens 2 Germaniumatomen gemäß der allgemeinen Formel (I) enthält, 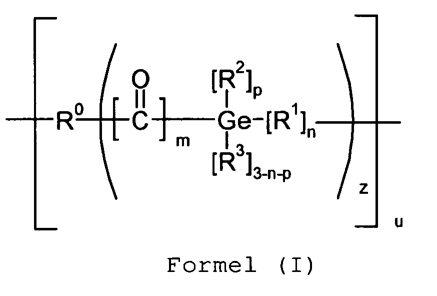 in der jeweils 1 bis 3 Acylgruppen an jedes Germaniumatom gebunden sind und
in der gemäß einer ersten Alternative
u 1 ist und die beiden yl-Stellen an R⁰ und R¹ entfallen,
z eine ganze Zahl von 2 bis 10 ist,
wobei
R⁰ ein z-wertiger verzweigter oder linearer aliphatischer, aromatischer oder aliphatisch- aromatischer Kohlenwasserstoffrest mit 1 bis 50 Kohlenstoffatomen und 0 bis 10 Heteroatomen ist, der z-fach durch die in runden Klammern stehende Gruppe substituiert ist, wobei R⁰ im Fall z = 2 oder 3 auch N bzw. NH, N-C₁₋₃-Alkyl oder N-Phenyl sein kann, und wobei R⁰ im Fall z = 2 und m = 0 auch entfallen kann, so daß zwei der in runden Klammern stehenden Gruppen durch eine chemische Bindung zwischen den Germaniumatomen miteinander verbunden sind, und wobei der Rest R⁰ durch ein oder mehrere Sauerstoffatome (=O), CN, Halogen, ein oder mehrere verzweigte oder lineare C₁₋₆-Alkylreste, -O-C₁₋₆-Alkylreste und/oder polymerisationsfähige Gruppen substituiert sein kann;
R¹, R² unabhängig voneinander 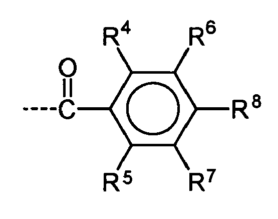 oder H sind, oder eine der für R³ angegebenen Bedeutungen haben; wobei
R⁴, R⁵ unabhängig voneinander jeweils H, Halogen, ein verzweigter oder linearer C₁₋₆-Alkyl- oder -O-C₁₋₆- Alkylrest sind, der durch ein oder mehrere Sauerstoffatome unterbrochen sein kann;
R⁶, R⁷, R⁸ unabhängig voneinander jeweils H, Halogen, ein verzweigter, cyclischer oder vorzugsweise linearer C₁₋₂₀-Alkyl-, -Alkenyl-, -Alkyloxy- oder -Alkenoxy- Rest sind, der durch ein oder mehrere O, S oder -NR²⁰- unterbrochen und durch eine oder mehrere polymerisationsfähige Gruppen und/oder Reste R⁹ substituiert sein kann, wobei R⁹ -OH, -CₓF₂ₓ₊₁ mit x = 1 bis 20, -[Si(CH₃)₂]_{y}-CH₃ mit y = 1 bis 20 ist, und R²⁰ H, Halogen, ein verzweigter, cyclischer oder vorzugsweise linearer C₁₋₂₀-Alkyl-, -Alkenyl-, -Alkyloxy- oder -Alkenoxy-Rest ist, und wobei R² auch ein linearer oder verzweigter aliphatischer, aromatischer oder aliphatisch- aromatischer Kohlenwasserstoffrest mit 1 bis 50 Kohlenstoffatomen und 0 bis 10 Heteroatomen sein kann, der zwei Germaniumatome miteinander verbrückt, oder in der gemäß einer zweiten Alternative
u eine ganze Zahl von 2 bis 100 ist und die beiden yl- Stellen an den endständigen Resten R⁰ und R¹, bzw. Ge wenn R¹ entfällt, durch H oder OH abgesättigt oder unter Ausbildung einer chemischen Bindung zwischen R⁰ und R¹ bzw. Ge miteinander verbunden sind,
z 1 ist,
wobei
R⁰ ein zweiwertiger linearer oder verzweigter aliphatischer, aromatischer oder aliphatisch- aromatischer Kohlenwasserstoffrest mit 1 bis 50 Kohlenstoffatomen und 0 bis 10 Heteroatomen ist, wobei R⁰ auch N-R²² oder -O-Si(R²³)₂-O- bedeuten kann, wobei R²² H, C₁₋₁₀-Alkyl oder Phenyl ist, und R²³ C₁₋₁₀- Alkyl ist oder zwei Reste R²³ eine Sauerstoffbrücke (-O-) zwischen zwei Si-Atomen bilden, und wobei der Rest R⁰ durch ein oder mehrere Sauerstoffatome (=O), CN, Halogen, ein oder mehrere verzweigte oder lineare C₁₋₆-Alkylreste, -O-C₁₋₆-Alkylreste und/oder polymerisationsfähige Gruppen substituiert sein kann;
R¹ -C(=O)- ist oder entfällt,
R² 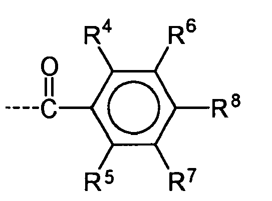 oder H ist
oder eine der für R³ angegebenen Bedeutungen hat;
wobei
R⁴, R⁵ unabhängig voneinander jeweils H, Halogen, ein verzweigter oder linearer C₁₋₆-Alkyl- oder -O-C₁₋₆- Alkylrest sind, der durch ein oder mehrere Sauerstoffatome unterbrochen sein kann;
R⁶, R⁷, R⁸ unabhängig voneinander jeweils H, Halogen, ein verzweigter, cyclischer oder vorzugsweise linearer C₁₋₂₀-Alkyl-, -Alkenyl-, -Alkyloxy- oder -Alkenoxy- Rest sind, der durch ein oder mehrere O, S oder -NR²⁰- unterbrochen und durch eine oder mehrere polymerisationsfähige Gruppen und/oder Reste R⁹ substituiert sein kann, wobei R⁹ -OH, -CₓF₂ₓ₊₁ mit x = 1 bis 20, -[Si(CH₃)₂]_{y}-CH₃ mit y = 1 bis 20 ist, und R²⁰ H, Halogen, ein verzweigter, cyclischer oder vorzugsweise linearer C₁₋₂₀-Alkyl-, -Alkenyl-, -Alkyloxy- oder -Alkenoxy-Rest ist,
und wobei die übrigen Variablen in beiden Alternativen die folgenden Bedeutungen haben:
m ist 0 oder 1,
n ist 0 oder 1,
p ist 0 oder 1,
R³ ist ein verzweigter oder linearer C₁₋₁₈-Alkyl-Rest oder C₂₋₁₈-Alkenyl-Rest, wobei diese Reste unsubstituiert oder einfach oder mehrfach durch einen Rest substituiert sein können, der aus der folgenden Gruppe ausgewählt ist: Halogen, CN, 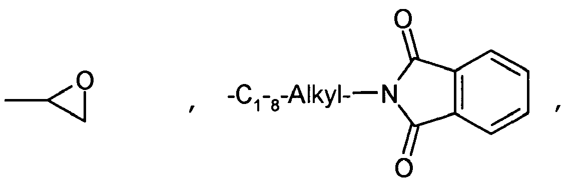 -OR¹⁰, -SR¹⁰, -OCO-R¹⁰, -COO-R¹⁰, -CH=CH-CO-OR¹⁰, -C(C₁₋₄-Alkyl)=C(C₁₋₄-Alkyl)-CO-OR¹⁰, -CO-R¹³, -CO-CH=CH-CO- C₁₋₆-Alkyl, -CO-CH=CH-CO-Phenyl, -CO-CH=CH-COO-C₁₋₁₈- Alkyl, -NR¹¹R¹², -N(R¹¹)-CO-R¹⁰, -N(R¹¹)-COO- R¹⁰, -N(R¹¹)-CO-NR¹¹R¹², -N(R¹¹)-CO-Hal, -CO-NR¹¹R¹², -SO₂-R¹⁰, -SO₂-OR¹⁰, -SO₂-NR¹¹R¹², -PO(OC₁₋₈-Alkyl)₂, -SiR¹⁴R¹⁵R¹⁶, - CH=CH-Phenyl, -C (C₁₋₄-Alkyl) =C (C₁₋₄-Alkyl)-Phenyl, Phenyl-C₁₋₄-Alkyl, Phenyl, Naphtyl, Biphenyl, C₅₋₁₂- Cycloalkyl, ein gesättigter oder ungesättigter 5- oder 6-gliedriger O-, S- oder N-haltiger heterocyclischer Ring, Benzophenonyl, Thisanthonyl, wobei
R¹⁰ H, C₁₋₁₈-Alkyl, C₁₋₁₈-Alkyl, das durch ein oder mehrere Sauerstoffatome unterbrochen ist, C₂₋₁₈-Alkenyl, C₂₋₁₈- Alkenyl, das durch ein oder mehrere Sauerstoffatome unterbrochen ist, C₃₋₁₂-Cycloalkyl, Tetrahydropyran-2- yl, Phenyl-C₁₋₂₀-Alkylen, Phenyl- C₁₋₂₀-Alkenylen, C₁₋₆- Alkyl, das unsubstituiert oder durch Halogen, Cyclohexyl, Cyclopentyl, Tetrahydrofuranyl, Furanyl oder Isopropyl-4-methyl-cyclohexyl substituiert sein kann, Phenyl, Naphthyl oder Biphenyl ist, wobei diese Ringsysteme unsubstituiert oder durch 1 bis 5 Halogenatome, C₁₋₈-Alkyl-, C₁₋₈-Alkoxy- und/oder C₁₋₈- Alkylthioreste substituiert sein können,
R¹¹, R¹² unabhängig voneinander H, C₁₋₁₈-Alkyl, C₁₋₁₈- Alkyl, das durch ein oder mehrere Sauerstoffatome unterbrochen ist, C₂₋₁₈-Alkenyl, C₂₋₁₈-Alkenyl, das durch ein oder mehrere Sauerstoffatome unterbrochen ist, C₃₋₁₂-Cycloalkyl, Phenyl-C₁₋₄-Alkyl, Phenyl, Naphthyl oder Pyridyl sind, wobei diese Ringsysteme unsubstituiert oder durch 1 bis 5 Halogenatome, C₁₋₈- Alkyl-, C₁₋₈-Alkoxy- und/oder C₁₋₈-Alkylthioreste substituiert sein können, oder R¹¹ und R¹² bilden zusammen einen 5- oder 6-gliedrigen O-, S- oder N- haltigen heterocyclischen Ring, der seinerseits mit einem aliphatischen oder aromatischen Ring anelliert sein kann,
R¹³ C₁₋₁₈-Alkyl, C₂₋₁₈-Alkenyl, das durch ein oder mehrere O-Atome unterbrochen ist, C₃₋₁₂-Cycloalkyl, Phenyl- C₁₋₄-Alkyl, Phenyl, Naphtyl oder Biphenyl ist, wobei die genannten Ringsysteme unsubstituiert oder durch 1 bis 5 C₁₋₈-Alkyl-, C₁₋₈-Alkoxy-, C₁₋₈-Alkylthioreste und/oder Halogenatome substituiert sein können;
R¹⁴, R¹⁵, R¹⁶ unabhängig voneinander jeweils H, C₁₋₈- Alkyl, C₂₋₈-Alkenyl, C₇₋₉-Phenylalkyl, -O-C₁₋₈-Alkyl, Phenyl oder -O-SiR¹⁷R¹⁸R¹⁹ sind, wobei R¹⁷, R¹⁸, R¹⁹ unabhängig voneinander jeweils H, C₁₋₈- Alkyl, C₂₋₈-Alkenyl, C₇₋₉-Phenylalkyl, -O-C₁₋₈-Alkyl oder Phenyl sind,
oder
R³ ist ein verzweigter oder vorzugsweise linearer C₂₋₁₈- Alkyl-Rest oder ein C₂₋₁₈-Alkylen-Rest, der einfach oder mehrfach durch -O-, -NH-, -NR¹¹-, -S- unterbrochen ist, wobei die Reste unsubstituiert oder einfach oder mehrfach durch einen Rest substituiert sein können, der aus der folgenden Gruppe gewählt ist: Halogen, CN, -OR¹⁰, -SR¹⁰ -OCO-R¹⁰, -COO-R¹⁰, -NR¹¹R¹², -N(R¹¹)-CO-R¹⁰, -N(R¹¹)-COO-R¹⁰, -N(R¹¹)-CO-NR¹¹R¹², -N(R¹¹)-CO-Hal, -CO-NR¹¹R¹², -SO₂-R¹⁰, -SO₂-OR¹⁰, -SO₂- NR¹¹R¹², -PO(OC₁₋₈-Alkyl)₂, -SiR¹⁴R¹⁵R¹⁶, Phenyl-C₁₋₄- Alkyl, Phenyl, C₅₋₁₂-Cycloalkyl; wobei R¹⁰, R¹¹, R¹², R¹⁴, R¹⁵ und R¹⁶ wie oben definiert sind;
oder
R³ ist ein verzweigter oder vorzugsweise linearer C₂₋₁₈- Alkyl-Rest oder ein C₂₋₁₈-Alkylen-Rest, der einfach oder mehrfach durch -CO-, -COO-, -OCO-, -OCOO- -CO- N(R¹¹)-, -N(R¹¹)-CO-, -N(R¹¹)-CO-N(R¹¹)-, -N(R¹¹)-COO-, -COO-C₁₋₆-Alkylen, -COS-C₁₋₁₈-Alkylen, -SO₂-, -SO₂-O-, -SO₂-N(R¹¹)-, -(CH₃)₂Si[OSi(CH₃)₂]_{q}-, mit q = 1 bis 6; Phenyl-C₁₋₄-Alkylen, Phenylen, Naphthylen, Biphenylen, C₅₋₁₂-Cycloakylen oder einen 5- oder 6-gliedrigen O-, S- oder N-haltigen heterocyclischen Ring unterbrochen ist;
wobei R¹¹ wie oben definiert ist;
oder
R³ ist Trimethylsilyl, Hal-(CH₃)₂Si-[OSi(CH₃)₂]ᵣ-, (CH₃)₃Si-[OSi(CH₃)₂]ᵣ,- mit r = 1 bis 6, -COOH, -COO- R¹⁰, -CO-NR¹¹R¹², -CO-Vinyl, -CO-Phenyl, wobei der Phenylrest unsubstituiert oder durch -CH₃, -OCH₃ und/oder -Cl substituiert sein kann; wobei R¹⁰, R¹¹ und R¹² wie oben definiert sind;
oder
R³ ist Phenyl-C₁₋₂₀-Alkyl, Phenyl, Naphthyl oder Biphenyl, C₅₋₁₂-Cycloalkyl oder ein gesättigter oder ungesättigter 5- oder 6-gliedriger O-, S- oder N- haltiger heterocyclischer Ring, wobei diese Ringsysteme unsubstituiert oder durch 1 bis 5 Halogenatome, C₁₋₈-Alkyl-, C₁₋₈-Alkoxy-, C₁₋₈- Alkylthioreste und/oder -NR¹¹R¹² substituiert sein können,
wobei R¹¹ und R¹² wie oben definiert sind,
wobei jeweils zwei der Reste R¹, R² oder R³ unter Ausbildung eines 5- bis 8-gliedrigen Rings, der seinerseits mit einem oder mehreren aliphatischen oder aromatischen Ringen anelliert sein kann, miteinander verbunden sein können, wobei diese Ringe neben dem Germaniumatom weitere Heteroatome enthalten können, und wobei unterschiedliche Reste oder im Fall von m, n, p oder 3-n-p > 1 auch gleiche Reste unter Ausbildung eines oder mehrerer Ringe miteinander verbunden sein können, und wobei die gebildeten Ringe unsubstituiert oder ein- oder mehrfach, substituiert sein können.

2. Zusammensetzung nach Anspruch 1, die mindestens eine Verbindung gemäß der Formel (I') enthält, 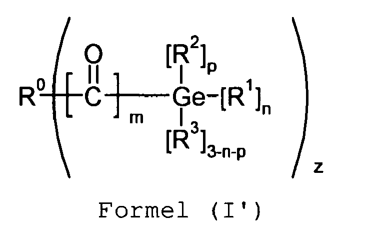 in der
R⁰ eine Alkylgruppe mit 1 bis 6 Kohlenstoffatomen ist, wobei z Wasserstoffatome dieser Gruppe durch den Klammerausdruck der Formel (I') substituiert sind,
R¹, R² unabhängig voneinander 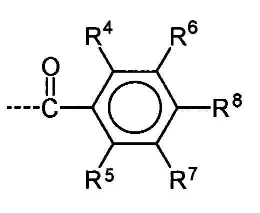 oder H sind oder eine der für R³ angegebenen Bedeutungen haben, wobei
R⁴, R⁵ unabhängig voneinander jeweils H, Halogen, ein verzweigter oder linearer C₁₋₆-Alkyl- oder -O-C₁₋₆- Alkylrest sind;
R⁶, R⁷, R⁸ unabhängig voneinander jeweils H, Halogen, ein verzweigter, cyclischer oder vorzugsweise linearer C₁₋₂₀-Alkyl-, -Alkenyl-, -Alkyloxy- oder -Alkenoxy- Rest sind, der durch ein oder mehrere O, S oder -NR'- unterbrochen und durch eine oder mehrere polymerisationsfähige Gruppen und/oder Reste R⁹ substituiert sein kann, wobei R' H, Halogen, ein verzweigter, cyclischer oder vorzugsweise linearer C₁₋₂₀-Alkyl-, -Alkenyl-, -Alkyloxy- oder -Alkenoxy- Rest ist;
R³ ein aromatischer C₆₋₁₀-Rest ist, der durch einen verzweigten, oder vorzugsweise linearen C₁₋₁₈-Alkyl-, -Alkenyl-, -Alkoxy- oder -Alkenoxy-Rest substituiert sein kann, wobei die genannten Reste durch ein oder mehrere O-Atome unterbrochen sein können, oder ein verzweigter oder vorzugsweise linearer C₁₋₁₈-Alkyl-, -Alkenyl-, -Alkoxy- oder -Alkenoxy-Rest ist, der ein- oder mehrfach durch O - unterbrochen sein kann,
m 0 oder 1,
n 0 oder 1,
p 0 oder 1,
z 2 bis 6 ist.

3. Zusammensetzung nach Anspruch 1, die mindestens eine Acylgermanium-Verbindung gemäß der allgemeinen Formel (II) enthält, 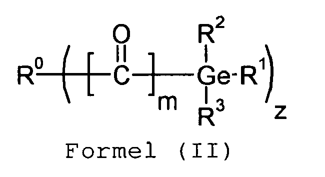 in der
R¹, R², R³ unabhängig voneinander 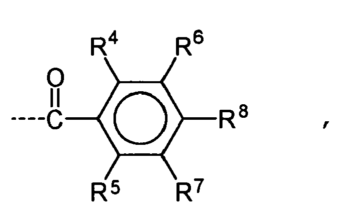 ein C₁₋₃-Alkylrest oder eine C₁₋₃-Acylgruppe sind;
R⁴, R⁵ unabhängig voneinander jeweils H, Halogen, ein linearer oder verzweigter C₁₋₆-Alkyl- oder -O-C₁₋₆- Alkylrest sind;
R⁶, R⁷, R⁸ unabhängig voneinander jeweils H, Halogen, ein verzweigter, cyclischer oder vorzugsweise linearer C₁₋₂₀-Alkyl-, -Alkenyl-, -Alkyloxy- oder -Alkenoxy- Rest sind, der ein- oder mehrfach durch O, S oder -NR²⁰- unterbrochen und durch eine oder mehrere polymerisationsfähige Gruppen und/oder Reste R⁹ substituiert sein kann;
R⁹ -OH, -CₓF₂ₓ₊₁ mit x = 1 bis 20, -[Si(CH₃)₂]_{y}-CH₃ mit y = 1 bis 20 ist; und
R²⁰ H, Halogen, ein verzweigter, cyclischer oder vorzugsweise linearer C₁₋₂₀-Alkyl-, -Alkenyl-, -Alkyloxy- oder -Alkenoxy-Rest ist.

4. Zusammensetzung nach Anspruch 1, die mindestens eine Acylgermanium-Verbindung gemäß der allgemeinen Formel (III) enthält, 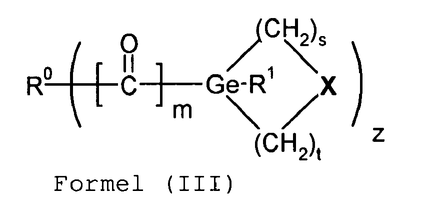 in der
R¹ 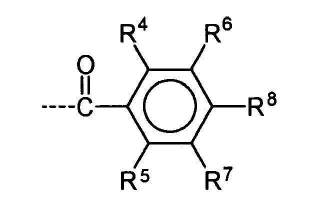 oder
ein C₁₋₃-Alkylrest oder eine C₁₋₃-Acylgruppe ist;
s,t unabhängig voneinander eine ganze Zahl von 0 bis 6 sind, wobei s und t so gewählt sind, daß die Summe der Ringatome einschließlich des Germaniumatoms 5 bis 8 beträgt, und
X N-R²¹, O, S oder entfällt bedeutet, wobei R²¹ H oder _{Cl-10}-_{A}lkyl ist,
wobei der Germanium enthaltende Ring mit einem oder mehreren aliphatischen oder aromatischen Ringen anelliert und unsubstituiert oder ein- oder mehrfach substituiert sein kann, wodurch die Anzahl der Wasserstoffatome des Rings entsprechend reduziert wird.

5. Zusammensetzung nach Anspruch 1, die mindestens eine Acylgermanium-Verbindung mit 2 Germaniumatomen gemäß der allgemeinen Formel (IV) enthält, 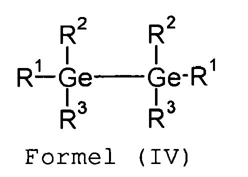 in der
R¹, R² und R³ unabhängig voneinander 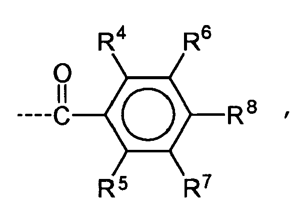 ein C₁₋₃-Alkylrest oder eine C₁₋₃-Acylgruppe sind;
R⁴, R⁵ unabhängig voneinander jeweils H, Halogen, ein linearer oder verzweigter C₁₋₆-Alkyl- oder -O-C₁₋₆- Alkylrest sind;
R⁶, R⁷, R⁸ unabhängig voneinander jeweils H, Halogen, ein verzweigter, cyclischer oder vorzugsweise linearer C₁₋₂₀-Alkyl-, -Alkenyl-, -Alkyloxy- oder -Alkenoxy- Rest sind, der ein- oder mehrfach durch O, S oder -NR²⁰- unterbrochen und durch eine oder mehrere polymerisationsfähige Gruppen und/oder Reste R⁹ substituiert sein kann;
R⁹ -OH, -CₓF₂ₓ₊₁ mit x = 1 bis 20, - [Si(CH₃)₂]_{y}-CH₃ mit y = 1 bis 20 ist; und
R²⁰ H, Halogen, ein verzweigter, cyclischer oder vorzugsweise linearer C₁₋₂₀-Alkyl-, -Alkenyl-, -Alkyloxy- oder -Alkenoxy-Rest ist.

6. Zusammensetzung nach Anspruch 1, die mindestens eine Acylgermanium-Verbindung mit 2 Germaniumatomen gemäß der allgemeinen Formel (V) enthält, 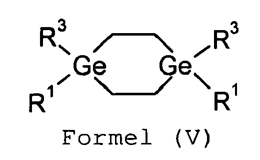 in der R¹ und R³ unabhängig voneinander 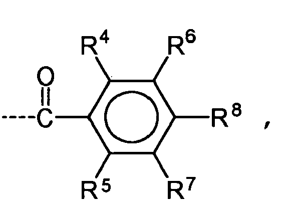 ein C₁₋₃-Alkylrest oder eine C₁₋₃-Acylgruppe sind;
R⁴, R⁵ unabhängig voneinander jeweils H, Halogen, ein linearer oder verzweigter C₁₋₆-Alkyl- oder -O-C₁₋₆- Alkylrest sind;
R⁶, R⁷, R⁸ unabhängig voneinander jeweils H, Halogen, ein verzweigter, cyclischer oder vorzugsweise linearer C₁₋₂₀-Alkyl-, -Alkenyl-, -Alkyloxy- oder -Alkenoxy- Rest sind, der ein- oder mehrfach durch O, S oder -NR²⁰- unterbrochen und durch eine oder mehrere polymerisationsfähige Gruppen und/oder Reste R⁹ substituiert sein kann;
R⁹ -OH, -CₓF₂ₓ₊₁ mit x = 1 bis 20, - [Si(CH₃)₂]_{y}-CH₃ mit y = 1 bis 20 ist; und
R²⁰ H, Halogen, ein verzweigter, cyclischer oder vorzugsweise linearer C₁₋₂₀-Alkyl-, -Alkenyl-, -Alkyloxy- oder -Alkenoxy-Rest ist.

7. Zusammensetzung nach Anspruch 1, die mindestens eine Acylgermanium-Verbindung mit mindestens 2 Germaniumatomen gemäß der allgemeinen Formel (VI) enthält, 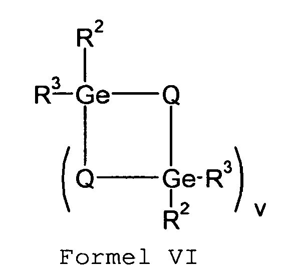 in der
R², R³ unabhängig voneinander 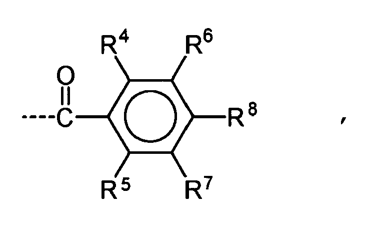 ein C₁₋₃-Alkylrest oder eine C₁₋₃-Acylgruppe sind;
v eine ganze Zahl von 1 bis 3 ist, und
Q N-R²², -CH₂-, -CH=CH-, -CH₂-CH₂- oder -O-Si(R²³)₂-O- bedeutet, wobei R²² H, C₁₋₁₀-Alkyl, vorzugsweise H oder C₁₋₄-Alkyl, oder Phenyl ist, und R²³ C₁₋₁₀-Alkyl ist oder zwei Reste R²³ eine Sauerstoffbrücke (-O-) zwischen zwei Si-Atomen bilden.

8. Zusammensetzung nach einem der vorhergehenden Ansprüche, bei der die Reste R², R³, R⁶, R⁷ und R⁸ jeweils mit 1 bis 3 polymerisierbaren Gruppe substituiert sind.

9. Zusammensetzung nach einem der vorhergehenden Ansprüche, bei der die polymerisationsfähigen Gruppen aus Vinyl, Styryl, (Meth)acrylat, (Meth)acrylamid oder N-Alkylacrylamid ausgewählt sind.

10. Zusammensetzung nach einem der vorhergehenden Ansprüche, die bezogen auf die Gesamtmasse der Zusammensetzung 0,001 bis 5 Gew.-% der Acylgermanium-Verbindung enthält.

11. Zusammensetzung nach einem der vorhergehenden Ansprüche, die als polymerisierbares Bindemittel mindestens ein radikalisch polymerisierbares Monomer und/oder Prepolymer enthält.

12. Zusammensetzung nach Anspruch 11, die als Bindemittel ein mono- oder multifunktionelles (Meth)acrylat oder eine Mischung davon enthält.

13. Zusammensetzung nach Anspruch 11 oder 12, die mindestens ein radikalisch ringöffnend polymerisierbares Monomer enthält.

14. Zusammensetzung nach einem der vorhergehenden Ansprüche, die als Bindemittel eine Mischung von mono- und/oder multifunktionellen Mercaptoverbindungen und di- und/oder multifunktionellen ungesättigten Monomeren enthält.

15. Zusammensetzung nach einem der vorhergehenden Ansprüche, die mindestens einen weiteren Initiator für die radikalische Polymerisation enthält.

16. Zusammensetzung nach einem der vorhergehenden Ansprüche, die mindestens einen weiteren Initiator für die kationische Polymerisation enthält.

17. Zusammensetzung nach einem der vorhergehenden Ansprüche, die zusätzlich Füllstoff enthält.

18. Zusammensetzung nach einem der vorhergehenden Ansprüche, die zusätzlich mindestens ein Additiv enthält, das aus Stabilisatoren, UV-Absorbern, Gleitmitteln, Netzmitteln, Dispergiermitteln, Haftvermittlern, Mattierungs- und Glanzmitteln, Verlaufs- und Filmbildehilfsmitteln, Hautverhinderungsmitteln, Lichtschutzmitteln, Korrosionsschutzmitteln, flammhemmenden Mitteln, Antioxidantien, optischen Aufhellern, Fließverbesserern, Verdickern und Antischaummitteln ausgewählt ist.

19. Zusammensetzung nach einem der vorhergehenden Ansprüche, die
0,001 bis 5 Gew.-% Acylgermanium-Verbindung,
5 bis 99,9 Gew.-% polymerisierbares Bindemittel,
0 bis 90 Gew.-% Füllstoff enthält, jeweils bezogen auf die Gesamtmasse der Zusammensetzung.

20. Zusammensetzung nach Anspruch 19, die 0 bis 50 Gew.-% weiteres Additiv enthält.

21. System zur Herstellung von Formkörpern, das eine Zusammensetzung gemäß einem der Ansprüche 1 bis 20 und eine LED-Lichtquelle umfaßt.

22. System nach Anspruch 21, bei dem die LED-Lichtquelle eine Wellenlänge im Bereich von 400 bis 550 nm hat und die Acylgermanium-Verbindung eine Aktivierungswellenlänge im Bereich von 400 bis 550 nm aufweist.

23. Verwendung eines Acylgermans gemäß Formel (I) als Initiator für die radikalische Polymerisation.

24. Verwendung eines Acylgermans gemäß Formel (I) zur Verhinderung der Ausbildung einer Inhibierungsschicht bei der radikalischen Polymerisation.

25. Verwendung eines Acylgermans gemäß Formel (I) zur Herstellung von Adhäsiven, Beschichtungen, Zementen, Kompositen, Formteilen oder Dentalwerkstoffen.

26. Verfahren zur Herstellung eines Formkörpers, bei dem man eine Zusammensetzung gemäß einem der Ansprüche 1 bis 20 zu einem Körper mit der gewünschten Form formt und anschließend ganz oder teilweise härtet.

27. Verfahren nach Anspruch 26, bei dem man den Körper durch Bestrahlen mit Licht einer Wellenlänge von 200 bis 700 nm härtet.

28. Verfahren nach Anspruch 27, bei dem man den Körper durch Bestrahlen mit Licht einer Wellenlänge von 300 bis 550 nm härtet.

29. Verfahren nach Anspruch 27, bei dem man den Körper durch Bestrahlen mit Licht einer Wellenlänge von 400 bis 800 nm härtet.

30. Verfahren nach einem der Ansprüche 26 bis 29 zur Herstellung von dentalen Kronen, Brücken, Inlays oder künstlichen Zähnen.

## Claims

1. Composition with at least one polymerisable binder and a polymerisation initiator, said composition comprising at least one acylgermanium compound containing at least 2 germanium atoms according to the general Formula (I), 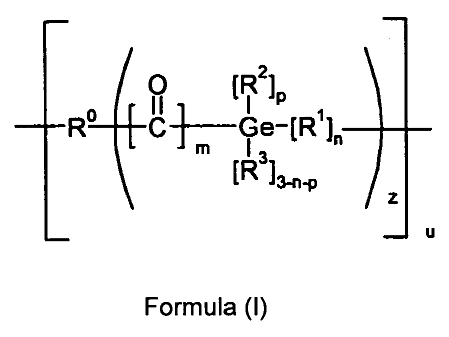 in which 1 to 3 acyl groups are bonded to each germanium atom and
in which according to a first alternative
u is 1 and both yl-positions on R⁰ and R¹ are missing,
z is a whole number from 2 to 10,
wherein
R⁰ is a z-valent branched or linear aliphatic, aromatic or aliphatic- aromatic hydrocarbon group containing 1 to 50 carbon atoms and 0 to 10 heteroatoms which is z-times substituted by the group in the round brackets, wherein R⁰ in the case that z = 2 or 3 can also be N or NH, N-C₁₋₃ alkyl or N-phenyl, and wherein R⁰ in the case where z = 2 and m = 0 can also be absent, such that two of the groups in the round brackets are bonded to each other through a chemical bond between the germanium atoms, and wherein the R⁰ group can be substituted by one or more oxygen atoms (=O), CN, halogen, one or more branched or linear C₁₋₆ alkyl groups, -O-C₁₋₆ alkyl groups and/or polymerisable groups;
R¹, R² independently of one another are 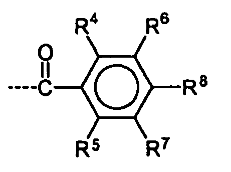 or H, or have one of the meanings cited for R³; wherein
R⁴, R⁵ independently of one another are each H, halogen, a branched or linear C₁₋₆ alkyl group, -O-C₁₋₆ alkyl group that can be interrupted by one or more oxygen atoms;
R⁶, R⁷, R⁸ are independently of each other H, halogen, a branched, cyclic or preferably linear alkyl, alkenyl, alkyloxy or alkenoxy C₁₋₂₀ group, which can be interrupted by one or more O, S or -NR²⁰ and substituted by one or more polymerisable groups and/or R⁹ groups, wherein R⁹ is -OH, -CₓF₂ₓ₊₁ with x = 1 to 20, -[Si(CH₃)₂]_{y}-CH₃ with y = 1 to 20, and R²⁰ is H, halogen, a branched, cyclic or preferably linear alkyl, alkenyl, alkyloxy or alkenoxy C₁₋₂₀ group, and wherein R² can also be a linear or branched aliphatic, aromatic or aliphatic-aromatic hydrocarbon group containing 1 to 50 carbon atoms and 0 to 10 heteroatoms which bridges two germanium atoms with one another,
or in a second alternative
u is a whole number from 2 to 100 and both yl-positions on the terminal groups R⁰ and R¹, or Ge when R¹ is absent, are saturated by H or OH or are bonded together with the formation of a chemical bond between R⁰ and R¹ or Ge,
z is 1,
wherein
R⁰ is a divalent linear or branched aliphatic, aromatic or aliphatic- aromatic hydrocarbon group containing 1 to 50 carbon atoms and 0 to 10 heteroatoms, wherein R⁰ can also mean N-R²² or -O- Si(R²³)₂-O-, wherein R²² is H, C₁₋₁₀ alkyl or phenyl, and R²³ is C₁₋₁₀ alkyl or two R²³ groups form an oxygen bridge (-O-) between two Si atoms, and wherein the R⁰ group can be substituted by one or more oxygen atoms (=O), CN, halogen, one or more branched or linear C₁₋₆ alkyl groups, -O-C₁₋₆ alkyl groups and/or polymerisable groups;
R¹ is -C(=O) or is absent,
R² is 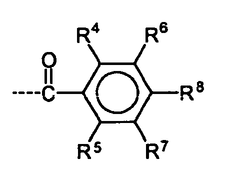 or H
or has one of the meanings cited for R³; wherein
R⁴, R⁵ independently of one another are each H, halogen, a branched or linear C₁-₆ alkyl group, -O-C₁₋₆ alkyl group that can be interrupted by one or more oxygen atoms;
R⁶, R⁷, R⁸ are independently of each other H, halogen, a branched, cyclic or preferably linear alkyl, alkenyl, alkyloxy or alkenoxy C₁₋₂₀ group, which can be interrupted by one or more O, S or -NR²⁰ and substituted by one or more polymerisable groups and/or R⁹ groups, wherein R⁹ is - OH, -CₓF₂ₓ₊₁ with x = 1 to 20, -[Si(CH₃)₂]_{y}-CH₃ with y = 1 to 20, and R²⁰ is H, halogen, a branched, cyclic or preferably linear alkyl, alkenyl, alkyloxy or alkenoxy C₁₋₂₀ group,
and wherein the other variables in both alternatives have the following meanings:
m is 0 or 1,
n is 0 or 1,
p is 0 or 1,
R³ is a branched or linear C₁₋₁₈ alkyl group or a C₂₋₁₈ alkenyl group, wherein these groups can be unsubstituted or mono or polysubstituted by a group that is selected from the following groups:
halogen, CN, 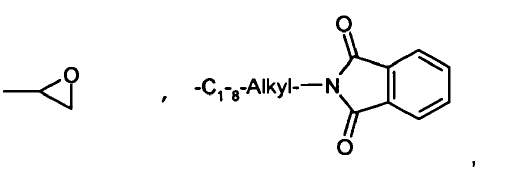 -OR¹⁰, -SR¹⁰, -OCO-R¹⁰, -COO-R¹⁰, -CH=CH-CO-OR¹⁰, -C(C₁₋₄ alkyl)=C(C₁₋₄ alkyl)-CO-OR¹⁰, -CO-R¹³, -CO-CH=CH-CO-C₁₋₆ alkyl, -CO-CH=CH-CO phenyl, -CO-CH=CH-COO-C₁₋₁₈ alkyl, -NR¹¹R¹², -N(R¹¹)-CO-R¹⁰, -N(R¹¹)-COO-R¹⁰, -N(R¹¹)-CO-NR¹¹R¹², -N(R¹¹)-CO-hal, -CO-NR¹¹R¹², -SO₂-R¹⁰, SO₂-OR¹⁰, -SO₂₋NR¹¹R¹², -PO(OC₁₋₈ alkyl)₂, - SiR¹⁴R¹⁵R¹⁶, -CH=CH phenyl, -C(C₁₋₄ alkyl)=C(C₁₋₄ alkyl)-phenyl, phenyl-(C₁₋₄ alkyl), phenyl, naphthyl, biphenyl, C₅₋₁₂ cycloalkyl, a saturated or unsaturated 5- or 6-membered O-, S- or N-containing heterocyclic ring, benzophenonyl, tris antimonyl, wherein
R¹⁰ is H, C₁₋₁₈ alkyl, C₁₋₁₈ alkyl, which is interrupted by one or more oxygen atoms, C₂₋₁₈ alkenyl, C₂₋₁₈ alkenyl, which is interrupted by one or more oxygen atoms, C₃₋₁₂ cycloalkyl, tetrahydropyran-2-yl, phenyl-C₁₋₂₀ alkylene, phenyl-C₁₋₂₀ alkenylene, C₁₋₆ alkyl, which can be unsubstituted or substituted by halogen, cyclohexyl, cyclopentyl, tetrahydrofuranyl, furanyl or isopropyl-4-methyl-cyclohexyl, phenyl, naphthyl or biphenyl, wherein these ring systems can be unsubstituted or substituted by 1 to 5 halogen atoms, C₁₋₈ alkyl, C₁₋₈ alkoxy and/or C₁₋₈- alkylthio groups,
R¹¹, R¹² is H, C₁₋₁₈ alkyl, C₁₋₁₈ alkyl, which is interrupted by one or more oxygen atoms, C₂₋₁₈ alkenyl, C₂₋₁₈ alkenyl, which is interrupted by one or more oxygen atoms, C₃₋₁₂ cycloalkyl, phenyl-C₁₋₄ alkyl, phenyl, naphthyl or pyridyl, wherein these ring systems can be unsubstituted or substituted by 1 to 5 halogen atoms, C₁₋₈ alkyl, C₁₋₈ alkoxy and/or C₁₋₈ alkylthio groups, or R¹¹, R¹² together form a 5- or 6-membered O-, S- or N-containing heterocyclic ring that itself can be anellated with an aliphatic or aromatic ring,
R¹³ is C₁₋₁₈ alkyl, C₂₋₁₈ alkenyl, which is interrupted by one or more oxygen atoms, C₃₋₁₂ cycloalkyl, phenyl-C₁₋₄ alkyl, phenyl, naphthyl or biphenyl, wherein the cited ring systems can be unsubstituted or substituted by 1 to 5 C₁₋₈ alkyl, C₁₋₈ alkoxy, C₁₋₈ alkylthio groups and/or halogen atoms;
R¹⁴, R¹⁵, R¹⁶ are independently of each other H, C₁₋₈ alkyl, C₂₋₈ alkenyl, C₇₋₈ phenylalkyl, -O-C₁₋₈ alkyl, phenyl or -O-SiR¹⁷R¹⁸R¹⁹, wherein
R¹⁷, R¹⁸, R¹⁹ are independently of each other H, C₁₋₈ alkyl, C₂₋₈ alkenyl, C₇₋₈ phenylalkyl, -O-C₁₋₈ alkyl or phenyl,
or
R³ is a branched or preferably linear C₂₋₁₈ alkyl group or a C₂₋₁₈ alkenyl group, which is interrupted by one or a plurality of -O-, -NH-, - NR¹¹-, -S- moieties, wherein the groups can be unsubstituted or mono or polysubstituted by a group that is selected from the following groups:
halogen, CN, -OR¹⁰, -SR¹⁰, -OCO-R¹⁰, -COO-R¹⁰, -NR¹¹R¹², N(R¹¹)-COR¹⁰, -N(R¹¹)-COO-R¹⁰, -N(R¹¹)-CO-NR¹¹R¹², -N(R¹¹)-CO-hal, -CO-NR¹¹R¹², -SO₂-R¹⁰, -SO₂-OR¹², -SO₂-NR¹¹R¹², -PO(OC₁₋₈ alkyl)₂, - SiR¹⁴R¹⁵R¹⁶, phenyl-C₁₋₄ alkyl, phenyl, C₅₋₁₂ cycloalkyl;
wherein R¹⁰, R¹¹, R¹², R¹⁴, R¹⁵ and R¹⁶ are defined as above;
or
R³ is a branched or preferably linear C₂₋₁₈ alkyl group or a C₂₋₁₈ alkenyl group, which is interrupted by one or a plurality of the moieties - CO-, -COO-, -OCO-, -OCOO-, -CO-N(R¹¹)-, -N(R¹¹)-CO-, -N(R¹¹)-CON(R¹¹)-, -N(R¹¹)-COO-, COO-C₁₋₆ alkylene, -COS-C₁₋₁₈ alkylene, -SO₂-, - SO₂-O-, -SO₂-N(R¹¹)-, -(CH₃)₂Si[OSi(CH₃)₂]_{q}-, with q = 1 to 6; phenyl-C₁₋₄ alkylene, phenylene, naphthylene, biphenylene, C₅₋₁₂ cycloakylene or a 5- or 6-membered O-, S- or N-containing heterocyclic ring;
wherein R¹¹ is as described above;
or
R³ is trimethylsilyl, hal-(CH₃)₂Si-[OSi(CH₃)₂]ᵣ-, (CH₃)₃Si-[OSi(CH₃)₂]ᵣ- with r = 1 to 6, -COOH, -COO-R¹⁰, -CO-NR¹¹R¹², -CO-vinyl, -CO-phenyl, wherein the phenyl group can be unsubstituted or substituted by -CH₃, - OCH₃ and/or -Cl;
wherein R¹⁰, R¹¹ and R¹² are as described above;
or
R³ is phenyl-C₁₋₂₀ alkyl, phenyl, naphthyl or biphenyl, C₅₋₁₂ cycloalkyl or a saturated or unsaturated 5- or 6-membered O-, S- or N-containing heterocyclic ring, wherein these ring systems can be unsubstituted or substituted by 1 to 5 halogen atoms, C₁₋₈ alkyl, C₁₋₈ alkoxy, C₁₋₈ alkylthio groups and/or -NR¹¹R¹²,
wherein R¹¹ and R¹² are as described above,
wherein two of the groups R¹, R² or R³ can be bonded to one another by forming a 5- or 8-membered ring that itself can be anellated with one or more aliphatic or aromatic rings, wherein these rings can comprise additional heteroatoms besides the germanium atom, and wherein different groups, or in the case of m, n, p or 3-n-p > 1, also the same groups can be bonded to one another by forming one or more rings, and wherein the formed rings can be unsubstituted or mono or polysubstituted.

2. Composition according to claim 1, comprising at least one compound according to the Formula(I'), 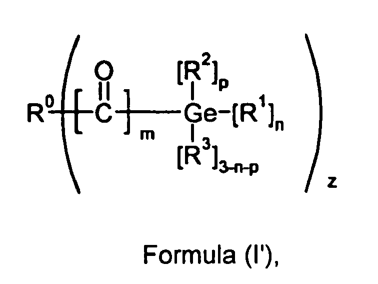 in which
R⁰ is an alkyl group containing 1 to 6 carbon atoms, wherein z hydrogen atoms of this group are substituted by the expression in brackets of the Formula (I'),
R¹, R² independently of one another are 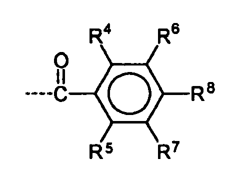 or H or have one of the meanings cited for R³; wherein
R⁴, R⁵ independently of one another are each H, halogen, a branched or linear C₁₋₆ alkyl or -O-C₁₋₆ alkyl group;
R⁶, R⁷, R⁸ are independently of each other H, halogen, a branched, cyclic or preferably linear alkyl, alkenyl, alkyloxy or alkenoxy C₁₋₂₀ group, which can be interrupted by one or more O, S or -NR' and substituted by one or more polymerisable groups and/or R⁹ groups, wherein R' is H, halogen, a branched, cyclic or preferably linear alkyl, alkenyl, alkyloxy or alkenoxy C₁₋₂₀ group;
R³ is an aromatic C₆₋₁₀ group that can be substituted by a branched, or preferably linear alkyl, alkenyl, alkyloxy or alkenoxy C₁₋₁₈ group, wherein the cited groups can be interrupted by one or more O atoms, or is a branched or preferably linear alkyl, alkenyl, alkyloxy or alkenoxy C₁₋₁₈ group that can be interrupted by one or more O- moieties,
m is 0 or 1,
n is 0 or 1,
p is 0 or 1,
z is 2 to 6.

3. Composition according to claim 1, comprising at least one acylgermanium compound according to the general Formula(II), 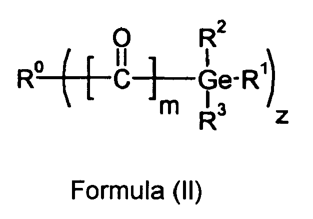 in which
R¹, R², R³ independently of each other are 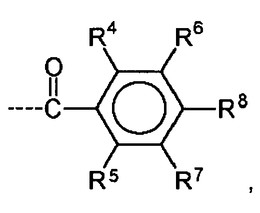 a C₁₋₃ alkyl group or a C₁₋₃ acyl group;
R^{4,} R⁵ independently of one another are each H, halogen, a branched or linear C₁₋₆ alkyl or -O-C₁₋₆ alkyl group;
R⁶, R⁷, R⁸ are independently of each other H, halogen, a branched, cyclic or preferably linear alkyl, alkenyl, alkyloxy or alkenoxy C₁₋₂₀ group, which can be interrupted by one or more O, S or -NR²⁰ and can be substituted by one or more polymerisable groups and/or R⁹ groups;
R⁹ is -OH, -CₓF₂ₓ₊₁ with x = 1 to 20, -[Si (CH₃)₂]_{y}-CH₃ with y = 1 to 20; and
R²⁰ is H, halogen, a branched, cyclic or preferably linear alkyl, alkenyl, alkyloxy or alkenoxy C₁₋₂₀ group.

4. Composition according to claim 1, comprising at least one acylgermanium compound according to the general Formula(III), 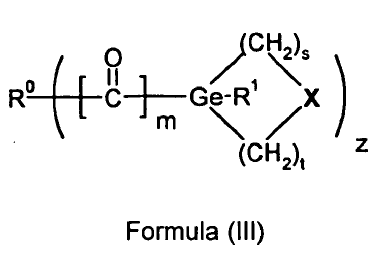 in which
R¹ is 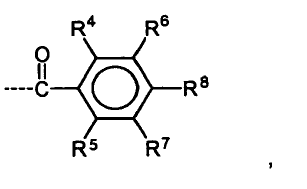 a C₁₋₃ alkyl group or a C₁₋₃ acyl group;
s,t are independently of one another a whole number from 0 to 6, wherein s and t are chosen, such that the sum of the ring atoms including the germanium atom is 5 to 8, and
X means N-R²¹, O, S or is absent, wherein R²¹ is H or C₁₋₁₀ alkyl,
wherein the germanium-containing ring can be anellated with one or more aliphatic or aromatic rings and unsubstituted or mono or polysubstituted, whereupon the number of the hydrogen atoms of the ring is correspondingly reduced.

5. Composition according to claim 1, comprising at least one acylgermanium compound with 2 germanium atoms according to the general Formula(IV), 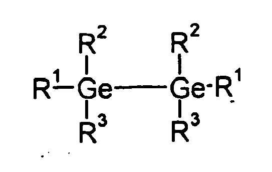 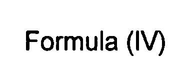 in which
R¹, R² and R⁹ independently of each other are 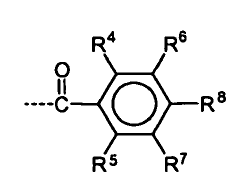 a C₁₋₃ alkyl group or a C₁₋₃ acyl group;
R⁴, R⁵ independently of one another are each H, halogen, a branched or linear C₁₋₆ alkyl or -O-C₁₋₆ alkyl group;
R⁶, R⁷, R⁸ are independently of each other H, halogen, a branched, cyclic or preferably linear alkyl, alkenyl, alkyloxy or alkenoxy group, which can be interrupted by one or more O, S or -NR²⁰ and can be substituted by one or more polymerisable groups and/or R⁹ groups;
R⁹ is -OH, -CₓF₂ₓ₊₁ with x = 1 to 20, -[Si (CH₃)₂]y-CH₃ with y = 1 to 20; and
R²⁰ is H, halogen, a branched, cyclic or preferably linear alkyl, alkenyl, alkyloxy or alkenoxy C₁₋₂₀ group.

6. Composition according to claim 1, comprising at least one acylgermanium compound with 2 germanium atoms according to the general Formula (V), 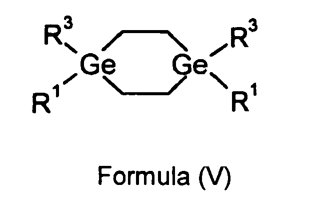 in which
R¹ and R³ independently of each other are 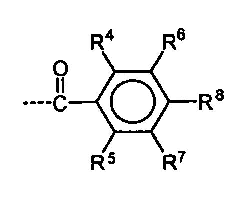 a C₁₋₃ alkyl group or a C₁₋₃ acyl group; ,
R⁴, R⁵ independently of one another are each H, halogen, a branched or linear C₁₋₆ alkyl or -O-C₁₋₆ alkyl group;
R⁶, R⁷, R⁸ are independently of each other H, halogen, a branched, cyclic or preferably linear alkyl, alkenyl, alkyloxy or alkenoxy C₁₋₂₀ group, which can be interrupted by one or more O, S or -NR²⁰ and can be substituted by one or more polymerisable groups and/or R⁹ groups;
R⁹ is -OH, -CₓF₂ₓ₊₁ with x = 1 to 20, -[Si (CH₃)₂]y-CH₃ with y = 1 to 20; and
R²⁰ is H, halogen, a branched, cyclic or preferably linear alkyl, alkenyl, alkyloxy or alkenoxy C₁₋₂₀ group.

7. Composition according to claim 1, comprising at least one acylgermanium compound with at least 2 germanium atoms according to the general Formula (VI), 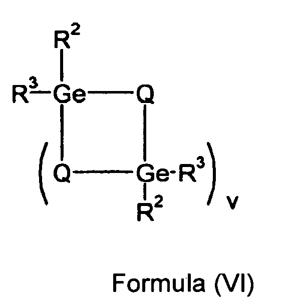 in which
R², R³ independently of each other are 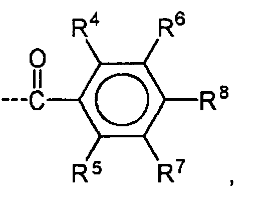 a C₁₋₃ alkyl group or a C₁₋₃ acyl group;
v is a whole number from 1 to 3, and
Q means N-R²², -CH₂-, -CH=CH-, -CH₂-CH₂- or -O-Si(R²³)₂-O-, wherein R²² is H, C₁₋₁₀ alkyl, preferably H or C₁₋₄ alkyl, or phenyl, and R²³ is C₁₋₁₀ alkyl or two groups R²³ form an oxygen bridge (-O-) between two Si atoms.

8. Composition according to one of the previous claims, in which the R², R³, R⁶, R⁷ and R⁸ groups are each substituted with 1 to 3 polymerisable groups.

9. Composition according to one of the previous claims, in which the polymerisable groups are selected from vinyl, styryl, (meth)acrylate, (meth)acrylamide or N-alkylacrylamide.

10. Composition according to one of the previous claims, comprising 0.001 to 5 wt % of the acylgermanium compound relative to the total mass of the composition.

11. Composition according to one of the previous claims, comprising at least one radically polymerisable monomer and/or pre-polymer as the polymerisable binder.

12. Composition according to claim 11, comprising a mono or polyfunctional (meth)acrylate or a mixture thereof as the binder.

13. Composition according to claim 11 or 12, comprising at least one ring-opening radically polymerisable monomer.

14. Composition according to one of the previous claims, comprising a mixture of mono and/or polyfunctional mercapto compounds and di and/or polyfunctional unsaturated monomers as the binder.

15. Composition according to one of the previous claims, comprising at least one additional initiator for the radical polymerisation.

16. Composition according to one of the previous claims, comprising at least one additional initiator for the cationic polymerisation.

17. Composition according to one of the preceding claims, additionally comprising filler.

18. Composition according to one of the previous claims, additionally comprising at least one additive that is selected from stabilisers, UV-absorbers, slip agents, wetting agents, dispersants, adhesion promoters, matting and gloss agents, levelling and film-forming auxiliaries, anti-skinning agents, light stabilisers, corrosion protection agents, flame retardants, antioxidants, optical brighteners, flow improvers, thickeners and foam suppressants.

19. Composition according to one of the previous claims, comprising
0.001 to 5 wt % acylgermanium compound,
5 to 99.9 wt % polymerisable binder,
0 to 90 wt % filler, each based on the total mass of the composition.

20. Composition according to claim 19, comprising 0 to 50 wt % of an additional additive.

21. System for manufacturing moulded articles which includes a composition according to one of the claims 1 to 20 and a LED light source.

22. System according to claim 21, in which the LED light source has a wavelength in the region 400 to 550 nm and the acylgermanium compound possesses an activation wavelength in the region 400 to 550 nm.

23. Use of an acylgermane according to Formula (I) as the initiator for the radical polymerisation.

24. Use of an acylgermane according to Formula (I) for preventing the formation of an inhibiting layer during radical polymerisation.

25. Use of an acylgermane according to Formula (I) for manufacturing adhesives, coatings, cements, composites, moulded articles or dental materials.

26. Process for manufacturing a moulded article, in which a composition according to one of the claims 1 to 20 is moulded into an article with the desired shape, and then partially or completely cured.

27. Process according to claim 26, in which the article is cured by irradiation with light having a wavelength from 200 to 700 nm.

28. Process according to claim 27, in which the article is cured by irradiation with light having a wavelength from 300 to 550 nm.

29. Process according to claim 27, in which the article is cured by irradiation with light having a wavelength from 400 to 800 nm.

30. Process according to one of the claims 26 to 29 for manufacturing dental crowns, bridges, inlays or artificial teeth.

## Revendications

1. Composition comportant au moins un liant polymérisable et un amorceur de polymérisation, **caractérisée en ce qu'**elle contient au moins un composé de type acyl-germanium doté d'au moins deux atomes de germanium, de formule générale (I) : 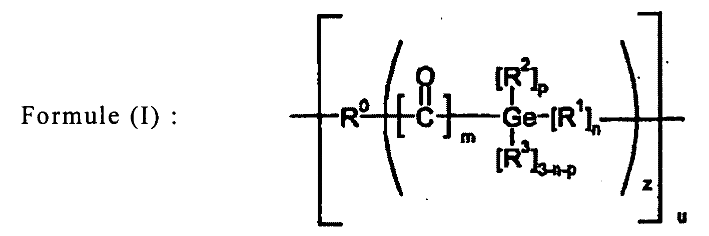 dans laquelle il y a de 1 à 3 groupe(s) acyle lié (s) à chacun des atomes de germanium,
et dans laquelle, selon une première possibilité :
- l'indice u vaut 1, et il n'y a de demi-liaison libre sur aucune des deux entités symbolisées par R⁰ et R¹,
- l'indice z est un nombre entier valant de 2 à 10,
- R⁰ représente un reste z-valent d'hydrocarbure aliphatique linéaire ou ramifié, aromatique ou aliphatique-aromatique, comportant de 1 à 50 atomes de carbone et de 0 à 10 hétéroatomes, qui porte en tant que substituants z groupes symbolisés dans les parenthèses, étant entendu que si z vaut 2 ou 3, R⁰ peut aussi représenter un atome d'azote N ou un groupe imino NH, (alkyle en C₁₋₃)-amino ou phénylamino, et que si z vaut 2 et m vaut 0, R⁰ peut aussi ne rien représenter du tout, de sorte que les deux groupes symbolisés dans les parenthèses sont alors reliés l'un à l'autre par une liaison chimique établie entre les atomes de germanium, étant aussi entendu que l'entité symbolisée par R⁰ peut porter en tant que substituant(s) un ou plusieurs atome(s) d'oxygène doublement lié(s) (soit le substituant oxo =O), groupe(s) cyano -CN et atome(s) d'halogène, ainsi qu'un ou plusieurs groupe(s) alkyle en C₁₋₆ ou alcoxy en C₁₋₆, linéaire(s) ou ramifié(s), et/ou un ou plusieurs groupe(s) polymérisable(s),
- et R¹ et R², indépendamment l'un de l'autre, représentent chacun un atome d'hydrogène ou un groupe de formule suivante : 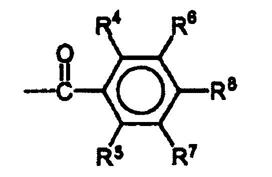 ou ont l'une des significations indiquées pour R³, étant entendu
- que R⁴ et R⁵ représentent chacun, indépendamment l'un de l'autre, un atome d'hydrogène ou d'halogène ou un groupe alkyle en C₁₋₆ ou alcoxy en C₁₋₆, linéaire ou ramifié, qui peut être interrompu par un ou plusieurs atome(s) d'oxygène,
- et que R⁶, R⁷ et R⁸ représentent chacun, indépendamment l'un de l'autre, un atome d'hydrogène ou d'halogène ou un groupe alkyle, alcényle, alcoxy ou alcényl-oxy en C₁₋₂₀, ramifié, cyclique ou de préférence linéaire, qui peut être interrompu par un ou plusieurs atome(s) d'oxygène ou de soufre ou groupe(s) de formule -NR²⁰-et porter en tant que substituant(s) un ou plusieurs groupe(s) polymérisable(s) et/ou reste(s) symbolisé(s) par R⁹, étant entendu que R⁹ représente un groupe hydroxyle, un groupe de formule -CₓF₂ₓ₊₁ où l'indice x vaut de 1 à 20, ou un groupe de formule -[Si(CH₃)₂]_{y}-CH₃ où l'indice y vaut de 1 à 20, et que R²⁰ représente un atome d'hydrogène ou d'halogène ou un groupe alkyle, alcényle, alcoxy ou alcényl-oxy en C₁₋₂₀, ramifié, cyclique ou de préférence linéaire,
étant entendu que R² peut aussi représenter un reste d'hydrocarbure aliphatique linéaire ou ramifié, aromatique ou aliphatique-aromatique, comportant de 1 à 50 atomes de carbone et de 0 à 10 hétéroatomes, qui relie l'un à l'autre, en pont, deux atomes de germanium ;
ou dans laquelle, selon une deuxième possibilité :
- l'indice u est un nombre entier valant de 2 à 100, et les deux demi-liaisons libres sur les restes terminaux symbolisés par R⁰ et R¹, ou sur l'atome de germanium dans le cas où il n'y a pas de reste R¹, sont occupées par un atome d'hydrogène ou un groupe hydroxyle ou raccordées l'une à l'autre grâce à l'existence d'une liaison chimique entre le reste R⁰ et le reste R¹ ou l'atome de germanium,
- l'indice z vaut 1,
- R⁰ représente un reste divalent d'hydrocarbure aliphatique linéaire ou ramifié, aromatique ou aliphatique-aromatique, comportant de 1 à 50 atomes de carbone et de 0 à 10 hétéroatomes, étant entendu que R⁰ peut aussi représenter un groupe de formule N-R²² ou de formule -O-Si(R²³)₂-O-, où R²² représente un atome d'hydrogène ou un groupe alkyle en C₁₋₁₀ ou phényle et R²³ représente un groupe alkyle en C₁₋₁₀ ou deux symboles R²³ représentent un atome d'oxygène en pont (-O-) entre deux atomes de silicium, étant aussi entendu que l'entité symbolisée par R⁰ peut porter en tant que substituant(s) un ou plusieurs atome(s) d'oxygène doublement lié(s) (soit le substituant oxo =O), groupe(s) cyano -CN et atome(s) d'halogène, ainsi qu'un ou plusieurs groupe(s) alkyle en C₁₋₆ ou alcoxy en C₁₋₆, linéaire(s) ou ramifié(s), et/ou un ou plusieurs groupe(s) polymérisable(s),
- R¹ représente un groupe carbonyle -C(=O)- ou ne représente rien,
- et R² représente un atome d'hydrogène ou un groupe de formule suivante : 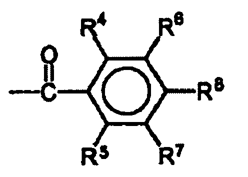 ou a l'une des significations indiquées pour R³, étant entendu
- que R⁴ et R⁵ représentent chacun, indépendamment l'un de l'autre, un atome d'hydrogène ou d'halogène ou un groupe alkyle en C₁₋₆ ou alcoxy en C₁₋₆, linéaire ou ramifié, qui peut être interrompu par un ou plusieurs atome(s) d'oxygène,
- et que R⁶, R⁷ et R⁸ représentent chacun, indépendamment l'un de l'autre, un atome d'hydrogène ou d'halogène ou un groupe alkyle, alcényle, alcoxy ou alcényl-oxy en C₁₋₂₀, ramifié, cyclique ou de préférence linéaire, qui peut être interrompu par un ou plusieurs atome(s) d'oxygène ou de soufre ou groupe(s) de formule -NR²⁰-et/ou porter en tant que substituant(s) un ou plusieurs groupe(s) polymérisable(s) et/ou reste(s) symbolisé(s) par R⁹, étant entendu que R⁹ représente un groupe hydroxyle, un groupe de formule -CₓF₂ₓ₊₁ où l'indice x vaut de 1 à 20, ou un groupe de formule -[Si(CH₃)₂]_{y}-CH₃ où l'indice y vaut de 1 à 20, et que R²⁰ représente un atome d'hydrogène ou d'halogène ou un groupe alkyle, alcényle, alcoxy ou alcényl-oxy en C₁₋₂₀, ramifié, cyclique ou de préférence linéaire ;
étant entendu que, dans les deux possibilités, les autres symboles ont les significations suivantes :
- l'indice m vaut 0 ou 1,
- l'indice n vaut 0 ou 1,
- l'indice p vaut 0 ou 1,
- et R³ représente un groupe alkyle en C₁₋₁₈ ou alcényle en C₂₋₁₈, linéaire ou ramifié, qui ne porte aucun substituant ou porte un ou plusieurs substituant(s) choisi(s) dans l'ensemble suivant :
atomes d'halogène, groupe cyano, groupes de formule 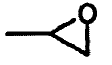
et -(alkyle en C₁₋₈)-. 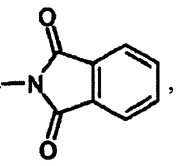 groupes symbolisés par -OR¹⁰, -SR¹⁰, -OCO-R¹⁰, -COO-R¹⁰, -CH=CH-CO-OR¹⁰, -C(alkyle en C₁₋₄)=C(alkyle en C₁₋₄)-CO-OR¹⁰, -CO-R¹³, -CO-CH=CH-CO-(alkyle en C₁₋₆), -CO-CH=CH-CO-phényle, -CO-CH=CH-COO-(alkyle en C₁₋₁₈), -NR¹¹R¹², -N(R¹¹)-CO-R¹⁰, -N(R¹¹)-COO-R¹⁰, -N(R¹¹)-CO-NR¹¹R¹², -N(R¹¹)-CO-Hal, -CO-NR¹¹R¹², -SO2-R¹⁰, -SO₂-OR¹⁰, -SO₂-NR¹¹R¹², -PO(O-alkyle en C₁₋₈)₂, -SiR¹⁴R¹⁵R¹⁶, -CH=CH-phényle, -C(alkyle en C₁₋₄)=C(alkyle en C₁₋₄)-phényle, groupes phényl-(alkyle en C₁₋₄), phényle, naphtyle, biphényle, cycloalkyle en C₅₋₁₂, groupes hétérocycliques oxygénés, soufrés ou azotés, saturés ou insaturés et comportant 5 ou 6 chaînons, et groupes benzophénonyle et thioxanthonyle,
étant entendu que :
- R¹⁰ représente un atome d'hydrogène, un groupe alkyle en C₁₋₁₈, alkyle en C₁₋₁₈ interrompu par un ou plusieurs atome(s) d'oxygène, alcényle en C₂₋₁₈, alcényle en C₂₋₁₈ interrompu par un ou plusieurs atome(s) d'oxygène, cycloalkyle en C₃₋₁₂, tétrahydropyrane-2-yle, phényl-(alkylène en C₁₋₂₀) ou phényl-(alcénylène en C₁₋₂₀), un groupe alkyle en C₁₋₁₈ sans substituant ou porteur d'un ou de plusieurs substituant(s) halogéno, cyclohexyle, cyclopentyle, tétrahydrofuranyle, furanyle ou isopropyl-4-méthyl-cyclohexyle, ou un groupe phényle, naphtyle ou biphénylyle, étant entendu que ces groupes cycliques peuvent ne porter aucun substituant ou porter de 1 à 5 substituant(s) halogéno, alkyle en C₁₋₈, alcoxy en C₁₋₈ et/ou alkylthio en C₁₋₈,
- R¹¹ et R¹² représentent chacun, indépendamment l'un de l'autre, un atome d'hydrogène ou un groupe alkyle en C₁₋₁₈, alkyle en C₁₋₁₈ interrompu par un ou plusieurs atome(s) d'oxygène, alcényle en C₂₋₁₈, alcényle en C₂₋₁₈ interrompu par un ou plusieurs atome(s) d'oxygène, cycloalkyle en C₃₋₁₂, phényl-(alkyle en C₁₋₄), phényle, naphtyle ou pyridyle, étant entendu que ces groupes cycliques peuvent ne porter aucun substituant ou porter de 1 à 5 substituant(s) halogéno, alkyle en C₁₋₈, alcoxy en C₁₋₈ et/ou alkylthio en C₁₋₈, ou bien R¹¹ et R¹² représentent des entités qui forment conjointement un hétérocycle oxygéné, soufré ou azoté comportant 5 ou 6 chaînons, qui peut de son côté être condensé avec un cycle aliphatique ou aromatique
- R¹³ représente un groupe alkyle en C₁₋₁₈ ou alcényle en C₂₋₁₈ qui peut être interrompu par un ou plusieurs atome(s) d'oxygène, cycloalkyle en C₃₋₁₂, phényl-(alkyle en C₁₋₄), phényle, naphtyle ou biphénylyle, étant entendu que les groupes cycliques mentionnés peuvent ne porter aucun substituant ou porter de 1 à 5 substituant(s) halogéno, alkyle en C₁₋₈, alcoxy en C₁₋₈ et/ou alkylthio en C₁₋₈,
- et R¹⁴, R¹⁵ et R¹⁶ représentent chacun, indépendamment l'un de l'autre, un atome d'hydrogène, un groupe alkyle en C₁₋₈, alcényle en C₂₋₈, phényl-alkyle en C₇₋₉, alcoxy en C₁₋₈ ou phényle, ou un groupe de formule -O-SiR¹⁷R¹⁸R¹⁹ où R¹⁷, R¹⁸ et R¹⁹ représentent chacun, indépendamment l'un de l'autre, un atome d'hydrogène ou un groupe alkyle en C₁₋₈, alcényle en C₂₋₈, phényl-alkyle en C₇₋₉, alcoxy en C₁₋₈ ou phényle ;
- ou bien R³ représente un groupe alkyle en C₂₋₁₈, ramifié ou de préférence linéaire, ou un groupe alkylène en C₂₋₁₈ qui est interrompu une ou plusieurs fois par un ou des chaînon(s) symbolisé(s) par -O-, -NH-, -NR¹¹- ou -S-, lesquels groupes peuvent ne porter aucun substituant ou porter un ou plusieurs substituant(s) choisi(s) dans l'ensemble suivant : atomes d'halogène, groupes symbolisés par -CN, -OR¹⁰, -SR¹⁰, -OCO-R¹⁰, -COO-R¹⁰, -NR¹¹R¹², -N(R¹¹)-CO-R¹⁰, -N(R¹¹)-COO-R¹⁰, -N(R¹¹)-CO-NR¹¹R¹², -N(R¹¹)-CO-Hal, -CO-NR¹¹R¹², -SO₂-R¹⁰, -SO₂-OR¹⁰, -SO₂-NR¹¹R¹², -PO(O-alkyle en C₁₋₈)₂ et -SiR¹⁴R¹⁵R¹⁶, et groupes phényl-(alkyle en C₁₋₄), phényle et cycloalkyle en C₅₋₁₂,
étant entendu que les symboles R¹⁰, R¹¹, R¹², R¹⁴, R¹⁵ et R¹⁶ ont les significations indiquées plus haut ;
- ou bien R³ représente un groupe alkyle en C₂₋₁₈, ramifié ou de préférence linéaire, ou un groupe alkylène en C₂₋₁₈ qui est interrompu une ou plusieurs fois par un ou des chaînon(s) ou fragment(s) symbolisé(s) par -CO-, -COO-, -OCO-, -OCOO-, -CO-N(R¹¹)-, -N(R¹¹)-CO-, -N(R¹¹)-CO-NR¹¹-, -N(R¹¹)-COO-, -COO-(alkylène en C₁₋₆)-, -COS-(alkylène en C₁₋₁₈)-, -SO₂-, -SO₂-O-, -SO₂-N(R¹¹)- ou -(CH₃)₂Si[OSi(CH₃)₂]_{q}- où l'indice q vaut de 1 à 6, ou par un ou des groupe(s) phényl-(alkylène en C₁₋₄), phénylène, naphtylène, biphénylène ou cycloalkylène en C₅₋₁₂, ou encore par un ou des groupe(s) de type hétérocycle oxygéné, soufré ou azoté à 5 ou 6 chaînons,
étant entendu que le symbole R¹¹ a la signification indiquée plus haut ;
- ou bien R³ représente un groupe triméthyl-silyle, un groupe de formule Hal-(CH₃)₂Si-[OSi(CH₃)₂]ᵣ- ou (CH₃)₃Si-[OSi(CH₃)₂]ᵣ-où l'indice r vaut de 1 à 6, ou un groupe symbolisé par -COOH, -COO-R¹⁰, -CO-NR¹¹R¹², -CO-vinyle ou -CO-phényle où le groupe phényle peut ne porter aucun substituant ou porter un ou des substituant(s) méthyle, méthoxy et/ou chloro,
étant entendu que les symboles R¹⁰, R¹¹ et R¹² ont les significations indiquées plus haut ;
- ou bien R³ représente un groupe phényl-(alkyle en C₁₋₂₀), phényle, naphtyle, biphénylyle ou cycloalkyle en C₅₋₁₂, ou un groupe hétéro-cyclyle oxygéné, soufré ou azoté, saturé ou insaturé et comportant 5 ou 6 chaînons, étant entendu que ces groupes cycliques peuvent ne porter aucun substituant ou porter de 1 à 5 substituant(s) halogéno, alkyle en C₁₋₈, alcoxy en C₁₋₈, alkylthio en C₁₋₈ et/ou de formule -NR¹¹R¹²,
étant entendu que les symboles R¹¹ et R¹² ont les significations indiquées plus haut ;
étant entendu que deux des groupes symbolisés par R¹, R² et R³ peuvent être raccordés l'un à l'autre et former ainsi un cycle-de 5 à 8 chaînons qui peut de son côté être condensé avec un ou plusieurs cycle(s) aliphatique(s) ou aromatique(s), ces cycles pouvant comporter d'autres hétéroatomes en plus de l'atome de germanium, et étant entendu que des groupes de symboles différents, ou encore des groupes de mêmes symboles dans le cas où l'indice 3-n-p est supérieur à 1, peuvent être raccordés l'un à l'autre et former ainsi un ou plusieurs cycle(s), lequel ou lesquels cycle(s) peut ou peuvent ne porter aucun substituant ou porter un ou plusieurs substituant(s).

2. Composition conforme à la revendication 1, qui contient au moins un composé de formule (I') : 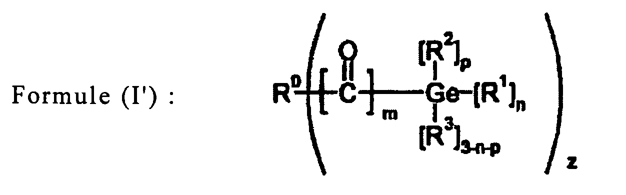 dans laquelle
- R⁰ représente un groupe alkyle comportant de 1 à 6 atomes de carbone dont z atomes d'hydrogène ont été remplacés par autant de groupes symbolisés entre les parenthèses de la formule (I'),
- R¹ et R², indépendamment l'un de l'autre, représentent chacun un atome d'hydrogène ou un groupe de formule suivante : 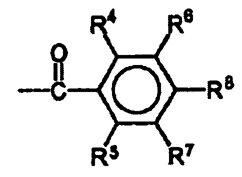 ou ont l'une des significations indiquées pour R³ étant entendu
- que R⁴ et R⁵ représentent chacun, indépendamment l'un de l'autre, un atome d'hydrogène ou d'halogène ou un groupe alkyle en C₁₋₆ ou alcoxy en C₁₋₆, linéaire ou ramifié,
- et que R⁶, R⁷ et R⁸ représentent chacun, indépendamment l'un de l'autre, un atome d'hydrogène ou d'halogène ou un groupe alkyle, alcényle, alcoxy ou alcényl-oxy en C₁₋₂₀, ramifié, cyclique ou de préférence linéaire, qui peut être interrompu par un ou plusieurs atome(s) d'oxygène ou de soufre ou groupe(s) de formule -NR'-et porter en tant que substituant(s) un ou plusieurs groupe(s) polymérisable(s) et/ou reste(s) symbolisé(s) par R⁹, étant entendu que R' représente un atome d'hydrogène ou d'halogène ou un groupe alkyle, alcényle, alcoxy ou alcényl-oxy en C₁₋₂₀, ramifié, cyclique ou de préférence linéaire,
- R³ représente un groupe aromatique en C₆₋₁₀, qui peut porter en tant que substituant un groupe alkyle, alcényle, alcoxy ou alcényl-oxy en C₁₋₁₈, ramifié ou de préférence linéaire, lesquels groupes peuvent être interrompus par un ou plusieurs atome(s) d'oxygène, ou représente un groupe alkyle, alcényle, alcoxy ou alcényl-oxy en C₁₋₁₈, ramifié ou de préférence linéaire, qui peut être interrompu par un ou plusieurs atome(s) d'oxygène,
- l'indice m vaut 0 ou 1,
- l'indice n vaut 0 ou 1,
- l'indice p vaut 0 ou 1,
- et l'indice z vaut de 2 à 6.

3. Composition conforme à la revendication 1, qui contient au moins un composé de type acyl-germanium de formule générale (II) : 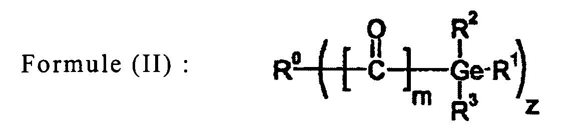 dans laquelle
- R¹, R² et R³, indépendamment l'un de l'autre, représentent chacun un groupe alkyle en C₁₋₃ ou acyle en C₁₋₃, ou un groupe de formule suivante : 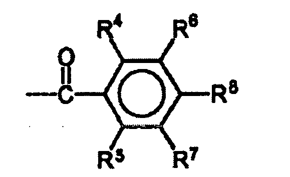 étant entendu
- que R⁴ et R⁵ représentent chacun, indépendamment l'un de l'autre, un atome d'hydrogène ou d'halogène ou un groupe alkyle en C₁₋₆ ou alcoxy en C₁₋₆, linéaire ou ramifié,
- et que R⁶, R⁷ et R⁸ représentent chacun, indépendamment l'un de l'autre, un atome d'hydrogène ou d'halogène ou un groupe alkyle, alcényle, alcoxy ou alcényl-oxy en C₁₋₂₀, ramifié, cyclique ou de préférence linéaire, qui peut être interrompu par un ou plusieurs atome(s) d'oxygène ou de soufre ou groupe(s) de formule -NR²⁰-et porter en tant que substituant(s) un ou plusieurs groupe(s) polymérisable(s) et/ou reste(s) symbolisé(s) par R⁹,
étant entendu que
R⁹ représente un groupe hydroxyle, un groupe de formule -CₓF₂ₓ₊₁ où l'indice x vaut de 1 à 20, ou un groupe de formule -[Si(CH₃)₂]_{y}-CH₃ où l'indice y vaut de 1 à 20,
et R²⁰ représente un atome d'hydrogène ou d'halogène ou un groupe alkyle, alcényle, alcoxy ou alcényl-oxy en C₁₋₂₀, ramifié, cyclique ou de préférence linéaire.

4. Composition conforme à la revendication 1, qui contient au moins un composé de type acyl-germanium de formule générale (III) : 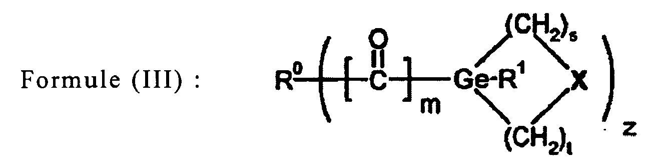 dans laquelle
- R¹ représente un groupe alkyle en C₁₋₃ ou acyle en C₁₋₃, ou un groupe de formule suivante : 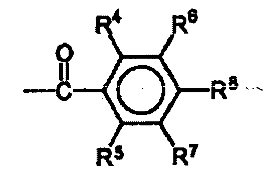
- les indices s et t sont chacun, indépendamment l'un de l'autre, un nombre entier valant de 0 à 6, étant entendu que les valeurs de ces indices s et t sont choisies de telle sorte que le nombre total des atomes du cycle, y compris l'atome de germanium, vaut de 5 à 8,
- et X représente un atome d'oxygène ou de soufre ou un groupe de formule N-R²¹ où R²¹ représente un atome d'hydrogène ou un groupe alkyle en C₁₋₁₀,
étant entendu que le cycle comportant l'atome de germanium peut être condensé avec un ou plusieurs cycle(s) aliphatique(s) ou aromatique(s), et peut ne porter aucun substituant ou porter un ou plusieurs substituant(s), le nombre des atomes d'hydrogène du cycle étant dans ce cas réduit en conséquence.

5. Composition conforme à la revendication 1, qui contient au moins un composé de type acyl-germanium doté de deux atomes de germanium, de formule générale (IV) : 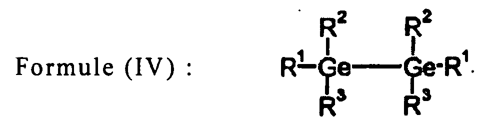 dans laquelle
- R¹, R² et R³, indépendamment l'un de l'autre, représentent chacun un groupe alkyle en C₁₋₃ ou acyle en C₁₋₃, ou un groupe de formule suivante : 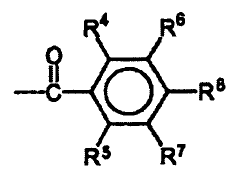 étant entendu
- que R⁴ et R⁵ représentent chacun, indépendamment l'un de l'autre, un atome d'hydrogène ou d'halogène ou un groupe alkyle en C₁₋₆ ou alcoxy en C₁₋₆, linéaire ou ramifié,
- et que R⁶, R⁷ et R⁸ représentent chacun, indépendamment l'un de l'autre, un atome d'hydrogène ou d'halogène ou un groupe alkyle, alcényle, alcoxy ou alcényl-oxy en C₁₋₂₀, ramifié, cyclique ou de préférence linéaire, qui peut être interrompu par un ou plusieurs atome(s) d'oxygène ou de soufre ou groupe(s) de formule -NR²⁰-et porter en tant que substituant(s) un ou plusieurs groupe(s) polymérisable(s) et/ou reste(s) symbolisé(s) par R⁹,
étant entendu que
R⁹ représente un groupe hydroxyle, un groupe de formule -CₓF₂ₓ₊₁ où l'indice x vaut de 1 à 20, ou un groupe de formule -[Si(CH₃)₂]_{y}-CH₃ où l'indice y vaut de 1 à 20,
et R²⁰ représente un atome d'hydrogène ou d'halogène ou un groupe alkyle, alcényle, alcoxy ou alcényl-oxy en C₁₋₂₀, ramifié, cyclique ou de préférence linéaire.

6. Composition conforme à la revendication 1, qui contient au moins un composé de type acyl-germanium doté de deux atomes de germanium, de formule générale (V) : 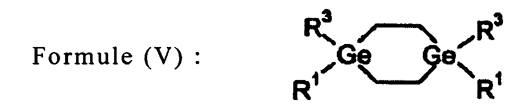 dans laquelle
- R¹ et R³, indépendamment l'un de l'autre, représentent chacun un groupe alkyle en C₁₋₃ ou acyle en C₁₋₃, ou un groupe de formule suivante : 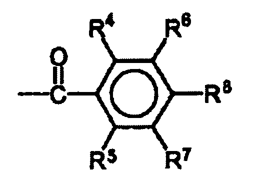 étant entendu
- que R⁴ et R⁵ représentent chacun, indépendamment l'un de l'autre, un atome d'hydrogène ou d'halogène ou un groupe alkyle en C₁₋₆ ou alcoxy en C₁₋₆, linéaire ou ramifié,
- et que R⁶, R⁷ et R⁸ représentent chacun, indépendamment l'un de l'autre, un atome d'hydrogène ou d'halogène ou un groupe alkyle, alcényle, alcoxy ou alcényl-oxy en C₁₋₂₀, ramifié, cyclique ou de préférence linéaire, qui peut être interrompu par un ou plusieurs atome(s) d'oxygène ou de soufre ou groupe(s) de formule -NR²⁰-et porter en tant que substituant(s) un ou plusieurs groupe(s) polymérisable(s) et/ou reste(s) symbolisés par R⁹,
étant entendu que
R⁹ représente un groupe hydroxyle, un groupe de formule -CₓF₂ₓ₊₁ où l'indice x vaut de 1 à 20, ou un groupe de formule -[Si(CH₃)₂]_{y}-CH₃ où l'indice y vaut de 1 à 20,
et R²⁰ représente un atome d'hydrogène ou d'halogène ou un groupe alkyle, alcényle, alcoxy ou alcényl-oxy en C₁₋₂₀, ramifié, cyclique ou de préférence linéaire.

7. Composition conforme à la revendication 1, qui contient au moins un composé de type acyl-germanium doté de deux atomes de germanium, de formule générale (VI) : 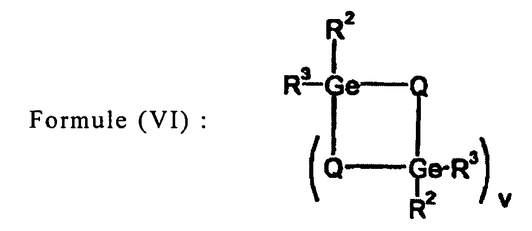 dans laquelle
- R² et R³ indépendamment l'un de l'autre, représentent chacun un groupe alkyle en C₁₋₃ ou acyle en C₁₋₃, ou un groupe de formule suivante : 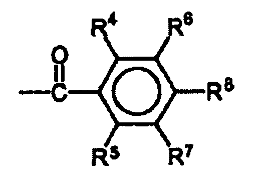
- l'indice v est un nombre entier valant de 1 à 3,
- et Q représente un groupe de formule >N-R²², -CH₂-, -CH=CH-, -CH₂-CH₂- ou -O-Si(R²³)₂-O-, où R²² représente un atome d'hydrogène ou un groupe alkyle en C₁₋₁₀, de préférence un atome d'hydrogène ou un groupe alkyle en C₁₋₄, ou un groupe phényle, et R²³ représente un groupe alkyle en C₁₋₁₀, ou deux entités symbolisées par R²³ représentent ensemble un atome d'oxygène placé en pont entre deux atomes de silicium.

8. Composition conforme à l'une des revendications précédentes, dans laquelle les groupes symbolisés par R², R³, R⁶, R⁷ et R⁸ portent chacun, en tant que substituant(s), de 1 à 3 groupe(s) polymérisable(s).

9. Composition conforme à l'une des revendications précédentes, dans laquelle les groupes polymérisables sont choisis parmi les groupes vinyle, styryle, acrylate, méthacrylate, acrylamido, méthacrylamido et N-alkyl-acrylamido.

10. Composition conforme à l'une des revendications précédentes, qui contient de 0,001 à 5 % de composé(s) de type acyl-germanium, en poids rapporté au poids total de la composition.

11. Composition conforme à l'une des revendications précédentes, qui contient, en tant que liant polymérisable, au moins un monomère et/ou prépolymère polymérisable par voie radicalaire.

12. Composition conforme à la revendication 11, qui contient en tant que liant un acrylate ou méthacrylate monofonctionnel ou poly-fonctionnel ou un mélange de tels composés.

13. Composition conforme à la revendication 11 ou 12, qui contient au moins un monomère polymérisable par ouverture de cycle par voie radicalaire.

14. Composition conforme à l'une des revendications précédentes, qui contient en tant que liant un mélange de composés de type thiol monofonctionnels et/ou polyfonctionnels et de monomères insaturés difonctionnels et/ou polyfonctionnels.

15. Composition conforme à l'une des revendications précédentes, qui contient au moins un autre amorceur pour polymérisation radicalaire.

16. Composition conforme à l'une des revendications précédentes, qui contient au moins un autre amorceur pour polymérisation cationique.

17. Composition conforme à l'une des revendications précédentes, qui contient en outre une charge.

18. Composition conforme à l'une des revendications précédentes, qui contient en outre au moins un adjuvant qui est choisi parmi les suivants : stabilisants, agents absorbant le rayonnement ultraviolet, agents lubrifiants, agents mouillants, agents dispersants, agents d'adhérence, agents de matité ou de brillance, auxiliaires de nivellement et de formation de film, agents empêchant la formation d'une peau, agents de protection contre la lumière, agents de protection contre la corrosion, retardateurs de combustion, anti-oxydants, azurants optiques, agents améliorant l'écoulement, épaississants, et agents anti-mousse.

19. Composition conforme à l'une des revendications précédentes, qui contient :
- de 0,001 à 5 % en poids de composé de type acyl-germanium,
- de 5 à 99,9 % en poids de liant polymérisable,
- et de 0 à 90 % en poids de charge,
chacun de ces pourcentages étant rapporté au poids total de la composition.

20. Composition conforme à la revendication 19, qui contient de 0 à 50 % en poids d'un autre adjuvant.

21. Système conçu pour la production de corps moulés, qui comprend une composition conforme à l'une des revendications 1 à 20 et une source de lumière de type diode LED.

22. Système conforme à la revendication 21, dans lequel la source de lumière de type diode LED présente une longueur d'onde d'émission dans le domaine allant de 400 à 550 nm, et le composé de type acyl-germanium présente une longueur d'onde d'activation dans le domaine allant de 400 à 550 nm.

23. Utilisation d'un acyl-germane de formule (I) en qualité d'amorceur pour une polymérisation radicalaire.

24. Utilisation d'un acyl-germane de formule (I) en vue d'empêcher la formation d'une couche d'inhibition lors d'une polymérisation radicalaire.

25. Utilisation d'un acyl-germane de formule (I) en vue de la fabrication d'adhésifs, de revêtements, de ciments, de composites, de pièces moulées ou de matériaux dentaires.

26. Procédé de fabrication d'une pièce moulée, dans lequel on moule une composition conforme à l'une des revendications 1 à 20 en une pièce de forme voulue, puis on fait complètement ou partiellement durcir celle-ci.

27. Procédé conforme à la revendication 26, dans lequel on fait durcir la pièce en l'irradiant avec de la lumière d'une longueur d'onde de 200 à 700 nm.

28. Procédé conforme à la revendication 27, dans lequel on fait durcir la pièce en l'irradiant avec de la lumière d'une longueur d'onde de 300 à 550 nm.

29. Procédé conforme à la revendication 26, dans lequel on fait durcir la pièce en l'irradiant avec de la lumière d'une longueur d'onde de 400 à 800 nm.

30. Procédé conforme à l'une des revendications 26 à 29, pour la fabrication de couronnes, ponts ou incrustations dentaires ou de dents artificielles.
